# EUROPEAN PATENT APPLICATION

(11) **EP 3 954 382 A1**
(43) Date of publication of application: **16.02.2022**
(21) Application number: 20784004.2
(22) Date of filing: 03.04.2020
(51) Int. Cl.: A61K 39/00, A61P 35/00, A61P 37/04, A61P 43/00, C07D 473/18, C07K 14/47, A61K 9/107, A61K 47/02, A61K 47/10, A61K 47/14, A61K 31/522

(54) **WATER SOLUBLE ADJUVANT AND COMPOSITION CONTAINING SAME**

(30) Priority: 05.04.2019 JP 2019072911
(71) Applicant: Sumitomo Dainippon Pharma Co., Ltd., Chuo-ku Osaka-shi Osaka 541-8524 (JP)
(72) Inventor: BAN, Hitoshi, Osaka-shi, Osaka 554-0022 (JP); TAKANASHI, Yosuke, Osaka-shi, Osaka 554-0022 (JP); IMAZAKI, Yusuke, Osaka-shi, Osaka 554-0022 (JP)
(74) Representative: Mewburn Ellis LLP
(86) International application number: PCT/JP2020/015364
(87) International publication number: WO 2020/204173

(57) **Abstract**

The present invention relates to a compound useful as a vaccine adjuvant for cancer vaccine, a preparation process thereof, a pharmaceutical composition comprising the compound, and use of the compound as a vaccine adjuvant for cancer vaccine.

## Description

### TECHNICAL FIELD

The present invention relates to a compound useful as vaccine adjuvant for vaccine (cancer vaccine or infection vaccine), a manufacturing process thereof, a pharmaceutical composition comprising the compound, and use of the compound as vaccine adjuvant for vaccine (cancer vaccine or infection vaccine).

### BACKGROUND ART

In general, cancer vaccine therapy activates an immune cell specific to tumor by using protein or peptide obtained from tumor antigen to treat a cancer. Among the therapy, a therapy in which tumor antigen peptide is used as the antigen is referred to as cancer peptide vaccine therapy. In general, the therapy with only tumor antigen peptide brings on low immunogenicity. Thus, in order to induce cytotoxic T-lymphocyte (CTL) which is important for antitumor immunity, a vaccine adjuvant is used together. For example, W/O emulsion can easily retain an antigen peptide in its internal phase because it has aqueous phase in the internal phase. Thus, it has been reported that use of W/O emulsion as a vaccine adjuvant shows effective CTL induction (Patent Literature 1).

W/O emulsion to be used as vaccine adjuvant for tumor antigen peptide includes an emulsion composition for dilution (Patent Literature 1), as well as Incomplete Freund's Adjuvant (IFA) and Montanide^{™} (Non-Patent Literatures 1 and 2). In addition, Complete Freund's Adjuvant (CFA) which is prepared by adding inactivated Mycobacterium Tuberculosis to W/O emulsion is also known. However, CFA has not been allowed to be used in human due to its toxicity (Non-Patent Literature 2).

Conventionally, adjuvant compositions prepared by adding an inactivated bacterial body itself to an adjuvant like CFA had been used for enhancing the target activity, but recently vaccine adjuvants comprising a compound whose working mechanism is known have been developed. Among them, Toll like receptor 7 (TLR7) has been reported to activate Th1 cell to enhance cellular immunity which is needed for antitumor activity (Non-Patent Literature 3). As TLR7 agonist, various small molecules have been reported (Patent Literatures 4 and 5).

It has been known that these small molecule TLR7 agonists can be chemically modified to adjust their physical property. For example, there are some reports of making a conjugate of a TLR7 agonist and polyethylene glycol (PEG) to increase the water-solubility (Non-patent Literatures 6 and 7). However, the TLR7 agonist conjugated with PEG exhibited no adjuvant activity, and it was necessary to further conjugate the PEG with lipid to recover the activity (Non-patent Literature 2).

### PRIOR ART

### [Patent Reference]

[Patent Literature 1] WO 2006/078059
[Patent Literature 2] WO 2010/093436

### [Non-patent Reference]

[Non-Patent Literature 1] J Immunother Cancer. 2016 Sep 20; 4: 56
[Non-Patent Literature 2] Semin Immunol. 2010 Jun; 22(3): 155-61.
[Non-Patent Literature 3] Vaccine. 2011 Apr 12; 29(17): 3341-55.
[Non-Patent Literature 4] Expert Opin Ther Pat. 2011 Jun; 21(6): 927-44
[Non-Patent Literature 5] Expert Opin Ther Pat. 2014 Apr; 24(4): 453-70
[Non-Patent Literature 6] Bioconjug Chem. 2009 20 (6): 1194-200
[Non-Patent Literature 7] Bioconjug Chem. 2011 Mar 16; 22(3): 445-54

### Summary of Invention

### (Technical Problem)

The purpose of the present invention may be to provide a conjugated TLR7 agonist for enhancing adjuvant activity.

### (Solution to Problem)

The present inventors have extensively studied to find a TLR7 agonist for enhancing adjuvant activity, and then have found that a TLR7 agonist to which water-solubility is added by conjugating a TLR7 agonist having an adenine structure with polyethylene glycol (PEG) has an excellent adjuvant activity. Based upon the findings, the present invention has been achieved. According to the present invention, an adenine derivative of the following formula (1) (hereinafter, also referred to as "the present compound") is provided.

The present invention is as described below.

### (Item 1)

A compound of formula (1): 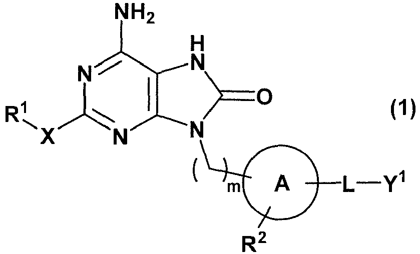 or a pharmaceutically acceptable salt thereof, wherein
X is single bond, oxygen atom, sulfur atom, SO, SO₂, or NR⁶, wherein R⁶ is hydrogen atom or C₁₋₆ alkyl,
R¹ is C₁₋₆ alkyl which may be substituted with 1 - 5 substituents selected independently from the group consisting of halogen, hydroxy, and C₁₋₆ alkoxy,
R² is hydrogen atom, halogen, hydroxy, C₁₋₆ alkyl, C₁₋₆ alkoxy or cyano,
m is an integer of 1 - 5,
A is 5- to 8-membered monocyclic aromatic carbon ring or 4- to 7-membered monocyclic aromatic hetero ring,
L is a linker, and
Y¹ is - (CH₂CH₂O)ₙ-R³, wherein R³ is hydrogen atom or C₁₋₆ alkyl, and n is an integer of 3 - 100,
provided that the compound having the following structure is excluded.

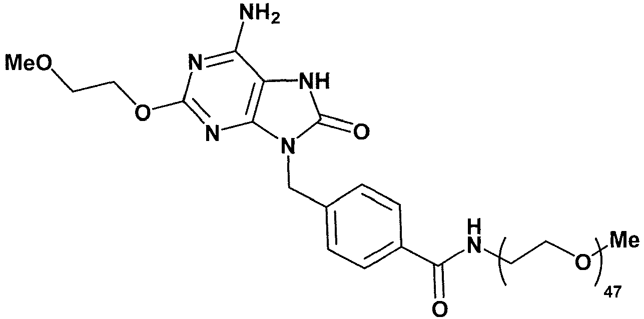

### (Item 2)

The compound of Item 1 or a pharmaceutically acceptable salt thereof, wherein X is single bond, oxygen atom, or NR⁶ (wherein R⁶ is hydrogen atom or C₁₋₆ alkyl) .

### (Item 3)

The compound of Item 2 or a pharmaceutically acceptable salt thereof, wherein X is single bond or oxygen atom.

### (Item 4)

The compound of any one of Items 1 to 3 or a pharmaceutically acceptable salt thereof, wherein R¹ is C₁₋₆ alkyl which may be substituted with 1 - 3 the same or different C₁₋₆ alkoxy.

### (Item 5)

The compound of Item 4 or a pharmaceutically acceptable salt thereof, wherein R¹ is C₁₋₆ alkyl which may be substituted with one C₁₋₆ alkoxy.

### (Item 6)

The compound of any one of Items 1 to 5 or a pharmaceutically acceptable salt thereof, wherein R² is hydrogen atom, halogen, C₁₋₆ alkyl, or C₁₋₆ alkoxy.

### (Item 7)

The compound of Item 6 or a pharmaceutically acceptable salt thereof, wherein R² is hydrogen atom or halogen.

### (Item 8)

The compound of any one of Items 1 to 7 or a pharmaceutically acceptable salt thereof, wherein m is 1.

### (Item 9)

The compound of any one of Items 1 to 8 or a pharmaceutically acceptable salt thereof, wherein A is benzene ring or pyridine ring.

### (Item 10)

The compound of Item 9 or a pharmaceutically acceptable salt thereof, wherein A is pyridine ring.

### (Item 11)

The compound of any one of Items 1 to 10 or a pharmaceutically acceptable salt thereof, wherein
L is -O-, -NR^{Y}-, -C(O)-, -C(O)O-, -OC(O)-, -C(O)NR^{Y}-,-NR^{Y}C(O) -, -CH₂NR^{Y}-, -CH₂O-, -OC(O)O-, -NR⁴C(O)O-, -OC(O)NR^{Y}-, -NR⁴C(O)NR^{Y}-, -OC(S)NR^{Y}-, or -NR⁴C(S)NR^{Y}-, wherein R⁴ is hydrogen atom or C₁₋₆ alkyl, and R^{Y} is hydrogen atom, C₁₋₆ alkyl or Y² wherein Y² is -(CH₂CH₂O)ₚ-R⁵ (wherein R⁵ is hydrogen atom or C₁₋₆ alkyl, and p is an integer of 3 - 100).

### (Item 12)

The compound of Item 11 or a pharmaceutically acceptable salt thereof, wherein L is -CH₂NR^{Y}-.

### (Item 13)

The compound of Item 12 or a pharmaceutically acceptable salt thereof, wherein L is -CH₂NR^{Y}- wherein R^{Y} is hydrogen atom, C₁₋₆ alkyl or Y² wherein Y² is - (CH₂CH₂O)ₚ-R⁵ (wherein R⁵ is hydrogen atom or C₁₋₆ alkyl, and p is an integer of 3 - 100) .

### (Item 14)

The compound of Item 12 or a pharmaceutically acceptable salt thereof, wherein L is -CH₂NR^{Y}- wherein R^{Y} is hydrogen atom or C₁₋₆ alkyl.

### (Item 15)

The compound of any one of Items 1 to 14 or a pharmaceutically acceptable salt thereof, wherein Y¹ is - (CH₂CH₂O)ₘ-R³ wherein m is an integer of 3 - 40, and R³ is hydrogen atom or C₁₋₆ alkyl.

### (Item 16)

The compound of any one of Items 1 to 14 or a pharmaceutically acceptable salt thereof, wherein Y¹ is - (CH₂CH₂O)ₘ-R³ wherein m is an integer of 3 - 20, and R³ is hydrogen atom or C₁₋₆ alkyl.

### (Item 17)

The compound of Item 1 or a pharmaceutically acceptable salt thereof, wherein the compound is a compound of formula (2) : 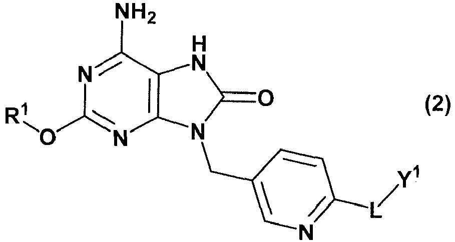 or formula (3): 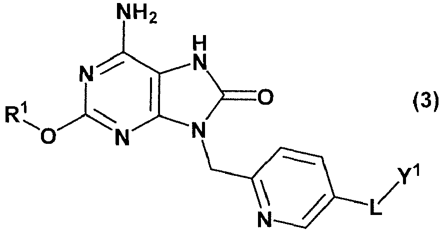 wherein
R¹ is C₁₋₆ alkyl which may be substituted with one C₁₋₆ alkoxy,
L is -CH₂NR^{Y}- wherein R^{Y} is hydrogen atom, C₁₋₆ alkyl or Y², wherein Y² is -(CH₂CH₂O)ₚ-R⁵ (wherein R⁵ is hydrogen atom or C₁₋₆ alkyl, and p is an integer of 3 - 40), and
Y¹ is - (CH₂CH₂O)ₙ-R³ (wherein R³ is hydrogen atom or C₁₋₆ alkyl, and n is an integer of 3 - 40).

### (Item 18)

The compound of Item 1 or a pharmaceutically acceptable salt thereof, wherein the compound is a compound of formula (2) : 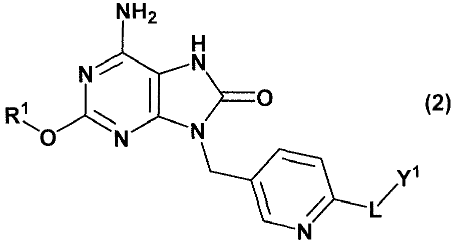 or formula (3): 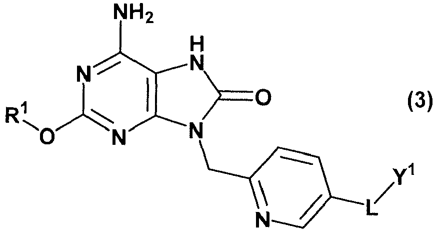 wherein
R¹ is C₁₋₆ alkyl which may be substituted with one C₁₋₆ alkoxy,
L is -CH₂NR^{Y}- wherein R^{Y} is hydrogen atom or C₁₋₆ alkyl,
Y¹ is - (CH₂CH₂O)ₙ-R³ (wherein R³ is hydrogen atom or C₁₋₆ alkyl, and n is an integer of 3 - 20).

### (Item 19)

The compound of Item 1 or a pharmaceutically acceptable salt thereof, which is selected from:
6-amino-2-butoxy-9-{[5-(16-hydroxy-2-methyl-5,8,11,14-tetraoxa-2-azahexadecan-1-yl)pyridin-2-yl]methyl}-7,9-dihydro-8H-purin-8-one (Example 1),
6-amino-2-butoxy-9-{[6-(16-hydroxy-2-methyl-5,8,11,14-tetraoxa-2-azahexadecan-1-yl)pyridin-3-yl]methyl}-7,9-dihydro-8H-purin-8-one (Example 2),
6-amino-2-butoxy-9-{[4-(16-hydroxy-2-methyl-5,8,11,14-tetraoxa-2-azahexadecan-1-yl)phenyl]methyl}-7,9-dihydro-8H-purin-8-one (Example 3),
6-amino-9-{[4- (16-hydroxy-2-methyl-5, 8,11, 14-tetraoxa-2-azahexadecan-1-yl)phenyl]methyl}-2-(2-methoxyethoxy)-7,9-dihydro-8H-purin-8-one (Example 4),
6-amino-2-butoxy-9-({6-[13-hydroxy-2-(2-{2-[2-(2-hydroxyethoxy)ethoxy]ethoxy}ethyl)-5,8,11-trioxa-2-azatridecan-1-yl]pyridin-3-yl}methyl)-7,9-dihydro-8H-purin-8-one (Example 6),
6-amino-2-butoxy-9-{[6-(31-hydroxy-2-methyl-5,8,11,14,17,20,23,26,29-nonaoxa-2-azahentriacontan-1-yl)pyridin-3-yl]methyl}-7,9-dihydro-8H-purin-8-one (Example 7),
6-amino-2-butoxy-9-{[6-(73-hydroxy-2-methyl-5,8,11,14,17,20,23,26,29,32,35,38,41,44,47,50,53,56,59,62,6 5,68,7l-tricosaoxa-2-azatriheptacontan-1-yl)pyridin-3-yl]methyl}-7,9-dihydro-8H-purin-8-one (Example 8), and
6-amino-2-butoxy-9-{[6-(109-hydroxy-2-methyl-5,8,11,14,17,20,23,26,29,32,35,38,41,44,47,50,53,56,59,62,6 5,68,71,74,77,80,83,86,89,92,95,98,101,104,107-pentatriacontaoxa-2-azanonahectan-1-yl)pyridin-3-yl]methyl}-7,9-dihydro-8H-purin-8-one (Example 9).

### (Item 20)

The compound of Item 1 or a pharmaceutically acceptable salt thereof, which is selected from:
6-amino-2-butoxy-9-{[5-(16-hydroxy-2-methyl-5,8,11,14-tetraoxa-2-azahexadecan-1-yl)pyridin-2-yl]methyl}-7,9-dihydro-8H-purin-8-one (Example 1),
6-amino-2-butoxy-9-{[6-(16-hydroxy-2-methyl-5,8,11,14-tetraoxa-2-azahexadecan-1-yl)pyridin-3-yl]methyl}-7,9-dihydro-8H-purin-8-one (Example 2),
6-amino-2-butoxy-9-{[4-(16-hydroxy-2-methyl-5,8,11,14-tetraoxa-2-azahexadecan-1-yl)phenyl]methyl}-7,9-dihydro-8H-purin-8-one (Example 3),
6-amino-9-{[4-(16-hydroxy-2-methyl-5,8,11,14-tetraoxa-2-azahexadecan-1-yl)phenyl]methyl}-2-(2-methoxyethoxy)-7,9-dihydro-8H-purin-8-one (Example 4), and
4-[(6-amino-2-butoxy-8-oxo-7,8-dihydro-9H-purin-9-yl)methyl]-N-(14-hydroxy-3,6,9,12-tetraoxatetradecan-1-yl)-N-methylbenzamide (Example 5).

### (Item 21)

A pharmaceutical composition comprising the compound of any one of Items 1 to 20 or a pharmaceutically acceptable salt thereof.

### (Item 22)

The pharmaceutical composition of Item 21, which is an emulsion formulation, an oil-based suspension, a hydrogel formulation, or a lipid formulation.

### (Item 23)

The pharmaceutical composition of Item 21, which is an emulsion formulation.

### (Item 24)

The pharmaceutical composition of Item 23, wherein the emulsion formulation is a water-in-oil emulsion.

### (Item 25)

The pharmaceutical composition of Item 24, wherein the emulsion formulation comprises (1) ethyl oleate, octyldodecyl myristate, sorbitan monooleate, glyceryl monooleate, polyoxyethylene hydrogenated castor oil 20, glycerin, and sodium dihydrogen phosphate, or (2) Montanide ISA 51VG.

### (Item 26)

The pharmaceutical composition of Item 21, which is a lipid formulation.

### (Item 27)

The pharmaceutical composition of Item 26, wherein the lipid formulation is a liposome formulation comprising phospholipid.

### (Item 28)

The pharmaceutical composition of Item 26 or 27, wherein the lipid formulation is a liposome formulation comprising sterols.

### (Item 29)

The pharmaceutical composition of Item 28, wherein the sterols is cholesterol.

### (Item 30)

The pharmaceutical composition of any one of Items 27 to 29, wherein the liposome formulation comprises at least one additive selected from the group consisting of inorganic acid, inorganic acid salt, organic acid, organic acid salt, sugars, buffering agent, antioxidant, and polymers.

### (Item 31)

The pharmaceutical composition of any one of Items 21 to 30, which further comprises an antigen.

### (Item 32)

The pharmaceutical composition of Items 31, wherein the antigen is a pathogen-derived antigen or a tumor antigen.

### (Item 33)

The pharmaceutical composition of Item 31, wherein the antigen is a tumor antigen.

### (Item 34)

The pharmaceutical composition of Item 33, wherein the tumor antigen is a tumor antigen peptide.

### (Item 35)

The pharmaceutical composition of Item 34, wherein the tumor antigen peptide comprises at least one peptide or a pharmaceutically acceptable salt thereof which is selected from the group consisting of the following amino acid sequences:
RMFPNAPYL (SEQ ID NO: 1),
ALLPAVPSL (SEQ ID NO: 9),
SLGEQQYSV (SEQ ID NO: 10),
RVPGVAPTL (SEQ ID NO: 11),
VLDFAPPGA (SEQ ID NO: 5),
CMTWNQMNL (SEQ ID NO: 12),
CYTWNQMNL (SEQ ID NO: 2),
TYAGCLSQIF (SEQ ID NO: 15),
formula (4): 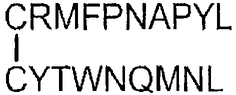 wherein the bond between C-C is disulfide bond, and formula (5): 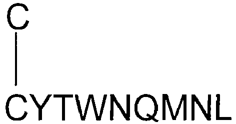
wherein the bond between C-C is disulfide bond; and
at least one peptide or a pharmaceutically acceptable salt thereof which is selected from the group consisting of the following amino acid sequences:
   WAPVLDFAPPGASAYGSL (SEQ ID NO: 3),
   CWAPVLDFAPPGASAYGSL (SEQ ID NO: 13),
   WAPVLDFAPPGASAYGSLC (SEQ ID NO: 14),
   CNKRYFKLSHLQMHSRKHTG (SEQ ID NO: 15),
   CNKRYFKLSHLQMHSRKH (SEQ ID NO: 16),
   CNKRYFKLSHLQMHSRK (SEQ ID NO: 17), and
   KRYFKLSHLQMHSRKH (SEQ ID NO: 4).

### (Item 36)

The pharmaceutical composition of Item 34, wherein the tumor antigen peptide comprises at least one peptide or a pharmaceutically acceptable salt thereof which is selected from the group consisting of the following amino acid sequences:
RMFPNAPYL (SEQ ID NO: 1),
ALLPAVPSL (SEQ ID NO: 9),
SLGEQQYSV (SEQ ID NO: 10),
RVPGVAPTL (SEQ ID NO: 11),
VLDFAPPGA (SEQ ID NO: 5),
CMTWNQMNL (SEQ ID NO: 12),
CYTWNQMNL (SEQ ID NO: 2), and
formula (4): 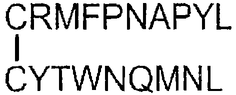
wherein the bond between C-C is disulfide bond, and
at least one peptide or a pharmaceutically acceptable salt thereof which is selected from the group consisting of the following amino acid sequences:
   WAPVLDFAPPGASAYGSL (SEQ ID NO: 3),
   CWAPVLDFAPPGASAYGSL (SEQ ID NO: 13),
   WAPVLDFAPPGASAYGSLC (SEQ ID NO: 14),
   CNKRYFKLSHLQMHSRKHTG (SEQ ID NO: 15),
   CNKRYFKLSHLQMHSRKH (SEQ ID NO: 16),
   CNKRYFKLSHLQMHSRK (SEQ ID NO: 17), and
   KRYFKLSHLQMHSRKH (SEQ ID NO: 4).

### (Item 37)

The pharmaceutical composition of Item 34, wherein the tumor antigen peptide is a combination of a peptide represented by the amino acid sequence of formula (4): 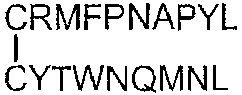 wherein the bond between C-C is disulfide bond, or a pharmaceutically acceptable salt thereof, and
a peptide represented by the amino acid sequence of SEQ ID NO 3: WAPVLDFAPPGASAYGSL, or a pharmaceutically acceptable salt thereof.

### (Item 38)

A vaccine adjuvant comprising a compound of formula (1) : 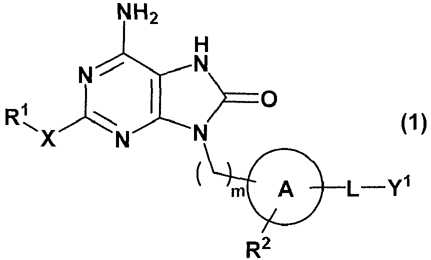 or a pharmaceutically acceptable salt thereof, wherein
X is single bond, oxygen atom, sulfur atom, SO, SO₂, or NR⁶, wherein R⁶ is hydrogen atom or C₁₋₆ alkyl,
R¹ is C₁₋₆ alkyl which may be substituted with 1 - 5 substituents selected independently from the group consisting of halogen, hydroxy, and C₁₋₆ alkoxy,
R² is hydrogen atom, halogen, hydroxy, C₁₋₆ alkyl, C₁₋₆ alkoxy or cyano,
m is an integer of 1 - 5,
A is 5- to 8-membered monocyclic aromatic carbon ring or 4- to 7-membered monocyclic aromatic hetero ring,
L is a linker, and
Y¹ is - (CH₂CH₂O)ₙ-R³, wherein R³ is hydrogen atom or C₁₋₆ alkyl, and n is an integer of 3 - 100.

### (Item 39)

The vaccine adjuvant of Item 38, which is a vaccine adjuvant for cancer vaccine.

### (Item 40)

The vaccine adjuvant of Item 38 or 39, wherein X is single bond, oxygen atom, or NR⁶ (wherein R⁶ is hydrogen atom or C₁₋₆ alkyl) .

### (Item 41)

The vaccine adjuvant of Item 40, wherein X is single bond or oxygen atom.

### (Item 42)

The vaccine adjuvant of any one of Items 38 to 41, wherein R¹ is C₁₋₆ alkyl which may be substituted with 1 - 3 the same or different C₁₋₆ alkoxy.

### (Item 43)

The vaccine adjuvant of Item 42, wherein R¹ is C₁₋₆ alkyl which may be substituted with one C₁₋₆ alkoxy.

### (Item 44)

The vaccine adjuvant of any one of Items 38 to 43, wherein R² is hydrogen atom, halogen, C₁₋₆ alkyl, or C₁₋₆ alkoxy.

### (Item 45)

The vaccine adjuvant of Item 44, wherein R² is hydrogen atom or halogen.

### (Item 46)

The vaccine adjuvant of any one of Items 38 to 45, wherein m is 1.

### (Item 47)

The vaccine adjuvant of any one of Items 38 to 46, wherein A is benzene ring or pyridine ring.

### (Item 48)

The vaccine adjuvant of Item 47, wherein A is pyridine ring.

### (Item 49)

The vaccine adjuvant of any one of Items 38 to 48, wherein
L is -O-, -NR^{Y}-, -C(O)-, -C(O)O-, -OC(O)-, -C(O)NR^{Y}-,-NR^{Y}C(O)-, -CH₂NR^{Y}-, -CH₂O-, -OC(O)O-, -NR⁴C(O)O-, -OC(O)NR^{Y}-, -NR⁹C(O)NR^{Y-}, -OC(S)NR^{Y}-, or -NR⁴C(S)NR^{Y}-, wherein R⁴ is hydrogen atom or C₁₋₆ alkyl, and R^{Y} is hydrogen atom, C₁₋₆ alkyl or Y² wherein Y² is - (CH₂CH₂O) ₚ-R⁵ (wherein R⁵ is hydrogen atom or C₁₋₆ alkyl, and p is an integer of 3 - 100).

### (Item 50)

The vaccine adjuvant of Item 49, wherein L is -CH₂NR^{Y}-.

### (Item 51)

The vaccine adjuvant of Item 50, wherein L is -CH₂NR^{Y}-wherein R^{Y} is hydrogen atom, C₁₋₆ alkyl or Y² wherein Y² is-(CH₂CH₂O)ₚ-R⁵ (wherein R⁵ is hydrogen atom or C₁₋₆ alkyl, and p is an integer of 3 - 100).

### (Item 52)

The vaccine adjuvant of Item 50, wherein L is -CH₂NR^{Y}-wherein R^{Y} is hydrogen atom or C₁₋₆ alkyl.

### (Item 53)

The vaccine adjuvant of any one of Items 38 to 52, wherein Y¹ is - (CH₂CH₂O)ₙ-R³ wherein n is an integer of 3 - 40, and R³ is hydrogen atom or C₁₋₆ alkyl.

### (Item 54)

The vaccine adjuvant of any one of Items 38 to 52, wherein Y¹ is - (CH₂CH₂O) ₙ-R³ wherein n is an integer of 3 - 20, and R³ is hydrogen atom or C₁₋₆ alkyl.

### (Item 55)

The vaccine adjuvant of Item 38 or 39, wherein the compound is a compound of formula (2): 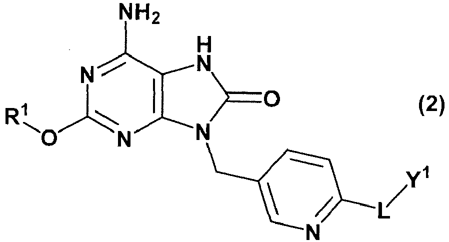 or formula (3): 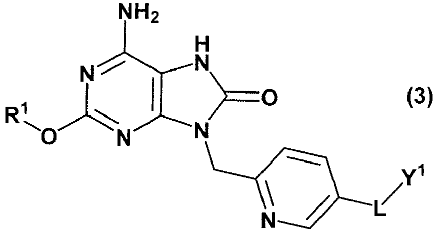 wherein
R¹ is C₁₋₆ alkyl which may be substituted with one C₁₋₆ alkoxy,
L is -CH₂NR^{Y}- wherein R^{Y} is hydrogen atom, C₁₋₆ alkyl or Y², wherein Y² is -(CH₂CH₂O)ₚ-R⁵ (wherein R⁵ is hydrogen atom or C₁₋₆ alkyl, and p is an integer of 3 - 100), and
Y¹ is - (CH₂CH₂O) ₙ-R³ (wherein R³ is hydrogen atom or C₁₋₆ alkyl, and n is an integer of 3 - 40).

### (Item 56)

The vaccine adjuvant of Item 55, wherein the compound is a compound of formula (2): 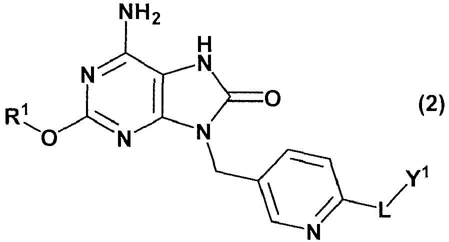 or formula (3): 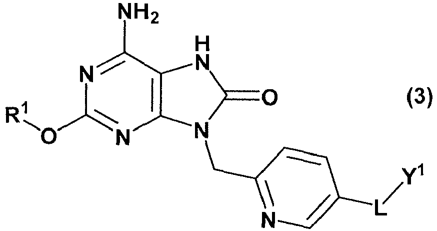 wherein
R¹ is C₁₋₆ alkyl which may be substituted with one C₁₋₆ alkoxy,
L is -CH₂NR^{Y}- wherein R^{Y} is hydrogen atom or C₁₋₆ alkyl,
Y¹ is - (CH₂CH₂O)ₙ-R³ (wherein R³ is hydrogen atom or C₁₋₆ alkyl, and n is an integer of 3 - 20).

### (Item 57)

The vaccine adjuvant of Item 38 or 39, wherein the compound is selected from:
6-amino-2-butoxy-9-{[5-(16-hydroxy-2-methyl-5,8,11,14-tetraoxa-2-azahexadecan-1-yl)pyridin-2-yl]methyl}-7,9-dihydro-8H-purin-8-one (Example 1),
6-amino-2-butoxy-9-{[6-(16-hydroxy-2-methyl-5,8,11,14-tetraoxa-2-azahexadecan-1-yl)pyridin-3-yl]methyl}-7,9-dihydro-8H-purin-8-one (Example 2),
6-amino-2-butoxy-9-{[4-(16-hydroxy-2-methyl-5,8,11,14-tetraoxa-2-azahexadecan-1-yl)phenyl]methyl}-7,9-dihydro-8H-purin-8-one (Example 3),
6-amino-9-{[4-(16-hydroxy-2-methyl-5,8,11,14-tetraoxa-2-azahexadecan-1-yl)phenyl]methyl}-2-(2-methoxyethoxy)-7,9-dihydro-8H-purin-8-one (Example 4),
4-[(6-amino-2-butoxy-8-oxo-7,8-dihydro-9H-purin-9-yl)methyl]-N-(14-hydroxy-3,6,9,12-tetraoxatetradecan-1-yl)-N-methylbenzamide (Example 5),
6-amino-2-butoxy-9-({6-[13-hydroxy-2-(2-{2-[2-(2-hydroxyethoxy)ethoxy]ethoxy}ethyl)-5,8,11-trioxa-2-azatridecan-l-yl]pyridin-3-yl}methyl)-7,9-dihydro-8H-purin-8-one (Example 6),
6-amino-2-butoxy-9-{[6-(31-hydroxy-2-methyl-5,8,11,14,17,20,23,26,29-nonaoxa-2-azahentriacontan-1-yl)pyridin-3-yl]methyl}-7,9-dihydro-8H-purin-8-one (Example 7),
6-amino-2-butoxy-9-{[6-(73-hydroxy-2-methyl-5,8,11,14,17,20,23,26,29,32,35,38,41,44,47,50,53,56,59,62,6 5,68,71-tricosaoxa-2-azatriheptacontan-1-yl)pyridin-3-yl]methyl}-7,9-dihydro-8H-purin-8-one (Example 8), and
6-amino-2-butoxy-9-([6-(109-hydroxy-2-methyl-5,8,11,14,17,20,23,26,29,32,35,38,41,44,47,50,53,56,59,62,6 5,68,71,74,77,80,83,86,89,92,95,98,101,104,107-pentatriacontaoxa-2-azanonahectan-1-yl)pyridin-3-yl]methyl}-7,9-dihydro-8H-purin-8-one (Example 9), or a pharmaceutically acceptable salt thereof.

### (Item 58)

The vaccine adjuvant of Item 38 or 39, wherein the compound is selected from:
6-amino-2-butoxy-9-{[5-(16-hydroxy-2-methyl-5,8,11,14-tetraoxa-2-azahexadecan-1-yl)pyridin-2-yl]methyl}-7,9-dihydro-8H-purin-8-one (Example 1),
6-amino-2-butoxy-9-{[6-(16-hydroxy-2-methyl-5,8,11,14-tetraoxa-2-azahexadecan-1-yl)pyridin-3-yl]methyl}-7,9-dihydro-8H-purin-8-one (Example 2),
6-amino-2-butoxy-9-{[4-(16-hydroxy-2-methyl-5,8,11,14-tetraoxa-2-azahexadecan-1-yl)phenyl]methyl)-7,9-dihydro-8H-purin-8-one (Example 3),
6-amino-9-{[4-(16-hydroxy-2-methyl-5,8,11,14-tetraoxa-2-azahexadecan-1-yl)phenyl]methyl}-2-(2-methoxyethoxy)-7,9-dihydro-8H-purin-8-one (Example 4), and
4-[(6-amino-2-butoxy-8-oxo-7,8-dihydro-9H-purin-9-yl)methyl]-N-(14-hydroxy-3,6,9,12-tetraoxatetradecan-1-yl)-N-methylbenzamide (Example 5) , or
a pharmaceutically acceptable salt thereof.

### (Item 59)

The compound of any one of Items 1 to 20, 38, and 40 to 58 or a pharmaceutically acceptable salt thereof, which is used as a vaccine adjuvant.

### (Item 60)

The compound of any one of Items 1 to 20, 38, and 40 to 58 or a pharmaceutically acceptable salt thereof, which is used as a vaccine adjuvant for cancer vaccine.

### (Item 61)

CTL inducer comprising the compound of any one of Items 38, and 40 to 58 or a pharmaceutically acceptable salt thereof.

### (Item 62)

An immunostimulant comprising the compound of any one of Items 38, and 40 to 58 or a pharmaceutically acceptable salt thereof.

### (Item 63)

A method for inducing CTL in mammal, comprising administering the compound of any one of Items 38, and 40 to 58 or a pharmaceutically acceptable salt thereof to the mammal.

### (Item 64)

A method for enhancing the CTL induction in mammal, comprising administering the compound of any one of Items 38, and 40 to 58 or a pharmaceutically acceptable salt thereof to the mammal.

### (Item 65)

A method for enhancing specific immune response in mammal to an antigen, comprising administering the compound of any one of Items 38, and 40 to 58 or a pharmaceutically acceptable salt thereof to the mammal.

### (Item 66)

Use of the compound of any one of Items 38, and 40 to 58 or a pharmaceutically acceptable salt thereof in the preparation of a vaccine adjuvant.

### (Item 67)

Use of the compound of any one of Items 38, and 40 to 58 or a pharmaceutically acceptable salt thereof in the preparation of a vaccine adjuvant for cancer vaccine.

### (Item 68)

A kit comprising
a) the compound of any one of Items 38, and 40 to 58 or a pharmaceutically acceptable salt thereof, or a pharmaceutical composition comprising the compound of any one of Items 38, and 40 to 58 or a pharmaceutically acceptable salt thereof; and
b) an antigen or a pharmaceutical composition comprising an antigen.

### (Item 69)

A kit comprising
a) the compound of any one of Items 38 to 58 or a pharmaceutically acceptable salt thereof, or a pharmaceutical composition comprising the compound of any one of Items 38 to 58 or a pharmaceutically acceptable salt thereof; and
b) a tumor antigen or a pharmaceutical composition comprising a tumor antigen.

### (Item 70)

A kit comprising
a) the compound of any one of Items 38, and 40 to 58 or a pharmaceutically acceptable salt thereof, or a pharmaceutical composition comprising the compound of any one of Items 38, and 40 to 58 or a pharmaceutically acceptable salt thereof; and
b) a pathogen-derived antigen or a pharmaceutical composition comprising a pathogen-derived antigen.

### BRIEF DESCRIPTION OF DRAWINGS

Fig. 1-A shows the results of Test 3, i.e., which shows the effect of the compounds prepared in Example 2 and Example 3 to IFNα2 yield on human peripheral blood mononuclear cell.
Fig. 1-B shows the results of Test 3, i.e., which shows the effect of the compounds prepared in Example 2 and Example 3 to GM-CSF yield on human peripheral blood mononuclear cell.
Fig. 1-C shows the results of Test 3, i.e., which shows the effect of the compounds prepared in Example 2 and Example 3 to IFNγ yield on human peripheral blood mononuclear cell.
Fig. 1-D shows the results of Test 3, i.e., which shows the effect of the compounds prepared in Example 2 and Example 3 to IL-12p40 yield on human peripheral blood mononuclear cell.
Fig. 1-E shows the results of Test 3, i.e., which shows the effect of the compounds prepared in Example 2 and Example 3 to IL-1β yield on human peripheral blood mononuclear cell.
Fig. 1-F shows the results of Test 3, i.e., which shows the effect of the compounds prepared in Example 2 and Example 3 to IL-6 yield on human peripheral blood mononuclear cell.
Fig. 1-G shows the results of Test 3, i.e., which shows the effect of the compounds prepared in Example 2 and Example 3 to IP-10 yield on human peripheral blood mononuclear cell.
Fig. 1-H shows the results of Test 3, i.e., which shows the effect of the compounds prepared in Example 2 and Example 3 to TNFα yield on human peripheral blood mononuclear cell.
Fig. 2-A shows the results of Test 4, i.e., which shows the effect of the compound prepared in Example 3 to IP-10 yield on mouse bone marrow-derived dendritic cell.
Fig. 2-B shows the results of Test 4, i.e., which shows the effect of the compound prepared in Example 3 to IL-12p70 yield on mouse bone marrow-derived dendritic cell.
Fig. 2-C shows the results of Test 4, i.e., which shows the effect of the compound prepared in Example 3 to IL-12p40 yield on mouse bone marrow-derived dendritic cell.
Fig. 2-D shows the results of Test 4, i.e., which shows the effect of the compound prepared in Example 3 to IL-6 yield on mouse bone marrow-derived dendritic cell.
Fig. 2-E shows the results of Test 4, i.e., which shows the effect of the compound prepared in Example 3 to IL-1β yield on mouse bone marrow-derived dendritic cell.
Fig. 2-F shows the results of Test 4, i.e., which shows the effect of the compound prepared in Example 3 to MIP-1α yield on mouse bone marrow-derived dendritic cell.
Fig. 2-G shows the results of Test 4, i.e., which shows the effect of the compound prepared in Example 3 to MIP-1β yield on mouse bone marrow-derived dendritic cell.
Fig. 2-H shows the results of Test 4, i.e., which shows the effect of the compound prepared in Example 3 to Rantes yield on mouse bone marrow-derived dendritic cell.

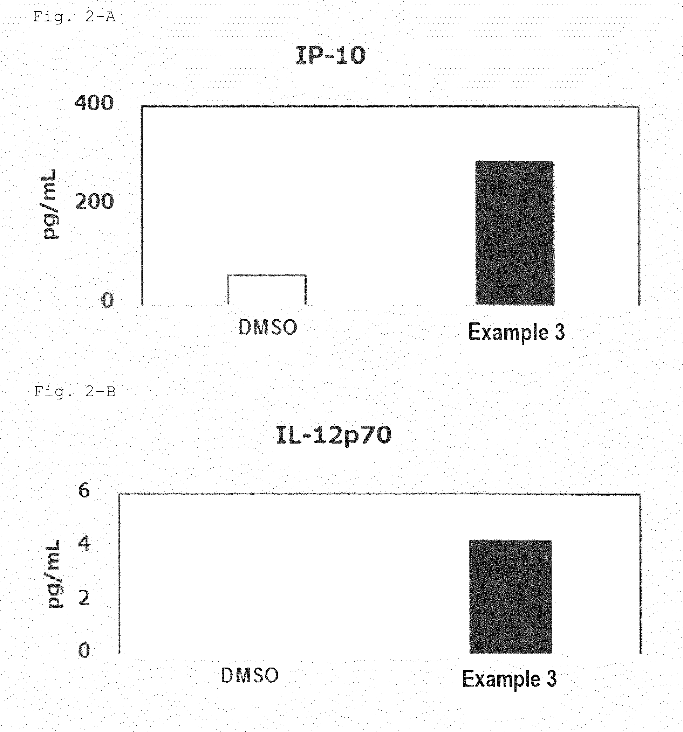

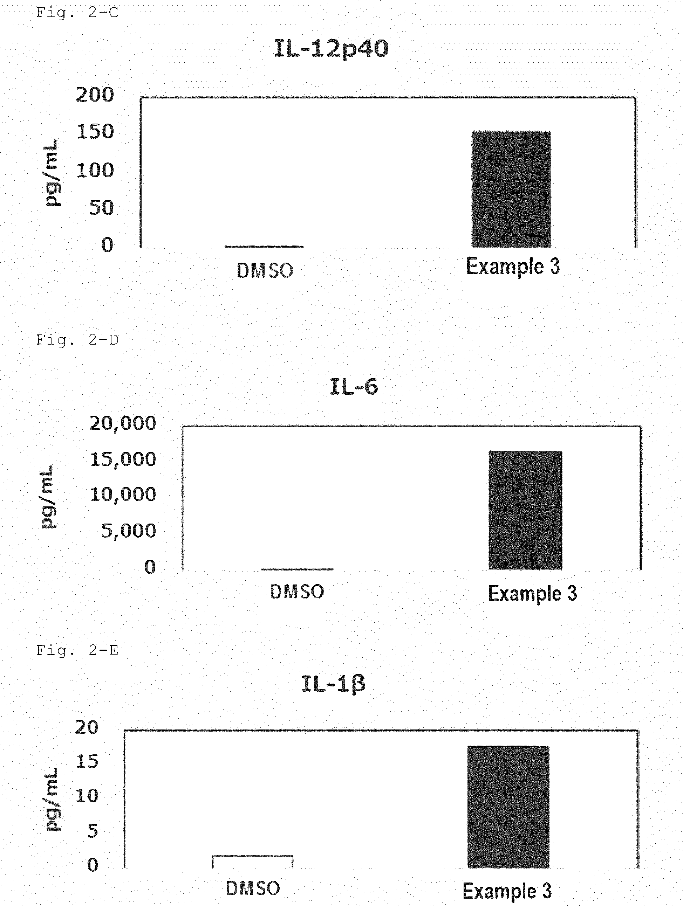

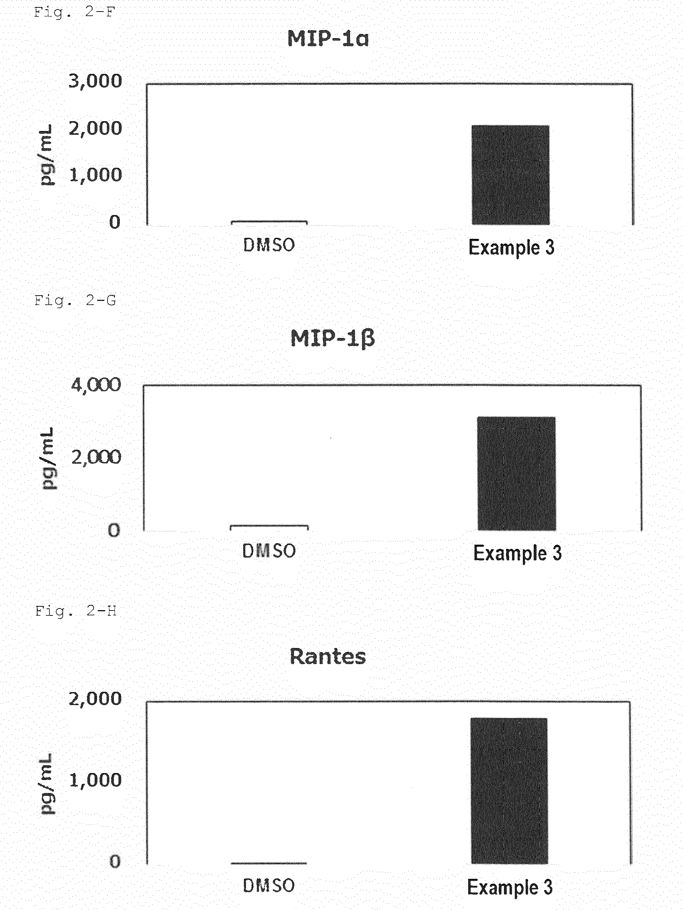

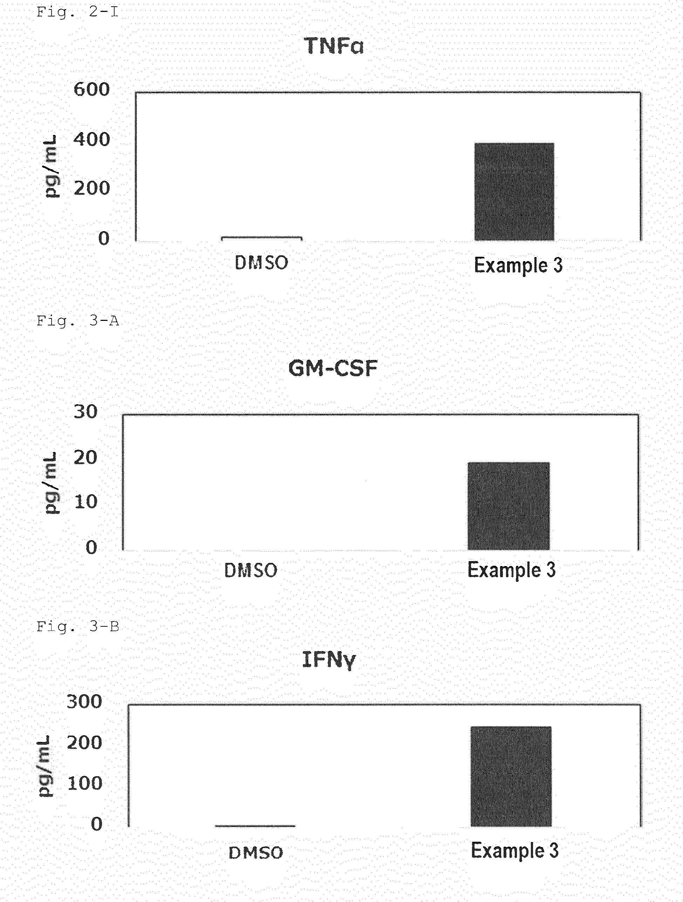

Fig. 2-I shows the results of Test 4, i.e., which shows the effect of the compound prepared in Example 3 to TNFα yield on mouse bone marrow-derived dendritic cell.

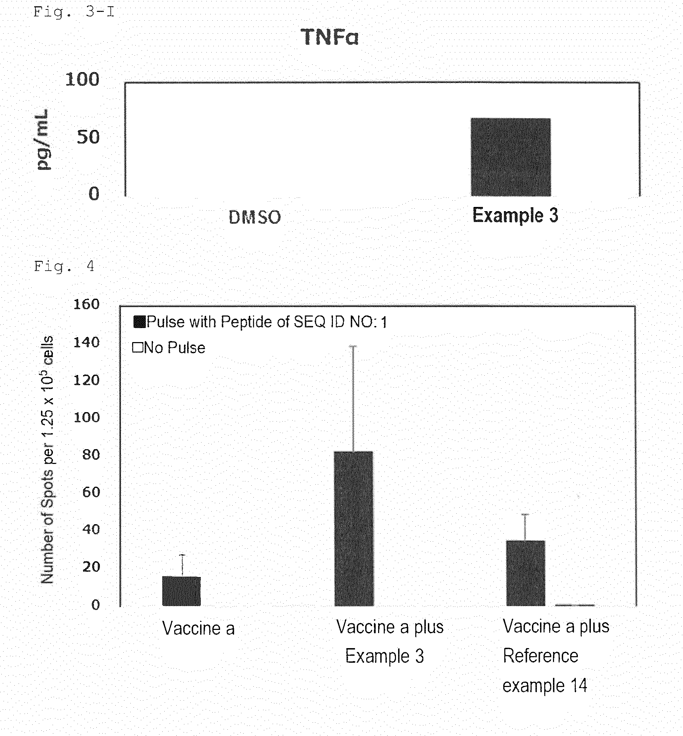

Fig. 3-A shows the results of Test 5, i.e., which shows the effect of the compound prepared in Example 3 to GM-CSF yield on mouse splenocyte.
Fig. 3-B shows the results of Test 5, i.e., which shows the effect of the compound prepared in Example 3 to IFNγ yield on mouse splenocyte.

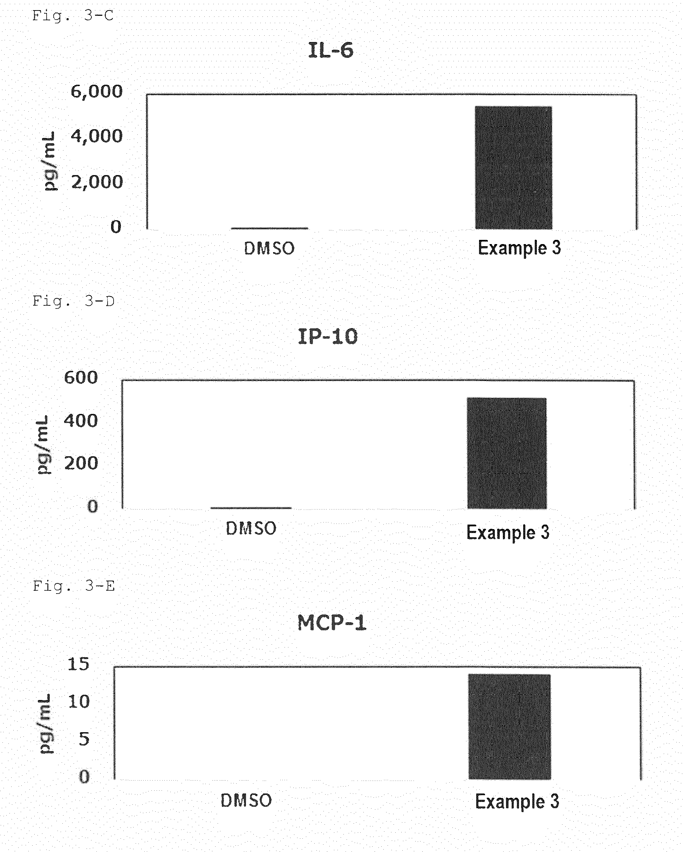

Fig. 3-C shows the results of Test 5, i.e., which shows the effect of the compound prepared in Example 3 to IL-6 yield on mouse splenocyte.
Fig. 3-D shows the results of Test 5, i.e., which shows the effect of the compound prepared in Example 3 to IP-10 yield on mouse splenocyte.
Fig. 3-E shows the results of Test 5, i.e., which shows the effect of the compound prepared in Example 3 to MCP-1 yield on mouse splenocyte.

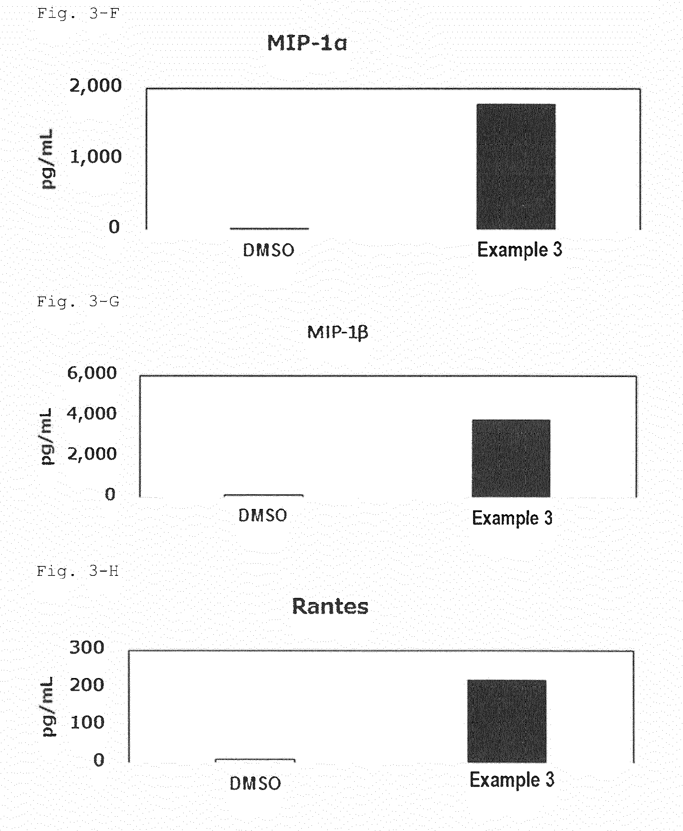

Fig. 3-F shows the results of Test 5, i.e., which shows the effect of the compound prepared in Example 3 to MIP-1α yield on mouse splenocyte.
Fig. 3-G shows the results of Test 5, i.e., which shows the effect of the compound prepared in Example 3 to MIP-1β yield on mouse splenocyte.
Fig. 3-H shows the results of Test 5, i.e., which shows the effect of the compound prepared in Example 3 to Rantes yield on mouse splenocyte.
Fig. 3-I shows the results of Test 5, i.e., which shows the effect of the compound prepared in Example 3 to TNFα yield on mouse splenocyte.
Fig. 4 shows the results of Test 6, i.e., a vaccine was prepared by adding the compound prepared in Example 3 or Reference example 14 to a cocktail vaccine comprising Compound of formula 4 and Peptide SEQ ID NO. 3 with a preliminarily-emulsified composition; and each vaccine was tested about *in vivo* CTL induction for SEQ ID No. 1 with a HLA-A^{∗}02:01 transgenic mouse by IFNγ ELISPOT assay. The results are shown in Fig. 4.

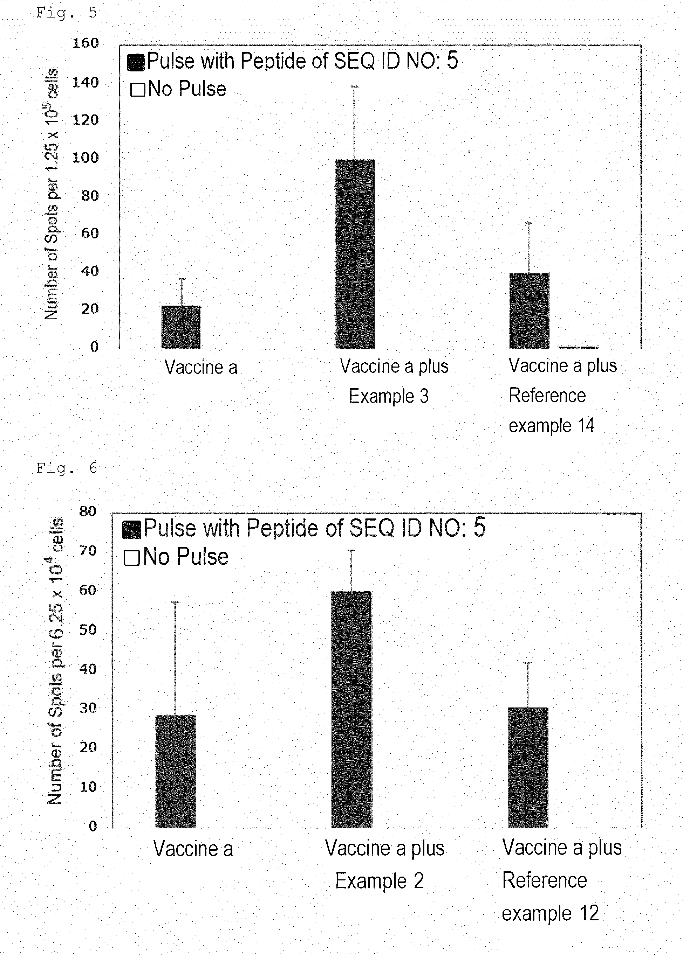

Fig. 5 shows the results of Test 6, i.e., a vaccine was prepared by adding the compound prepared in Example 3 or Reference example 14 to a cocktail vaccine comprising Compound of formula 4 and Peptide SEQ ID NO. 3 with a preliminarily-emulsified composition; and each vaccine was tested about *in vivo* CTL induction for SEQ ID No. 5 with a HLA-A^{∗}02:01 transgenic mouse by IFNγ ELISPOT assay. The results are shown in Fig. 5.
Fig. 6 shows the results of Test 7, i.e., a vaccine was prepared by adding the compound prepared in Example 2 or Reference example 12 to a cocktail vaccine comprising Compound of formula 4 and Peptide SEQ ID NO. 3 with a preliminarily-emulsified composition; and each vaccine was tested about *in vivo* CTL induction for SEQ ID No. 5 with a HLA-A^{∗}02:01 transgenic mouse by IFNγ ELISPOT assay. The results are shown in Fig. 6.

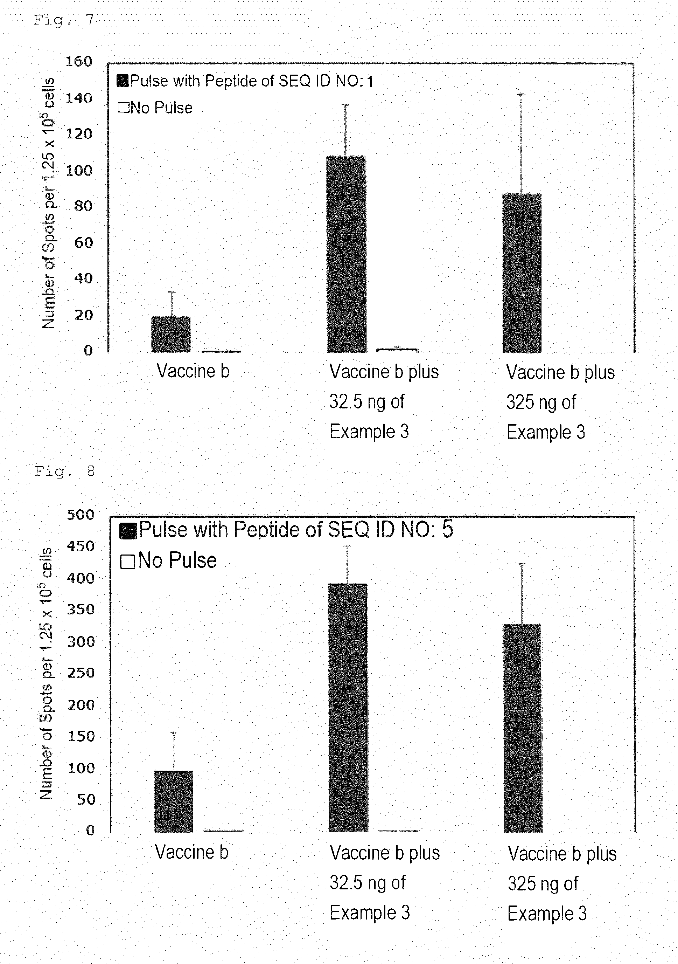

Fig. 7 shows the results of Test 8, i.e., a vaccine was prepared by adding the compound prepared in Example 3 to a cocktail vaccine comprising Compound of formula 4 and Peptide SEQ ID NO. 3 with Montanide ISA 51 VG; and the vaccine was tested about *in vivo* CTL induction for SEQ ID No. 1 with a HLA-A^{∗}02:01 transgenic mouse by IFNγ ELISPOT assay. The results are shown in Fig. 7.
Fig. 8 shows the results of Test 8, i.e., a vaccine was prepared by adding the compound prepared in Example 3 to a cocktail vaccine comprising Compound of formula 4 and Peptide SEQ ID NO. 3 with Montanide ISA 51 VG; and the vaccine was tested about *in vivo* CTL induction for SEQ ID No. 5 with a HLA-A^{∗}02:01 transgenic mouse by IFNγ ELISPOT assay. The results are shown in Fig. 8.

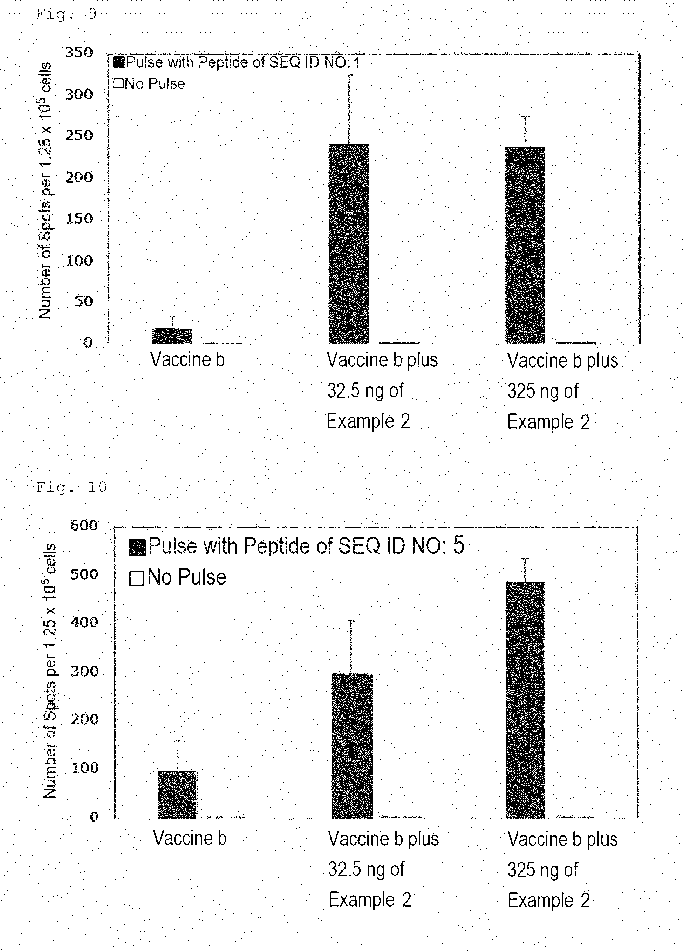

Fig. 9 shows the results of Test 8, i.e., a vaccine was prepared by adding the compound prepared in Example 2 to a cocktail vaccine comprising Compound of formula 4 and Peptide SEQ ID NO. 3 with Montanide ISA 51 VG; and the vaccine was tested about *in vivo* CTL induction for SEQ ID No. 1 with a HLA-A^{∗}02:01 transgenic mouse by IFNγ ELISPOT assay. The results are shown in Fig. 9.
Fig. 10 shows the results of Test 8, i.e., a vaccine was prepared by adding the compound prepared in Example 2 to a cocktail vaccine comprising Compound of formula 4 and Peptide SEQ ID NO. 3 with Montanide ISA 51 VG; and the vaccine was tested about *in vivo* CTL induction for SEQ ID No. 5 with a HLA-A^{∗}02:01 transgenic mouse by IFNγ ELISPOT assay. The results are shown in Fig. 10.

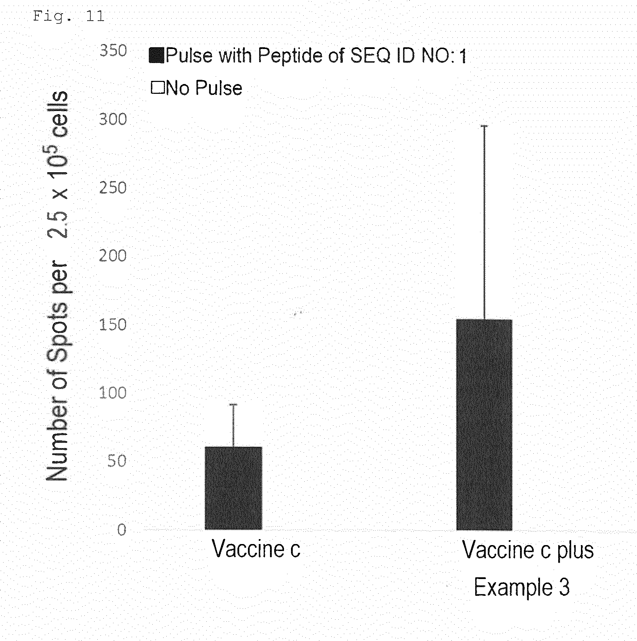

Fig. 11 shows the results of Test 9, i.e., a vaccine was prepared by adding the compound prepared in Example 3 to a vaccine comprising Peptide SEQ ID NO. 1 and a preliminarily-emulsified composition; and the vaccine was tested about in *vivo* CTL induction for SEQ ID No. 1 with a HLA-A^{∗}02:01 transgenic mouse by IFNγ ELISPOT assay. The results are shown in Fig. 11.

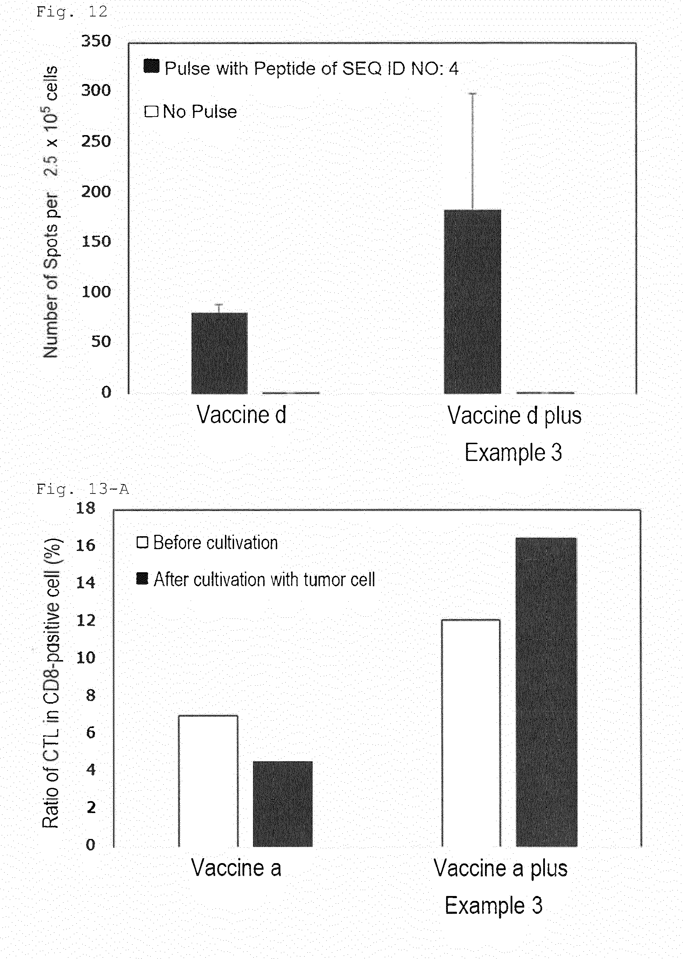

Fig. 12 shows the results of Test 10, i.e., a vaccine was prepared by adding the compound prepared in Example 3 to a cocktail vaccine comprising Peptide SEQ ID No. 1 and Peptide SEQ ID No. 4 with a preliminary emulsified composition; and the vaccine was tested about *in vivo* helper T-cell induction for SEQ ID No. 4 with a HLA-A^{∗}02:01/HLA-DRB1^{∗}01:01 transgenic mouse by IFNγ ELISPOT assay. The results are shown in Fig. 12.
Fig. 13-A shows the results of Test 11, i.e., a vaccine was prepared by adding the compound prepared in Example 3 to a cocktail vaccine comprising Compound of formula 4 and Peptide SEQ ID NO. 3 with a preliminarily-emulsified composition; the vaccine was administered to a HLA-A^{∗}02:01 transgenic mouse; the splenocyte was derived from the mouse; and the splenocyte was analyzed about the ratio of CTLs specific to Peptide SEQ ID NO. 1 before/after the three-day cultivation in the presence of Peptide SEQ ID NO. 1 and tumor cells with a flow cytometry. The results are shown in Fig. 13-A.

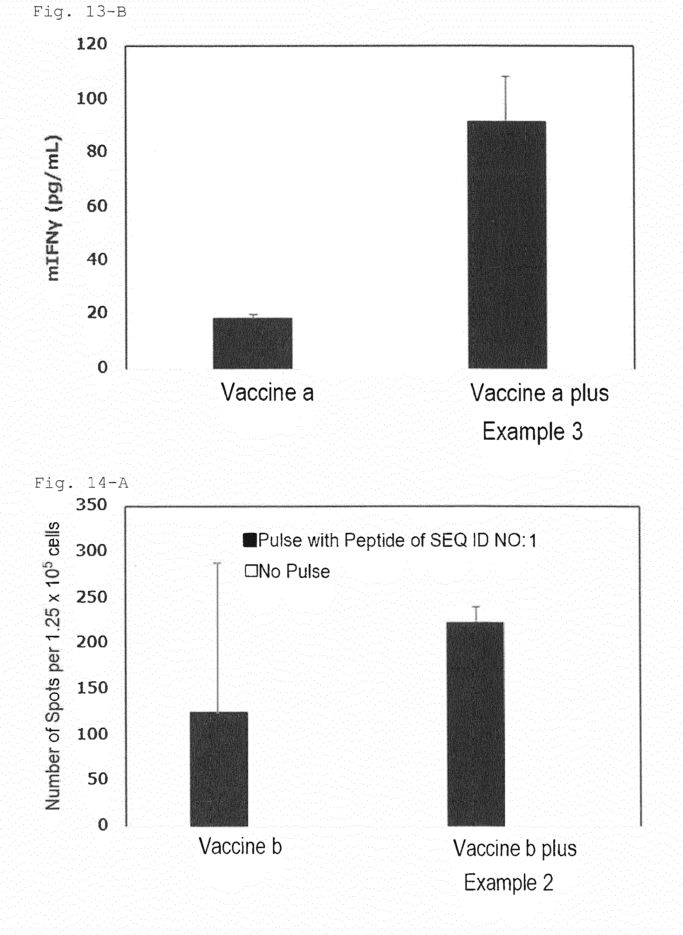

Fig. 13-B shows the results of Test 11, i.e., a vaccine was prepared by adding the compound prepared in Example 3 to a cocktail vaccine comprising Compound of formula 4 and Peptide SEQ ID No. 3 with a preliminarily-emulsified composition; the vaccine was administered to a HLA-A^{∗}02:01 transgenic mouse; the splenocyte was derived from the mouse; and the splenocyte was analyzed about the amount of the produced IFN-γ after the three-day cultivation in the presence of Peptide SEQ ID No. 1 and tumor cells. The results are shown in Fig. 13-B.
Fig. 14-A shows the results of Test 12, i.e., a vaccine was prepared by adding the compound prepared in Example 2 to a cocktail vaccine comprising Compound of formula 4 and Peptide SEQ ID No. 3 with Montanide ISA 51 VG; and the vaccine was tested about *in vivo* CTL induction for SEQ ID No. 1 with a HLA-A^{∗}02:01 transgenic mouse by IFNγ ELISPOT assay. The results are shown in Fig. 14-A.

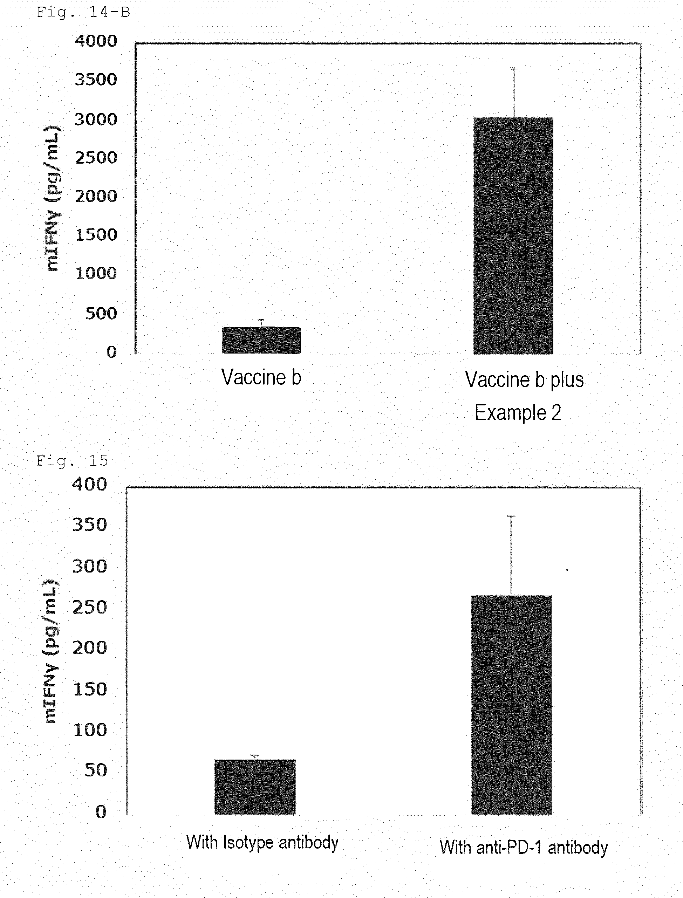

Fig. 14-B shows the results of Test 12, i.e., a vaccine was prepared by adding the compound prepared in Example 2 to a cocktail vaccine comprising Compound of formula 4 and Peptide SEQ ID No. 3 with Montanide ISA 51 VG; the vaccine was administered to a HLA-A^{∗}02:01 transgenic mouse; the splenocyte was derived from the mouse; and the splenocyte was analyzed about the amount of the produced IFN-γ after the three-day cultivation in the presence of Peptide SEQ ID No. 1 and tumor cells. The results are shown in Fig. 14-B.
Fig. 15 shows the results of Test 13, i.e., a vaccine was prepared by adding the compound prepared in Example 2 to a cocktail vaccine comprising Compound of formula 4 and Peptide SEQ ID NO. 3 with Montanide ISA 51 VG; the vaccine was administered to a HLA-A^{∗}02:01 transgenic mouse; the splenocyte was derived from the mouse; the splenocyte was cultured with an isotype control antibody or anti-PD-1 antibody in the presence of Peptide SEQ ID No. 1 and tumor cells for three days; and the amount of the produced IFN-γ was analyzed. The results are shown in Fig. 15.

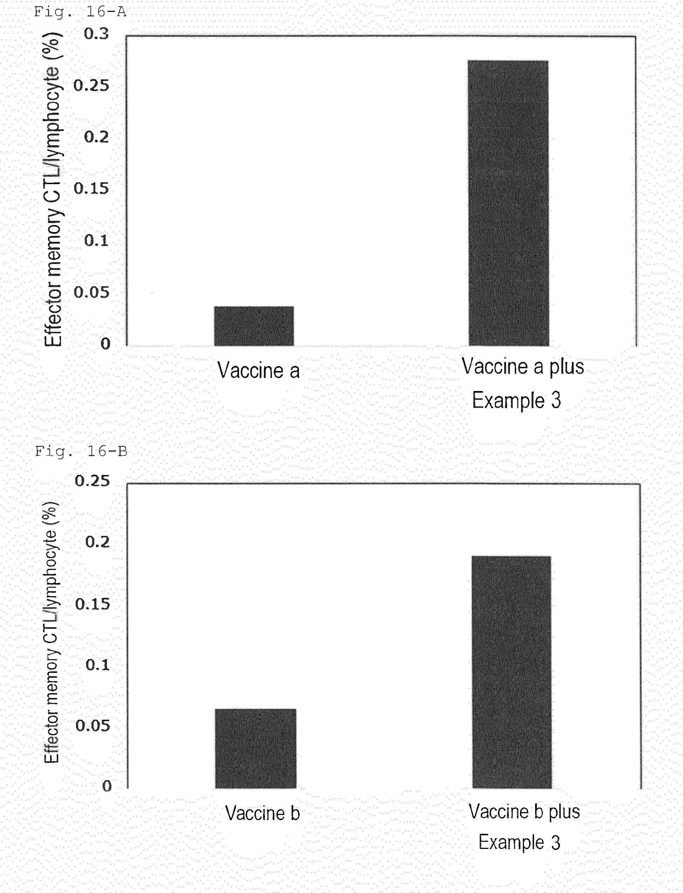

Fig. 16-A shows the results of Test 14, i.e., a vaccine was prepared by adding the compound prepared in Example 3 to a cocktail vaccine comprising Compound of formula 4 and Peptide SEQ ID NO. 3 with a preliminarily-emulsified composition; the vaccine was administered to a HLA-A^{∗}02:01 transgenic mouse; the splenocyte was derived from the mouse; and the splenocyte was analyzed about the frequency of effector memory CTL specific to Peptide SEQ ID No. 1 with a flow cytometry. The results are shown in Fig. 16-A.
Fig. 16-B shows the results of Test 14, i.e., a vaccine was prepared by adding the compound prepared in Example 3 to a cocktail vaccine comprising Compound of formula 4 and Peptide SEQ ID NO. 3 with Montanide ISA 51 VG; the vaccine was administered to a HLA-A^{∗}02:01 transgenic mouse; the splenocyte was derived from the mouse; and the splenocyte was analyzed about the frequency of effector memory CTL specific to Peptide SEQ ID NO. 1 with a flow cytometry. The results are shown in Fig. 16-B.

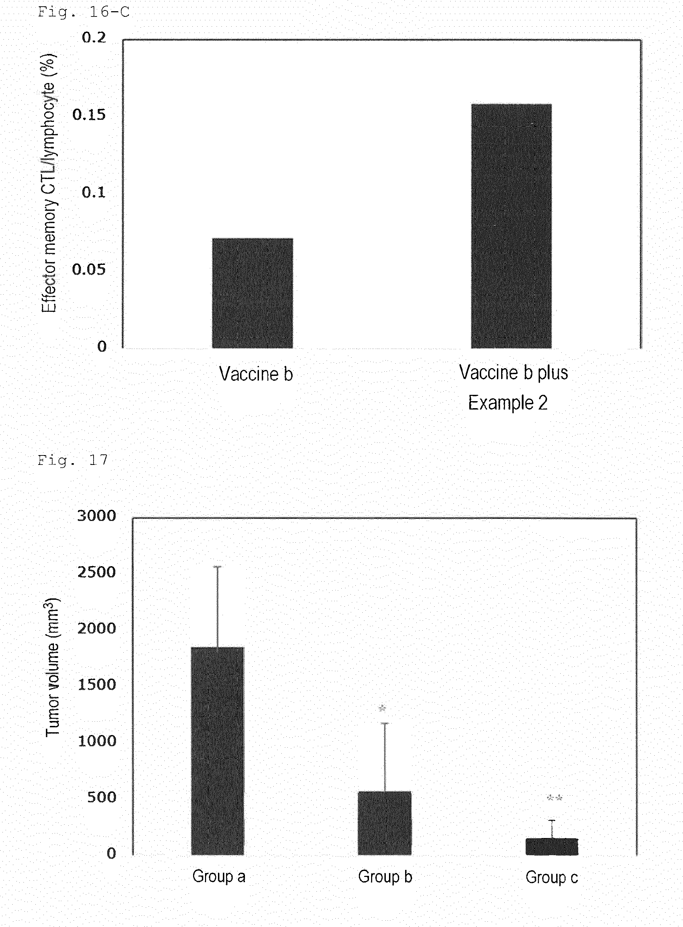

Fig. 16-C shows the results of Test 14, i.e., a vaccine was prepared by adding the compound prepared in Example 2 to a cocktail vaccine comprising Compound of formula 4 and Peptide SEQ ID NO. 3 with Montanide ISA 51 VG; the vaccine was administered to a HLA-A^{∗}02:01 transgenic mouse; the splenocyte was derived from the mouse; and the splenocyte was analyzed about the frequency of effector memory CTL specific to Peptide SEQ ID NO. 1 with a flow cytometry. The results are shown in Fig. 16-C.
Fig. 17 shows the results of Test 15, i.e., a vaccine was prepared by adding the compound prepared in Example 2 to a cocktail vaccine comprising Compound of formula 4 and Peptide SEQ ID No. 3 with Montanide ISA 51 VG; seven days before the tumor transplantation of MCA-A24/Kb-WT1 tumor cells to a HLA-A^{∗}24:02 transgenic mouse and seven days after the transplantation, the vaccine was administered to the mouse; and 27 days after the transplantation, the tumor volume was measured. The results are shown in Fig. 17.

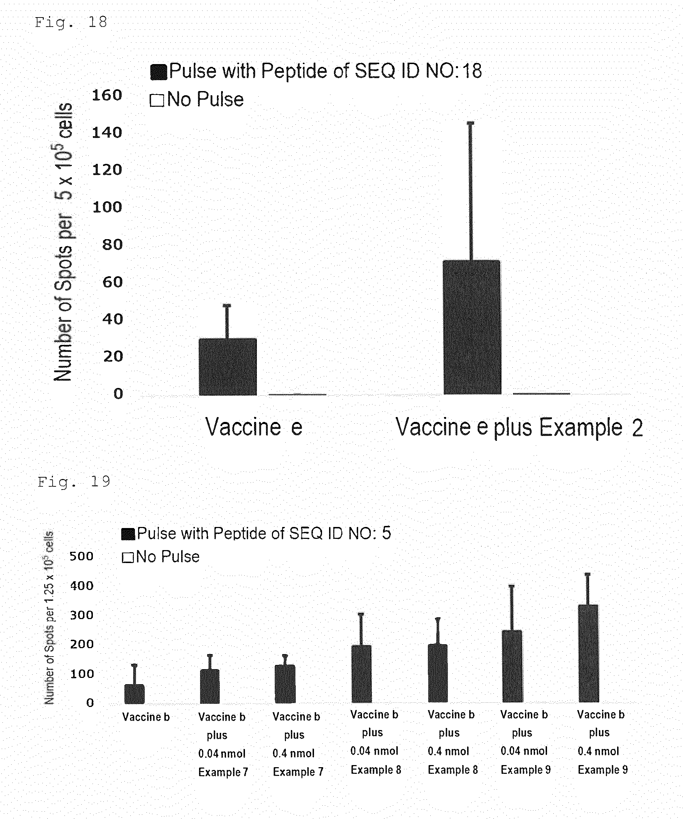

Fig. 18 shows the results of Test 16, i.e., a vaccine was prepared by adding the compound prepared in Example 2 to a vaccine comprising Peptide SEQ ID NO. 18 and Montanide ISA 51 VG; and the vaccine was tested about *in vivo* CTL induction for SEQ ID No. 18 with a HLA-A^{∗}24:02 transgenic mouse by IFNγ ELISPOT assay. The results are shown in Fig. 18.
Fig. 19 shows the results of Test 17, i.e., a vaccine was prepared by adding the compound prepared in Example 7, 8, or 9 to a cocktail vaccine comprising Compound of formula 4 and Peptide SEQ ID No. 3 with Montanide ISA 51 VG; and the vaccine was tested about *in vivo* CTL induction for SEQ ID No. 5 with a HLA-A^{∗}02:01 transgenic mouse by IFNγ ELISPOT assay. The results are shown in Fig. 19.

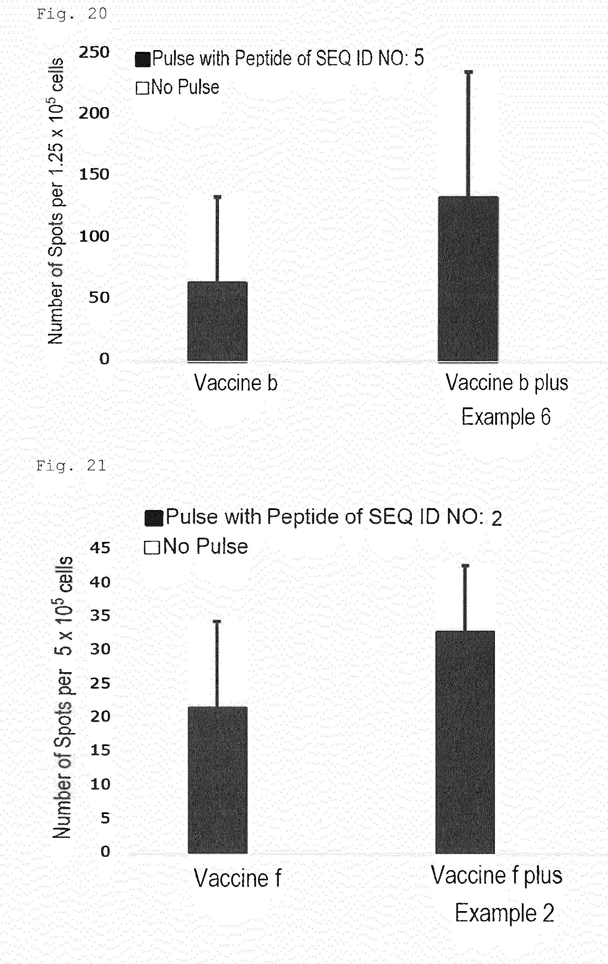

Fig. 20 shows the results of Test 18, i.e., a vaccine was prepared by adding the compound prepared in Example 6 to a cocktail vaccine comprising Compound of formula 4 and Peptide SEQ ID No. 3 with Montanide ISA 51 VG; and the vaccine was tested about *in vivo* CTL induction for SEQ ID No. 5 with a HLA-A^{∗}02:01 transgenic mouse by IFNγ ELISPOT assay. The results are shown in Fig. 20.
Fig. 21 shows the results of Test 19, i.e., a vaccine was prepared by adding the compound prepared in Example 2 to a vaccine comprising Compound of formula 5 and a preliminarily-emulsified composition; and the vaccine was tested about *in vivo* CTL induction for SEQ ID No. 2 with a HLA-A^{∗}24: 02 transgenic mouse by IFNγ ELISPOT assay. The results are shown in Fig. 21.

### DESCRIPTION OF EMBODIMENTS

Hereinafter, terms used herein are explained as follows.

The number of substituents that are defined posterior to "optionally-substituted" or "substituted" should not be limited, if it is possible to be substituted. Unless otherwise specified, the definition of each substituent group also extends over the case of partially-including the substituent group or the case of the substituent group existing on another substituent group.

The "halogen" used herein includes, for example, fluorine, chlorine, bromine, and iodine. It is preferably fluorine or chlorine, more preferably fluorine.

The "C₁₋₆ alkyl" means straight or branched chain saturated hydrocarbon group having 1 to 6 carbon atoms. The C₁₋₆ alkyl includes preferably "C₁₋₄ alkyl", more preferably "C₁₋₃ alkyl". The "C₁₋₆ alkyl" includes, for example, methyl, ethyl, propyl, 1-methylethyl, butyl, 2-methylpropyl, 1-methylpropyl, 1,1-dimethylethyl, pentyl, 3-methylbutyl, 2-methylbutyl, 2,2-dimethylpropyl, 1-ethylpropyl, 1,1-dimethylpropyl, hexyl, 4-methylpentyl, 3-methylpentyl, 2-methylpentyl, and 1-methylpentyl, and the "C₁₋₄ alky" includes the examples of the "C₁₋₆ alkyl" provided that the number of carbon atoms is 1 - 4. The "C₁₋₃ alkyl" includes the examples of the "C₁₋₆ alkyl" provided that the number of carbon atoms is 1 - 3.

The "C₁₋₆ alkoxy" means "C₁₋₆ alkyloxy", and the part "C₁₋₆ alkyl" is as defined in the said "C₁₋₆ alkyl". The "C₁₋₆ alkoxy" includes preferably "C₁₋₄ alkoxy", more preferably "C₁₋₃ alkoxy". The "C₁₋₆ alkoxy" includes, for example, methoxy, ethoxy, propoxy, 1-methylethoxy, butoxy, 2-methylpropoxy, 1-methylpropoxy, 1,1-dimethylethoxy, pentyloxy, 3-methylbutoxy, 2-methylbutoxy, 2,2-dimethylpropoxy, 1-ethylpropoxy, 1,1-dimethylpropoxy, hexyloxy, 4-methylpentyloxy, 3-methylpentyloxy, 2-methylpentyloxy, 1-methylpentyloxy, 3,3-dimethylbutoxy, 2,2-dimethylbutoxy, 1,1-dimethylbutoxy, and 1,2-dimethylbutoxy, and the "C₁₋₄ alkoxy" includes the examples of the "C₁₋₆ alkoxy" provided that the number of carbon atoms is 1 - 4. The "C₁₋₃ alkoxy" includes the examples of the "C₁₋₆ alkoxy" provided that the number of carbon atoms is 1 - 3.

The "linker" means a bivalent group having two binding sites in the functional group. The bivalent group includes, for example, C₁₋₆ alkylene, C₂₋₇ alkenylene, C₂₋₇ alkynylene, C₃₋₁₀ cycloalkylene, C₆₋₁₀ arylene, C₅₋₁₀ heteroarylene, ether, amine, carbonyl, ester, amido, carbonate, carbamate, thiocarbamate, and thiourea. And, a bivalent group prepared by optionally-combining these exemplified bivalent groups may be used herein. The linker includes, preferably, -O-, -NR^{Y}-, -C(O)-, -C(O)O-, -OC(O)-, -C(O)NR^{Y}-, -NR^{Y}C(O)-,-CH₂NR^{Y}-, -CH₂O-, -OC(O)O-, -NR⁴C(O)O-, -OC (O)NR^{Y}-,-NR⁴C(O)NR^{Y}-, -OC(S)NR^{Y}-, and -NR⁴C(S)NR^{Y}-, wherein R^{Y} and R⁴ are as defined in Item 11, and more preferably -CH₂NR^{Y}-. As for the two binding sites in these exemplified linkers, the left binding site is attached to Ring A in the compound of formula (1), and the right binding site is attached to Y¹ in the compound of formula (1). Specifically, when linker L is "-CH₂NR^{Y}-", the compound of formula (1) is represented as the following structure.

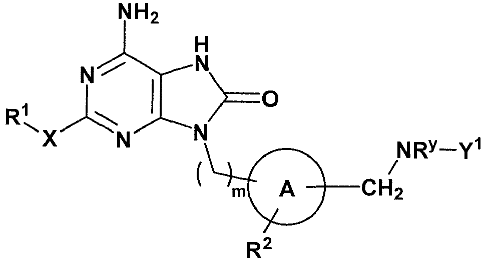

The "C₁₋₆ alkylene" means straight or branched chain saturated hydrocarbon group having 1 to 6 carbon atoms. The "C₁₋₆ alkylene" includes, for example, methylene, ethylene, propylene, 1-methylethylene, butylene, 2-methylpropylene, 1-methylpropylene, 1,1-dimethylethylene, pentylene, 3-methylbutylene, 2-methylbutylene, 2,2-dimethylpropylene, 1-ethylpropylene, 1,1-dimethylpropylene, hexylene, 4-methylpentylene, and 3-methylpentylene, and preferably methylene and ethylene.

The "C₂₋₇ alkenylene" means straight or branched chain unsaturated hydrocarbon group having 2 to 7 carbon atoms and 1 to 3 double bonds. The "C₂₋₇ alkenylene" includes, for example, vinylene, propenylene, methylpropenylene, butenylene, methylbutenylene, pentenylene, hexenylene, and heptenylene, and preferably vinylene and propenylene.

The "C₂₋₇ alkynylene" means straight or branched chain unsaturated hydrocarbon group having 2 to 7 carbon atoms and one triple bond. The "C₂₋₇ alkynylene" includes, for example, ethynylene, propynylene, methyl propynylene, butynylene, methylbutynylene, pentynylene, hexynylene, and heptynylene, and preferably ethynylene and propynylene.

The "C₃₋₁₀ cycloalkylene" means cyclic alkylene having 3 to 10 carbon atoms, which may have a bridged structure. The "C₃₋₁₀ cycloalkylene" includes, for example, cyclopropylene, cyclobutylene, cyclopentylene, cyclohexylene, cycloheptylene, cyclooctylene, and adamantylene, and preferably cyclopropylene and cyclobutylene.

The "C₆₋₁₀ arylene" means aromatic hydrocarbon group having 6 to 10 carbon atoms. The "C₆₋₁₀ arylene" includes, for example, phenylene, 1-naphthylene, and 2-naphthylene, and preferably phenylene.

The "C₅₋₁₀ heteroarylene" means monocyclic 5- to 7-membered aromatic heterocycle or bicyclic 8- to 10-membered aromatic heterocycle having 1 to 4 atoms selected independently from the group consisting of nitrogen atom, oxygen atom and sulfur atom. The "C₅₋₁₀ heteroarylene" includes, for example, pyridylene, pyridazinylene, isothiazolylene, pyrrolylene, furylene, thienylene, thiazolylene, imidazolylene, pyrimidinylene, thiadiazolylene, pyrazolylene, oxazolylene, isooxazolylene, pyrazinylene, triazinylene, triazolylene, imidazolidinylene, oxadiazolylene, triazolylene, tetrazolylene, indolylene, indazolylene, quinolylene, isoquinolylene, benzofuranylene, benzothienylene, benzooxazolylene, benzothiazolylene, benzoisooxazolylene, benzoisothiazolylene, benzotriazolylene, benzoimidazolylene, and 6,11-dihydrodibenzo[b,e]thiepinylene. Preferably, it includes pyridylene, pyrimidinylene, quinolylene, and isoquinolylene, and more preferably pyridylene, furylene, and thienylene.

The "5- to 8-membered monocyclic aromatic carbon ring" means monocyclic aromatic hydrocarbon having 5 - 8 carbon atoms. The "5- to 8-membered monocyclic aromatic carbon ring" includes, for example, phenyl.

The "4- to 7-membered monocyclic aromatic hetero ring" means 4- to 7-membered monocyclic aromatic heterocyclyl group having 1 to 4 atoms selected independently from the group consisting of nitrogen atom, oxygen atom and sulfur atom which constitute the ring. The "4- to 7-membered monocyclic aromatic hetero ring" includes, preferably 5- to 7-membered aromatic heterocyclyl group having at least one nitrogen atom in the ring (5- to 7-membered nitrogen-containing monocyclic aromatic hetero ring"). The "4- to 7-membered monocyclic aromatic hetero ring" includes, for example, pyridyl, pyridazinyl, isothiazolyl, pyrrolyl, furyl, thienyl, thiazolyl, imidazolyl, pyrimidinyl, thiadiazolyl, pyrazolyl, oxazolyl, isoxazolyl, pyrazinyl, triazinyl, triazolyl, imidazolidinyl, oxadiazolyl, triazolyl, and tetrazolyl, preferably pyridyl and pyrimidinyl, and more preferably pyridyl.

In the present compound of formula (1), preferred X, Y¹, Y², R¹, R², R³, R⁴, R⁵, R⁶, R^{Y}, A, L, m, n, and p are shown below, but the technical scope of the present invention is not limited to the scope of compounds listed below.

X includes preferably single bond, oxygen atom, and NR⁶ wherein R⁶ is hydrogen atom or C₁₋₆ alkyl, more preferably single bond and oxygen atom, and even more preferably oxygen atom.

Y¹ includes - (CH₂CH₂O) ₙ-R³.

Y² includes - (CH₂CH₂O) ₚ-R⁵.

R¹ includes preferably C₁₋₆ alkyl which may be substituted with 1 - 3 the same or different C₁₋₆ alkoxy. More preferably, it includes C₁₋₆ alkyl which may be substituted with one C₁₋₆ alkoxy, and even more preferably methyl, ethyl, propyl, butyl, and 1-methoxyethyl.

R² includes preferably
(1) hydrogen atom,
(2) halogen,
(3) C₁₋₆ alkyl, and
(4) C₁₋₆ alkoxy.

R² includes more preferably hydrogen atom and halogen, and even more preferably hydrogen atom.

R³ and R⁵ independently includes preferably hydrogen atom and C₁₋₃ alkyl, more preferably, independently hydrogen atom, methyl, ethyl, and propyl, and even more preferably hydrogen atom.

R⁴ and R⁶ independently includes preferably hydrogen atom and C₁₋₃ alkyl, more preferably, independently hydrogen atom, methyl, ethyl, and propyl.

A includes preferably
(1) 5- to 6-membered monocyclic aromatic carbon ring, and
(2) 5- to 6-membered monocyclic aromatic hetero ring.

A includes more preferably benzene ring and pyridine ring.

A includes even more preferably pyridine ring.

R^{Y} includes preferably hydrogen atom, C₁₋₆ alkyl, and Y², more preferably hydrogen atom, methyl, ethyl, propyl, and - (CH₂CH₂O)ₚ-R⁵, and even more preferably hydrogen atom and - (CH₂CH₂O)ₚ-R⁵. In another embodiment, R^{Y} includes preferably hydrogen atom and C₁₋₆ alkyl, and more preferably hydrogen atom, methyl, ethyl, and propyl.

L includes preferably
(1) -O- ,
(2) -NR^{Y}-,
(3) -C(O)-,
(4) -C(O)O-,
(5) -OC(O)-,
(6) -C(O)NR^{Y}-,
(7) -NR^{Y}C(O)-,
(8) -CH₂NR^{Y}-,
(9) -CH₂O-,
(10) -OC(O)O-,
(11) -NR⁴C(O)O-,
(12) -OC(O)NR^{Y}-,
(13) -NR⁴C(O)NR^{Y}-,
(14) -OC (S) NR^{Y}-, and
(15) -NR⁴C(S)NR^{Y}- .

L includes more preferably
(1) -O-,
(2) -NR^{Y}-,
(3) -C(O)-,
(4) -C(O)O-,
(5) -OC(O)-,
(6) -C(O)NR^{Y}-,
(7) -NR^{Y}C(O) -,
(8) -CH₂NR^{Y}-, and
(9) -CH₂O-.

L includes even more preferably -C(O)NR^{Y}- and -CH₂NR^{Y}-.

L includes the most preferably -CH₂NR^{Y}-.

m is preferably an integer of 1 - 2, and more preferably 1.

n and p include preferably independently an integer of 3 - 40, more preferably an integer of 4 - 40, and the most preferably an integer of 4 - 36.

In another embodiment, n and p include independently an integer of 3 - 40, preferably an integer of 3 - 20, and more preferably an integer of 5 - 20.

In a preferred embodiment, the present compound of formula (1) includes the following (A).
(A) A compound of formula (1) or a pharmaceutically acceptable salt thereof, wherein
   X is single bond, oxygen atom, sulfur atom, SO, SO₂, or NR⁶;
   Y¹ is - (CH₂CH₂O)ₙ-R³,
   Y² is - (CH₂CH₂O)ₚ-R⁵,
   R¹ is C₁₋₆ alkyl which may be substituted with 1 - 5 substituents selected independently from the group consisting of halogen, hydroxy, and C₁₋₆ alkoxy,
   R² is
      (1) hydrogen atom,
      (2) halogen,
      (3) hydroxy,
      (4) C₁₋₆ alkyl,
      (5) C₁₋₆ alkoxy, or
      (6) cyano,
   R³ and R⁵ are independently
      (1) hydrogen atom, or
      (2) C₁₋₆ alkyl,
   R⁴ and R⁶ are independently
      (1) hydrogen atom, or
      (2) C₁₋₆ alkyl,
   A is
      (1) 5- to 8-membered monocyclic aromatic carbon ring, or
      (2) 4- to 7-membered monocyclic aromatic hetero ring,
   R^{Y} is
      (1) hydrogen atom
      (2) C₁₋₆ alkyl, or
      (3) Y²,
   L is
      (1) -O-
      (2) -NR^{Y}-
      (3) -C(O)-
      (4) -C(O)O-
      (5) -OC(O)-
      (6) -C(O)NR^{Y}-
      (7) -NR^{Y}C(O)-
      (8) -CH₂NR^{Y}-
      (9) -CH₂O-
      (10) -OC(O)O-
      (11) -NR⁴C(O)O-
      (12) -OC(O)NR^{Y}-
      (13) -NR⁴C(O)NR^{Y}-
      (14) -OC(S)NR^{Y}-, or
      (15) -NR⁴C(S)NR^{Y}-,
   m is an integer of 1 - 4, and
   n and p are independently an integer of 3 - 100.

In a preferred embodiment, the present compound of formula (1) includes the following (B).

(B)
A compound of formula (1) or a pharmaceutically acceptable salt thereof, wherein
X is single bond, oxygen atom, or NR⁶,
Y¹ is - (CH₂CH₂O) ₙ-R³,
R¹ is C₁₋₆ alkyl which may be substituted with 1 - 3 the same or different C₁₋₆ alkoxy,
R² is
   (1) hydrogen atom,
   (2) halogen,
   (3) C₁₋₆ alkyl, or
   (4) C₁₋₆ alkoxy,
R³ and R⁵ are independently hydrogen atom, or C₁₋₃ alkyl,
R⁶ is hydrogen atom, or C₁₋₃ alkyl,
A is
   (1) 5- to 6-membered monocyclic aromatic carbon ring, or
   (2) 5- to 6-membered monocyclic aromatic hetero ring,
R^{Y} is hydrogen atom, or C₁₋₆ alkyl, or Y²,
Y² is -(CH₂CH₂O)ₚ-R⁵,
L is
   (1) -O-,
   (2) -NR^{Y}-,
   (3) -C(O)-,
   (4) -C(O)O-,
   (5) -OC(O)-,
   (6) -C(O)NR^{Y}-,
   (7) -NR^{Y}C(O)-,
   (8) -CH₂NR^{Y}-, or
   (9) -CH₂O-,
m is an integer of 1 - 2, and
n and p are independently an integer of 3 - 40.

In a preferred embodiment, the present compound of formula (1) includes the following (C).

(C)
A compound of formula (1) or a pharmaceutically acceptable salt thereof, wherein
X is single bond or oxygen atom,
Y¹ is -(CH₂CH₂O)ₙ-R³,
R¹ is C₁₋₆ alkyl which may be substituted with one C₁₋₆ alkoxy,
R² is hydrogen atom, or halogen,
R³ and R⁵ are independently hydrogen atom, methyl, ethyl, or propyl,
A is phenyl, or pyridyl,
R^{Y} is hydrogen atom, methyl, ethyl, propyl, or Y²,
Y² is -(CH₂CH₂O)ₚ-R⁵,
L is -C(O)NR^{Y}-, or -CH₂NR^{Y}-,
m is 1, and
n and p are independently an integer of 3 - 40.

In another embodiment, the present compound of formula (1) includes the following (D).

(D)
A compound of formula (2) or (3) or a pharmaceutically acceptable salt thereof, 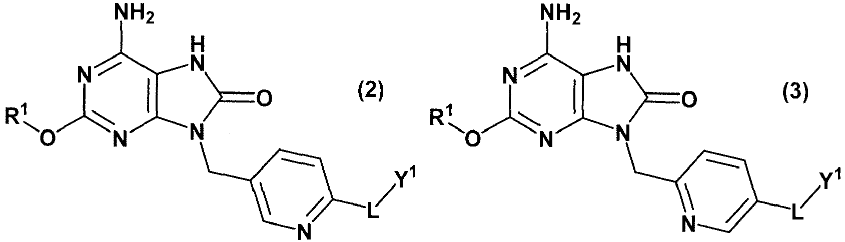 wherein
R¹ is C₁₋₆ alkyl which may be substituted with one C₁₋₆ alkoxy,
L is -CH₂NR^{Y}-,
R^{Y} is hydrogen atom, methyl, ethyl, propyl, or Y²,
Y¹ is -(CH₂CH₂O)ₙ-R³,
Y² is -(CH₂CH₂O)ₚ-R⁵,
R³ and R⁵ are independently hydrogen atom, methyl, ethyl, or propyl, and
n and p are independently an integer of 3 - 40.

In another embodiment, the present compound of formula (1) includes the following (E).

(E)
A compound of formula (2) or (3) or a pharmaceutically acceptable salt thereof, 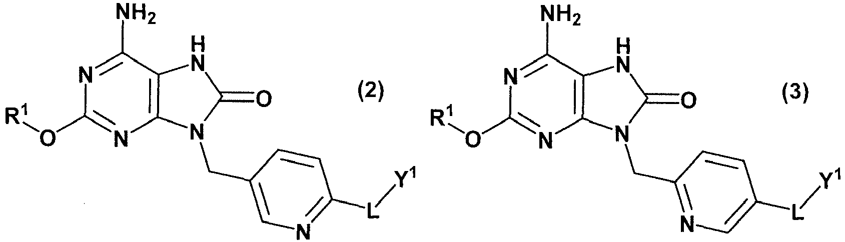 wherein
R¹ is C₁₋₆ alkyl which may be substituted with one C₁₋₆ alkoxy,
L is -CH₂NR^{Y}-,
R^{Y} is hydrogen atom, methyl, ethyl, or propyl,
Y¹ is -(CH₂CH₂O)ₙ-R³,
R³ is hydrogen atom, methyl, ethyl, or propyl, and
n is an integer of 3 - 40.

The processes for preparing the compound of the present invention are shown below. For example, the compound of the formula (1) or a pharmaceutically acceptable salt thereof can be produced by the following processes.

### Process A-1

In compounds according to formula (1) or a pharmaceutically acceptable salt thereof, the compound (a1-2) which has a linker of -CR^{A1}R^{A2}NR^{Y}- or -CR^{A1}R^{A2}O- can be prepared by the following process. 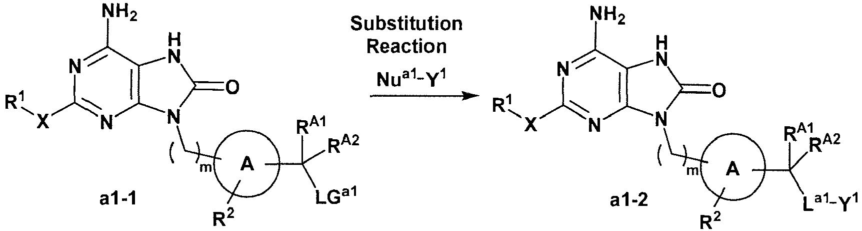 wherein R¹, R², m, A, X, and Y¹ are as defined in Item 1, R^{A1} and R^{A2} are independently hydrogen atom or C₁₋₆ alkyl, LG^{a1} is a leaving group, Nu^{a1} is a nucleophile, and L^{a1} is a linker prepared in the present process.

The present process is a substitution reaction to substitute a leaving group, LG^{a1} with a nucleophile, Nu^{a1}-Y¹. In the present process, Compound (a1-2) can be obtained by reacting Compound (a1-1) and Nu^{a1}-Y¹ in the presence or absence of a suitable base in a suitable solvent. The leaving group includes, but should not be limited to, preferably fluorine, chlorine, bromine, iodine, methanesulfonyl, ethanesulfonyl, and p-toluenesulfonyl, and more preferably chlorine, bromine, and methanesulfonyl. The nucleophile includes, but should not be limited to, preferably amino which may be substituted with R^{Y} defined in Item 11, alcohol, and thiol, and more preferably amino which may be substituted with R^{Y} defined in Item 11, and alcohol. The base used herein can be selected from the bases exemplified below, preferably which includes sodium hydride and potassium hydride. The solvent used herein can be selected from the solvents exemplified below, preferably which includes DMF. The reaction time is generally about 5 minutes to about 48 hours, and preferably about 10 minutes to about 24 hours. The reaction temperature is generally about -78°C to about 100°C, and preferably about 0°C to about 100°C.

### Process A-2

In compounds according to formula (1) or a pharmaceutically acceptable salt thereof, the compound (a2-2) which has a linker of -O-, -NR^{Y}-, -C(O)O-, -CH₂NR^{Y}-, or - CH₂O- can be prepared by the following process. 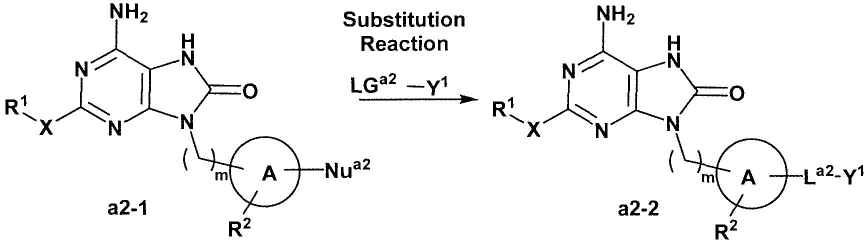 wherein R¹, R², m, A, X, and Y¹ are as defined in Item 1, LG^{a2} is a leaving group, Nu^{a2} is a nucleophile, and L^{a2} is a linker prepared in the present process.

The present process is a substitution reaction to substitute a leaving group, LG^{a2} with a nucleophile, Nu^{a2}. In the present process, Compound (a2-2) can be obtained by reacting Compound (a2-1) and LG^{a2}-Y¹ in the presence or absence of a suitable base in a suitable solvent. LG^{a2} and Nu^{a2} are identical to the leaving group and the nucleophile mentioned in Process A-1, respectively. Each reaction condition of the present process complies with Process A-1.

The processes for preparing the compound of the present invention are shown below. For example, the compound of the formula (1) or a pharmaceutically acceptable salt thereof can be produced by the following processes.

### Process B-1

In compounds according to formula (1) or a pharmaceutically acceptable salt thereof, the compound (b1-2) which has a linker of -CH₂NR^{Y}- can be prepared by the following process. 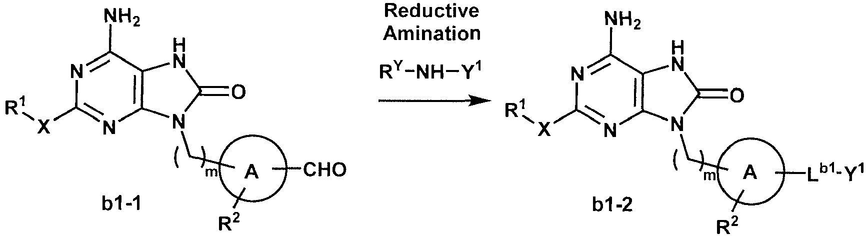 wherein R¹, R², m, A, X, Y¹, and R^{Y} are as defined in Item 1, and L^{b1} is a linker prepared in the present process.

The present process is a reductive amination with an aldehyde and an amine. In the present process, Compound (b1-2) can be obtained by reacting Compound (b1-1) and R^{Y}-NH-Y¹ in the presence of a suitable reductant in a suitable solvent. The reductant used herein includes, but not limited to, preferably sodium borohydride, triacetoxyborohydride, and picoline borane. The solvent used herein can be selected from the solvents exemplified below, preferably which includes THF and chloroform. The reaction time is generally about 5 minutes to about 48 hours, and preferably about 10 minutes to about 24 hours. The reaction temperature is generally about -78°C to about 100°C, and preferably about 0°C to about 100°C.

### Process B-2

In compounds according to formula (1) or a pharmaceutically acceptable salt thereof, the compound (b2-2) which has a linker of -NR^{Y}- or -CH₂NR^{Y}- can be prepared by the following process. 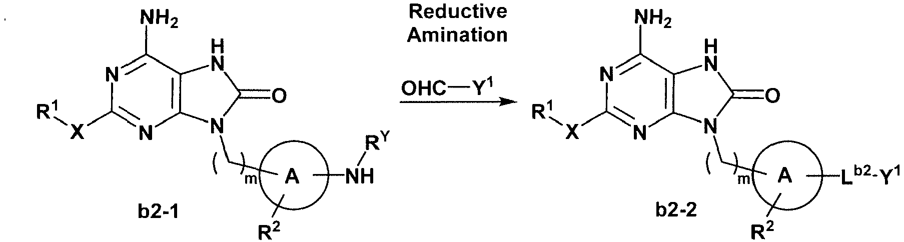 wherein R¹, R², m, A, X, and Y¹ are as defined in Item 1, R^{Y} is as defined in Item 11, and L^{b2} is a linker prepared in the present process.

The present process is a reductive amination with an aldehyde and an amine. In the present process, Compound (b2-2) can be obtained by reacting Compound (b2-1) and Y¹-CHO in the presence of a suitable reductant in a suitable solvent. Each reaction condition of the present process complies with Process B-1.

The processes for preparing the compound of the present invention are shown below. For example, the compound of the formula (1) or a pharmaceutically acceptable salt thereof can be produced by the following process.

### Process C-1

In compounds according to formula (1) or a pharmaceutically acceptable salt thereof, the compound (c1-2) which has a linker of -O-, -NR^{Y}-, or -NR^{Y}C(O)- can be prepared by the following process. 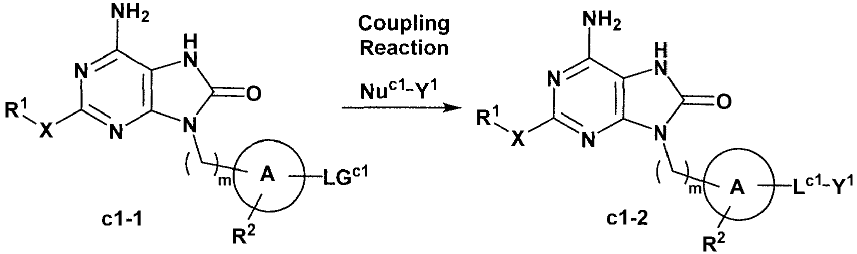 wherein R¹, R², m, A, X, and Y¹ are as defined in Item 1, LG^{c1} is a leaving group, Nu^{c1} is a nucleophile, and L^{c1} is a linker prepared in the present process.

The present process is a coupling reaction with a leaving group (LG^{c1}) and a nucleophile (Nu^{c1}-Y¹). In the present process, Compound (c1-2) can be obtained by reacting Compound (c1-1) and a nucleophile (Nu^{c1}-Y¹) in the presence of a suitable catalyst in the presence or absence of a suitable base in a suitable solvent. The catalyst used herein includes a transition metal such as palladium or a salt thereof, a complex containing it, and a carrier-supported (e.g. polymer-supported) one. The leaving group includes, but should not be limited to, preferably boronic acid, boronate, halogen, and trifluoromethanesulfonyl, and more preferably boronic acid, boronate, bromine, iodine, and trifluoromethanesulfonyl. The nucleophile includes, but should not be limited to, amine which may be substituted with C₁₋₆ alkyl, alcohol, alkylmagnesium, alkylzinc, and alkyllithium, and more preferably amine which may be substituted with C₁₋₆ alkyl, and alcohol. The solvent used herein can be selected from the solvents exemplified below, preferably which includes dioxane-water mixture. The reaction time is generally about 5 minutes to about 48 hours, and preferably about 10 minutes to about 24 hours. The reaction temperature is generally about -78°C to about 100°C, and preferably about 0°C to about 100°C.

The processes for preparing the compound of the present invention are shown below. For example, the compound of the formula (1) or a pharmaceutically acceptable salt thereof can be produced by the following processes.

### Process D-1

In compounds according to formula (1) or a pharmaceutically acceptable salt thereof, the compound (d1-2) which has a linker of -C(O)O- or -C(O)NR^{Y}-linker can be prepared by the following process. 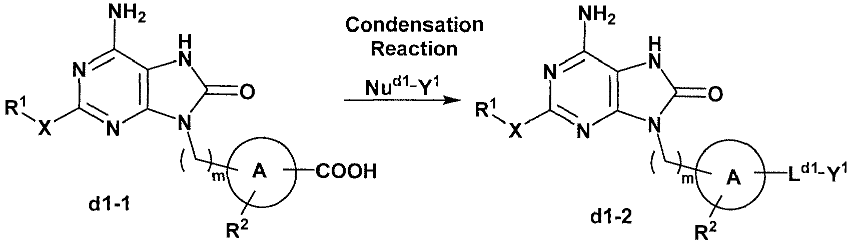 wherein R¹, R², m, A, X, and Y¹ are as defined in Item 1, Nu^{d1} is a nucleophile, and L^{d1} is a linker prepared in the present process.

The present process is a condensation reaction with Compound (d1-1) which has a carboxylic acid and Nu^{d1}-Y¹. In the present process, Compound (d1-2) can be obtained by reacting Compound (d1-1) and a nucleophile (Nu^{d1}-Y¹) in the presence of a suitable condensation agent in the presence or absence of a suitable base in a suitable solvent. The nucleophile includes, but should not be limited to, preferably amine which may be substituted with one C₁₋₆ alkyl, alcohol, and thiol, and preferably amine which may be substituted with one C₁₋₆ alkyl, and alcohol. The condensation agent used herein can be selected from condensation agents used in conventional manners, preferably which includes HBTU, HATU, and 1-ethyl-3-(3-dimethylaminopropyl)carbodiimide (including its hydrochloride). The base used herein can be selected from the bases exemplified below, preferably which includes tert-alkylamine, more preferably DIPEA and triethylamine. The solvent used herein can be selected from the solvents exemplified below, preferably which includes DMF, dichloromethane, chloroform, and THF. The reaction time is generally about 5 minutes to about 48 hours, and preferably about 10 minutes to about 24 hours. The reaction temperature is generally about -78°C to about 100°C, and preferably about 0°C to about 100°C.

The processes for preparing the compound of the present invention are shown below. For example, the compound of the formula (1) or a pharmaceutically acceptable salt thereof can be produced by the following processes.

### Process D-2

In compounds according to formula (1) or a pharmaceutically acceptable salt thereof, the compound (d2-2) which has a linker of -OC(O)- or -NR^{Y}C(O)- can be prepared by the following process. 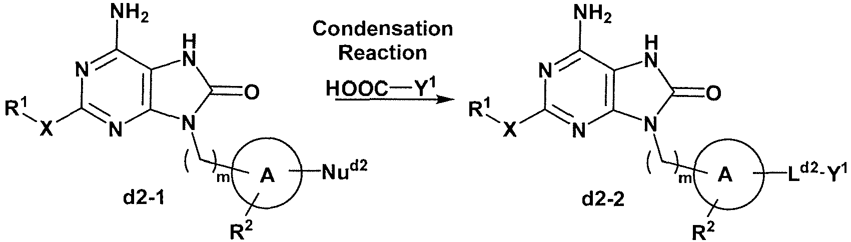 wherein R¹, R², m, A, X, and Y¹ are as defined in Item 1, NU^{d2} is a nucleophile, and L^{d2} is a linker prepared in the present process.

The present process is a condensation reaction with Compound (d2-1) having Nu^{d2} and Y¹-COOH. In the present process, Compound (d2-2) can be obtained by reacting Compound (d2-1) having a nucleophile and a carboxylic compound (Y¹-COOH) in the presence of a suitable condensation agent in the presence or absence of a suitable base in a suitable solvent. Each reaction condition of the present process complies with Process D-1.

### Process E-1

The compound of the formula (1) or a pharmaceutically acceptable salt thereof can be produced, for example, by the following processes. 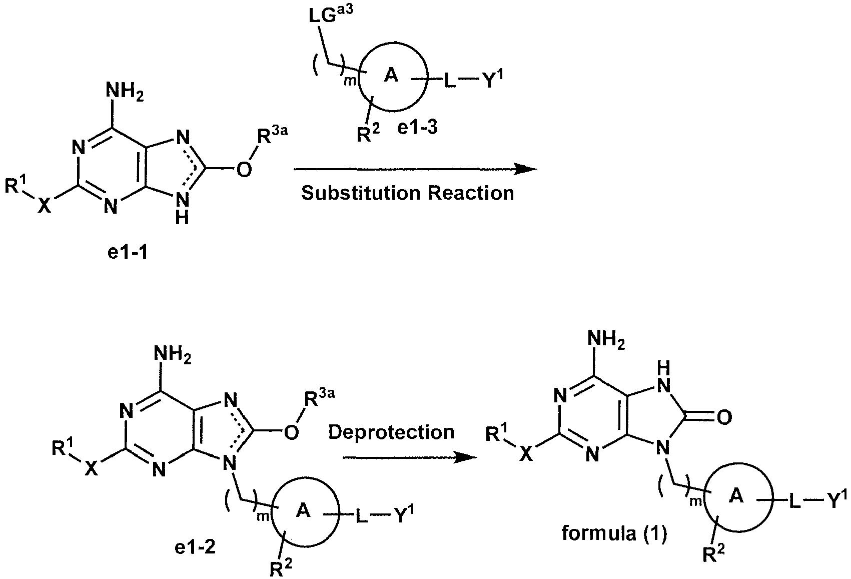 wherein R¹, R², m, A, L, X, and Y¹ are as defined in Item 1, LG^{a3} is a leaving group, R^{3a} is C₁₋₆ alkyl, and the dotted line denotes double bond or single bond.

The present process is a substitution reaction to substitute a leaving group (LG^{a3}) with a nucleophile, the nitrogen in Compound (e1-1), followed by a deprotection. In the present process, Compound (e1-2) can be obtained by reacting Compound (e1-1) and Compound (e1-3) in the presence or absence of a suitable base in a suitable solvent, and then Compound of formula (1) can be prepared by hydrolyzing Compound (e1-2) in the presence of a suitable acid in a suitable solvent. LG^{a3} is identical to the leaving group mentioned in Process A-1. Each reaction condition of the present process complies with Process A-1.

The suitable acid used herein may be an organic acid or an inorganic acid, and preferably hydrochloric acid, but should not be limited thereto.

The base used in each step of the above processes should be suitably selected based on the reaction, the starting compound, etc., which includes alkaline bicarbonates such as sodium bicarbonate, and potassium bicarbonate; alkaline carbonate such as sodium carbonate, and potassium carbonate; metallic hydrides such as sodium hydride, and potassium hydride; alkaline metal hydroxides such as sodium hydroxide, and potassium hydroxide; alkaline metal alkoxides such as sodium methoxide, and sodium t-butoxide; organic metal bases such as butyllithium, and lithium diisopropylamide; and organic bases such as triethylamine, diisopropylethylamine, pyridine, 4-dimethylaminopyridine (DMAP), and 1,8-diazabicyclo[5.4.0]undec-7-ene (DBU).

The condensation agent may be those described in Jikken Kagaku Kouza (The Chemical Society of Japan ed., Maruzen) Vol. 22, which includes, for example, phosphates such as diethyl cyanophosphate and diphenylphosphoryl azide; carbodiimides such as 1-ethyl-3-(3-dimethylaminopropyl)-carbodiimide hydrochloride (WSC·HCl) and dicyclohexylcarbodiimide (DCC); combinations of a disulfide such as 2,2'-dipyridyldisulfide and a phosphine such as triphenylphosphine; phosphorus halides such as N,N'-bis(2-oxo-3-oxazolidinyl)phosphinic chloride (BOPCl); combinations of an azodicarboxylate diester such as diethyl azodicarboxylate and a phosphine such as triphenylphosphine; 2-halo-1-lower alkylpyridinium halides such as 2-chloro-1-methylpyridinium iodide; 1,1'-carbonyldiimidazole (CDI); diphenylphosphoryl azide (DPPA); diethylphosphoryl cyanide (DEPC); tetrafluoroborates such as 2-(1H-benzotriazol-1-yl)-1,1,3,3-tetramethyluronium tetrafluoroborate (TBTU) and 2-chloro-1,3-dimethylimidazolidinium tetrafluoroborate (CIB); phosphates such as 2-(1H-benzotriazol-1-yl)-1,1,3,3-tetramethyluronium hexafluorophosphate (HBTU), benzotriazol-1-yloxytris(dimethylamino)phosphonium hexafluorophosphate (BOP), benzotriazol-1-yloxytris(pyrrolidino)phosphonium hexafluorophosphate (PYBOP), and 2-(7-aza-1H-benzotriazol-1-yl)-1,1,3,3-tetramethyluronium hexafluorophosphate (HATU).

The solvent used in each step of the above processes should be suitably selected based on the reaction, the starting compound, etc., which includes, for example, alcohol solvents such as methanol, ethanol, and isopropanol; ketone solvents such as acetone and methylketone; halogenated hydrocarbon solvents such as methylene chloride and chloroform; ether solvents such as tetrahydrofuran (THF) and dioxane; aromatic hydrocarbon solvents such as toluene and benzene; aliphatic hydrocarbon solvents such as hexane and heptane; ester solvents such as ethyl acetate and propyl acetate; amide solvents such as N,N-dimethylformamide (DMF) and N-methyl-2-pyrrolidone; sulfoxide solvents such as dimethylsulfoxide (DMSO); nitrile solvents such as acetonitrile; and water. The solvent used herein may be one of these solvents or a mixture of two or more solvents selected from these solvents. And, if possible in the reaction, an organic base may be used as a solvent used herein.

The "pharmaceutically acceptable salt" includes an acid addition salt and a base addition salt. For example, the acid addition salt includes an inorganic acid salt such as hydrochloride, hydrobromide, sulfate, hydroiodide, nitrate, and phosphate; and an organic acid salt such as citrate, oxalate, phthalate, fumarate, maleate, succinate, malate, acetate, formate, propionate, benzoate, trifluoroacetate, methanesulfonate, benzenesulfonate, para-toluenesulfonate, and camphorsulfonate; and the base addition salt includes an inorganic base salt such as sodium salt, potassium salt, calcium salt, magnesium salt, barium salt, and aluminium salt; and an organic base salt such as trimethylamine, triethylamine, pyridine, picoline, 2,6-lutidine, ethanolamine, diethanolamine, triethanolamine, tromethamine [tris(hydroxymethyl)methylamine], tert-butylamine, cyclohexylamine, dicyclohexylamine, and N,N-dibenzylethylamine. Furthermore, they include a basic or acidic amino acid salt such as arginine, lysine, ornithine, aspartate, and glutamate.

The suitable salts of starting compounds or desired compounds, and pharmaceutically acceptable salts are conventional non-toxic salts, which include an acid addition salt such as an organic acid salt (e.g. acetate, trifluoroacetate, maleate, fumarate, citrate, tartrate, methanesulfonate, benzenesulfonate, formate, para-toluenesulfonate, etc.) and an inorganic acid salt (e.g. hydrochloride, hydrobromide, hydroiodide, sulfate, nitrate, phosphate, etc.); a salt with an amino acid (e.g. arginine, aspartate, glutamate, etc.); a metallic salt such as an alkaline metal salt (e.g. sodium salt, potassium salt, etc.) and an alkaline-earth metal salt (e.g. calcium salt, magnesium salt, etc.); ammonium salt; and an organic base salt (e.g. trimethylamine salt, triethylamine salt, pyridine salt, picoline salt, dicyclohexylamine salt, N,N'-dibenzylethylenediamine salt, etc.); and furthermore, what a skilled person selects suitably.

If it is desirable to fix the compound of the present invention as a salt, when the compound of the present invention is obtained as a salt, it may be purified without further reaction, and when it is obtained in a free form, it may be dissolved or suspended in an appropriate organic solvent and an acid or base may be added therein to form a salt in a general manner.

The compound of the present invention or a pharmaceutically acceptable salt thereof may sometimes exist in form of solvate with water or various solvents. Such solvates are also included in the present invention.

The compound of formula (1) in which any one or more ¹H atoms are replaced by ²H(D) atoms is also within the scope of the present invention of formula (1).

The present invention encompasses the compound of formula (1) or a pharmaceutically acceptable salt thereof. In addition, the present invention encompasses a hydrate thereof and a solvate thereof such as ethanolate thereof. Furthermore, the present invention encompasses all tautomers, stereoisomers, and crystal forms thereof.

And, the adenine compound of formula (1) and its tautomer are chemically equivalent, and the adenine compound of the present invention encompasses the tautomer. The specific tautomer is a hydroxy compound of formula (1'): 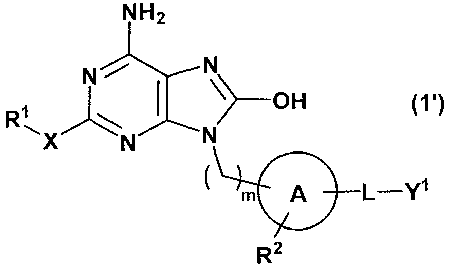 wherein X, R¹, R², m, A, L, and Y¹ are as defined above.

The present compound (1) also includes an optical isomer which is based on chiral center, an atropisomer which is based on axiality caused by intramolecular rotational hindrance or planar-chirality, other stereoisomers, tautomer, and geometric isomer, all possible isomers of which and a mixture thereof are encompassed in the present invention.

The optical isomer mixture of the present compounds can be prepared in a conventional manner. The compounds having an asymmetric structure can be prepared, for example, by using a starting material having an asymmetric center or by introducing an asymmetric structure anywhere along the process. For example, in case of optical isomers, optical isomers can be obtained by using an optically active starting material or resolving a mixture of optical isomers at an appropriate step. In case that the compound of formula (1) or its intermediate has a basic functional group, the optical resolution thereof includes, for example, diastereomer method, wherein the compound is transformed to a salt thereof by reacting with an optically active acid (for example, a monocarboxylic acid such as mandelic acid, N-benzyloxyalanine, and lactic acid; dicarboxylic acid such as tartaric acid, o-diisopropylidene-tartaric acid, and malic acid; or a sulfonic acid such as camphorsulfonic acid and bromocamphorsulfonic acid), in an inert solvent (for example, an alcohols such as methanol, ethanol, and 2-propanol; an ether solvent such as diethyl ether; an ester solvent such as ethyl acetate; a hydrocarbon solvent such as toluene; an aprotic solvent such as acetonitrile; or a mixed solvent thereof). In case that the compound of formula (1) or its intermediate has an acidic functional group such as carboxyl group, the compound can be also optically resolved after forming its salt with an optically active amine (for example, an organic amine such as 1-phenylethylamine, kinin, quinidine, cinchonidine, cinchonine, and strychnine).

The present compounds of formula (1) and their intermediates can be isolated and purified in a manner known by a skilled person. It includes, for example, extraction, partition, reprecipitation, column chromatography (e.g. silica gel column chromatography, ion exchange column chromatography, and preparative liquid chromatography), and recrystallization.

The solvent for recrystallization used herein includes, for example, an alcohols solvent such as methanol, ethanol, and 2-propanol; an ether solvent such as diethyl ether; an ester solvent such as ethyl acetate; an aromatic hydrocarbon solvent such as benzene and toluene; a ketone solvent such as acetone; a halogenated solvent such as dichloromethane and chloroform; a hydrocarbon solvent such as hexane; an aprotic solvent such as dimethylformamide and acetonitrile; water; and a mixed solvent thereof. As other methods for purification, for example, methods described in Jikken Kagaku Kouza (The Chemical Society of Japan ed., Maruzen) Vol. 1 can be used. And, the structural determination of the present compounds can be easily done by spectroscopic analytical method such as nuclear magnetic resonance method, infrared absorption technique, and circular dichroism spectra analysis, and mass spectrometry, considering the structure of each starting compound.

In addition, each intermediate or each final product in the above preparation processes can be also transformed to another compound of the present invention by suitably modifying its functional group, especially extending various side-chains from amino, hydroxy, carbonyl, halogen, etc.; and optionally making the above-mentioned protection and deprotection if necessary. The modification of functional group and the extension of side-chain can be done by a conventional method (for example, see Comprehensive Organic Transformations, R. C. Larock, John Wiley & Sons Inc. (1999), etc.).

The temperature for forming a salt is selected from the range of generally -50°C to boiling point of a solvent used herein, preferably 0°C to the boiling point, and more preferably room temperature to the boiling point. In order to enhance the optical purity, it is desirable to make the temperature raised to around boiling point of a solvent used herein. In collecting a precipitated crystal on a filter, an optional cooling can make the yield increased. The amount of an optically active acid or amine used herein is suitably about 0.5 - about 2.0 equivalents against that of the substance compound, preferably around one equivalent. If appropriate, the obtained crystal may be recrystallized in an inert solvent (for example, an alcohols such as methanol, ethanol, and 2-propanol; an ether solvent such as diethyl ether; an ester solvent such as ethyl acetate; a hydrocarbon solvent such as toluene; an aprotic solvent such as acetonitrile; or a mixed solvent thereof) to obtain its highly pure salt thereof. And, if appropriate, the optically-resolved salt can be also treated with an acid or a base to obtain its free form.

Among the starting materials and the intermediates in each preparation process mentioned above, the compounds that are not described in each process are commercially available or can be prepared by a skilled person with a commercial available material in a known manner or a similar manner thereto.

The present invention provides the above-defined compound of formula (1) or a pharmaceutically acceptable salt thereof which is useful as vaccine adjuvant, preferably vaccine adjuvant for cancer vaccine.

In addition, the present invention provides a pharmaceutical composition comprising the above-defined compound of formula (1) or a pharmaceutically acceptable salt thereof in combination with a pharmaceutically acceptable diluent or carrier (hereinafter, referred to as the present pharmaceutical composition).

The present compound or a pharmaceutically acceptable salt thereof may be used as an adjuvant for maintaining or enhancing the immunostimulatory of an active ingredient having an immunostimulating activity.

Namely, the present compound or a pharmaceutically acceptable salt thereof has an activity for inducing or enhancing antigen-specific antibody, specifically antigen-specific IgG, and in more detail Th1-type antigen-specific IgG (e.g. IgG2c).

And, the present compound or a pharmaceutically acceptable salt thereof has an activity for increasing cytotoxic T-lymphocyte (CTL). Or, the present compound or a pharmaceutically acceptable salt thereof has an activity for inducing CTL in mammal or enhancing the CTL induction in mammal.

And, the present compound or a pharmaceutically acceptable salt thereof has an activity for enhancing CD4-positive (i.e., MHC class II-restricted) and/or CD8-positive (i.e., MHC Class I-restricted) T-cell.

And, the present compound or a pharmaceutically acceptable salt thereof has an activity for increasing antigen-specific T-cell.

And, the present compound or a pharmaceutically acceptable salt thereof has an activity for increasing memory T-cell, specifically, CD8-positive effector memory T-cell.

And, the present compound or a pharmaceutically acceptable salt thereof has a character to increase CTL more highly than the same moles of a compound having no PEG structure when administered to mammal.

And, the present compound or a pharmaceutically acceptable salt thereof has an activity for activating immunocompetent cells.

The present pharmaceutical composition may comprise a tumor antigen. As the tumor antigen, tumor antigen protein, or tumor antigen peptide derived from the tumor antigen protein may be used. The tumor antigen peptide used herein includes, preferably the antigen peptide mentioned below, more preferably tumor antigen peptide derived from NY-ESO-1, MAGE-3, WT1, OR7C1, and Her2/neu, and even more preferably tumor antigen peptide derived from WT1. Further, a peptide derived from a neoantigen which results from tumor genetic abnormality may be also used with the compound of the present invention or a pharmaceutically acceptable salt thereof.

In addition, a pharmaceutical composition comprising the present compound or a pharmaceutically acceptable salt thereof and a tumor antigen has an action for inhibiting the growth of tumor which expresses the antigen or the occurrence of tumor which expresses the antigen.

Thus, the present compound or a pharmaceutically acceptable salt thereof is useful as a medicament for treating or preventing cancer by using as a pharmaceutical composition in combination with the tumor antigen mentioned below.

The tumor antigen peptide used herein should not be limited to a particular one, but which may be selected from the peptides and the like disclosed in WO 2014/157692 or WO 2014/157704 A1.

In one embodiment of the tumor antigen peptide, it includes, for example, peptides or pharmaceutically acceptable salts thereof of the following amino acid sequences:
RMFPNAPYL (SEQ ID NO: 1),
ALLPAVPSL (SEQ ID NO: 9),
SLGEQQYSV (SEQ ID NO: 10),
RVPGVAPTL (SEQ ID NO: 11),
VLDFAPPGA (SEQ ID NO: 5),
CMTWNQMNL (SEQ ID NO: 12),
CYTWNQMNL (SEQ ID NO: 2),
WAPVLDFAPPGASAYGSL (SEQ ID NO: 3),
CWAPVLDFAPPGASAYGSL (SEQ ID NO: 13),
WAPVLDFAPPGASAYGSLC (SEQ ID NO: 14),
CNKRYFKLSHLQMHSRKHTG (SEQ ID NO: 15),
CNKRYFKLSHLQMHSRKH (SEQ ID NO: 16),
CNKRYFKLSHLQMHSRK (SEQ ID NO: 17),
KRYFKLSHLQMHSRKH (SEQ ID NO: 4), and
TYAGCLSQIF (SEQ ID NO: 18).

And, peptides or pharmaceutically acceptable salt thereof of the following amino acid sequences of
formula (4): 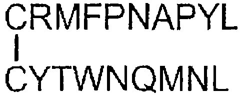 wherein the bond between C-C is disulfide bond, and formula (5): 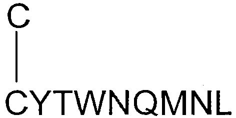 wherein the bond between C-C is disulfide bond
may be used as a tumor antigen peptide in the present invention.

The tumor antigen peptides can be prepared in a general manner used in peptide chemical field. The synthetic methods include what a reference (Peptide Synthesis, Interscience, New York, 1966; The Proteins, Vol. 2, Academic Press Inc., New York, 1976), etc. discloses.

In another embodiment, the pharmaceutical composition of the present invention may include an antigen. The antigen includes a pathogen-derived antigen, for example, a protein derived from virus or bacterium or its partial protein. And, a complex of the antigen and carrier, etc. is included in the scope of the antigen in the present invention. The complex includes an antigen (including protein and peptide, but not limited thereto) bridged to a protein which is a carrier via a linker which is well known by a skilled person, and an antigen contained in virus-like particle (VLP). Thus, the present compound or a pharmaceutically acceptable salt thereof is useful as a medicament for treating or preventing infection of virus or bacterium by using in combination with the above-mentioned antigen.

Examples of the administration route of the pharmaceutical composition of the present invention includes parenteral administration, specifically intravascular (e.g., intravenous), subcutaneous, intradermal, intramuscular, intratumor, lymph node, and transdermal administrations.

In one embodiment, the pharmaceutical composition of the present invention may comprise a compound of the formula (1) or a pharmaceutically acceptable salt thereof and a pharmaceutically acceptable diluent or carrier.

The drug formulation of the present pharmaceutical composition includes a liquid formulation.

The liquid formulation of the present invention includes an aqueous solution formulation/an aqueous suspension formulation, an oily solution formulation/an oily suspension formulation, a hydrogel formulation, a lipid formulation, and an emulsion formulation.

The aqueous solution formulation or the aqueous suspension formulation includes, for example, a formulation prepared by dissolving or dispersing an antigen (tumor antigen or pathogen-derived antigen), and/or the compound of formula (1) or a pharmaceutically acceptable salt thereof in water.

The oily solution formulation or the oily suspension formulation includes, for example, a formulation prepared by dissolving or dispersing an antigen (tumor antigen or pathogen-derived antigen), and/or the compound of formula (1) or a pharmaceutically acceptable salt thereof in an oily ingredient.

The hydrogel formulation includes, for example, a formulation prepared by dissolving or dispersing an antigen (tumor antigen or pathogen-derived antigen), and/or the compound of formula (1) or a pharmaceutically acceptable salt thereof in water and adding viscosity to the formulation.

The lipid formulation includes, for example, a liposome formulation comprising an antigen (tumor antigen or pathogen-derived antigen), and/or the compound of formula (1) or a pharmaceutically acceptable salt thereof.

The emulsion formulation includes, for example, a formulation including an aqueous solution and an oily composition, which comprises an antigen (tumor antigen or pathogen-derived antigen), and/or the compound of formula (1) or a pharmaceutically acceptable salt thereof.

In another embodiment of the present liquid formulation, the liquid formulation of the present invention includes, an aqueous solution formulation or an aqueous suspension formulation prepared by dissolving or dispersing a tumor antigen, and/or the compound of formula (1) or a pharmaceutically acceptable salt thereof in water; an oily solution formulation or an oily suspension formulation prepared by dissolving or dispersing a tumor antigen, and/or the compound of formula (1) or a pharmaceutically acceptable salt thereof in an oily ingredient; and an emulsion formulation comprising an aqueous solution and an oily composition.

The additive used in the present aqueous solution formulation or aqueous suspension formulation includes, for example, purified water, water for injection, a buffering agent, a pH adjusting agent, a stabilizer, an isotonizing agent, a solubilizer, and a solubilizing agent.

The additive used in the present oily solution formulation or oily suspension formulation includes, for example, a buffering agent, a pH adjusting agent, a stabilizer, an isotonizing agent, animal or vegetable oil and fat, hydrocarbons, a fatty acid, fatty acid esters, a solubilizer, and a solubilizing agent.

The additive used in the present hydrogel formulation includes, for example, purified water, water for injection, a buffering agent, a pH adjusting agent, a stabilizer, an isotonizing agent, a solubilizer, a solubilizing agent, and a thickener.

The additive used in the present liposome formulation includes, for example, purified water, water for injection, a buffering agent, a pH adjusting agent, a stabilizer, an isotonizing agent, a solubilizer, a solubilizing agent, and lipids.

The present emulsion formulation used herein includes oil-in-water emulsion (also refered to as O/W emulsion), water-in-oil emulsion (also refered to as W/O emulsion), water-in-oil-in-water emulsion (also refered to as W/O/W emulsion), and oil-in-water-in-oil emulsion (also refered to as O/W/O emulsion). The present emulsion formulation includes, preferably water-in-oil emulsion (W/O emulsion). The present emulsion formulation can be prepared by emulsifying an aqueous phase and an oil phase in a general manner. An antigen (tumor antigen or pathogen-derived antigen), and/or the compound of formula (1) or a pharmaceutically acceptable salt thereof may be contained in an oil phase and/or an aqueous phase.

The additive used in the present emulsion formulation includes, for example, water, a buffering agent, a pH adjusting agent, a stabilizer, an isotonizing agent, animal or vegetable oil and fat, hydrocarbons, a fatty acid, fatty acid esters, glycerinfatty acid esters, a hydrophilic surfactant, and a lipophilic surfactant, wherein
the water includes purified water and water for injection,
the buffering agent includes phosphate and organic acid salt,
the pH adjusting agent includes hydrochloric acid and sodium hydroxide,
the stabilizer includes glycerin, propylene glycol, and sulfite,
the isotonizing agent includes sodium chloride, glucose, sucrose, and mannitol,
the animal or vegetable oil and fat includes olive oil, soybean oil, and liver oil,
the hydrocarbon includes liquid paraffin, squalene, and squalane,
the fatty acid includes oleic acid and myristic acid,
the fatty acid ester includes ethyl oleate, octyldodecyl myristate, cetyl 2-ethyl-hexanoate, and isopropyl myristate,
the glycerin fatty acid ester includes medium-chain triglyceride, medium-chain diglyceride, and medium-chain monoglyceride,
the hydrophilic surfactant includes polyoxyethylene castor oil, polyoxyethylene hydrogenated castor oil, polyoxyethylene sorbitan fatty acid ester, and polysorbates, and
the lipophilic surfactant includes glyceryl monooleate, glyceryl dioleate, sorbitan monooleate (Span^{™} 80), sorbitan sesquioleate, sorbitan dioleate, sorbitan trioleate (Span^{™} 85), PEG-30 dipolyhydroxy stearate, and plant-derived surfactant (saponin, etc.).

Specific composition of additives in the present emulsion formulation used herein includes, but not limited to, an emulsified composition for dilution disclosed in WO 2006/078059, Montanide ISA 51 VG (Seppic), Montanide ISA 720 VG (Seppic), and Incomplete Freund's Adjuvant (IFA).

The present W/O emulsion formulation includes a preparation comprising the compound of formula (1) or a pharmaceutically acceptable salt thereof, ethyl oleate, octyldodecyl myristate, sorbitan monooleate, glyceryl monooleate, polyoxyethylene hydrogenated castor oil 20, glycerin, and sodium dihydrogen phosphate; and a preparation comprising the compound of formula (1) or a pharmaceutically acceptable salt thereof, and Montanide ISA 51 VG.

In the liposome formulation of the present invention, the liposome means a microvesicle composed of lipid multiple layers such as bilayer membrane of amphiphilic lipid molecule (lipid bilayer), which has an internal phase. The preferred lipid multiple layer is lipid bilayer.

The present liposome formulation includes amphiphilic lipid molecule. The amphiphilic lipid molecule includes, preferably one or more "phospholipids". The "phospholipid" includes, for example, phosphatidylcholine, phosphatidylglycerol, phosphatidic acid, phosphatidylethanolamine, phosphatidylserine, phosphatidylinositol, and sphingomyelin. The "phospholipid" includes, preferably phosphatidylcholine, phosphatidylglycerol, phosphatidylethanolamine, sphingomyelin, and phosphatidylserine. The "phospholipid" includes, more preferably phosphatidylcholine, sphingomyelin, and phosphatidylserine.

The fatty acid residue of the "phospholipid" includes, but not limited to, C₁₄₋₁₈ saturated or unsaturated fatty acid residue, for example, an acyl group derived form a fatty acid such as myristic acid, palmitic acid, stearic acid, oleic acid, and linoleic acid. And, naturally-derived phospholipid such as egg-yolk lecithin and soybean lecithin, and the phospholipid whose unsaturated fatty acid residue is hydrogenated such as hydrogenated egg-yolk lecithin and hydrogenated soybean lecithin (also referred to as hydrogenated soybean phospholipid, or hydrogenated soybean phosphatidylcholine) may be also used herein.

The content of phospholipid per the whole component of the liposome membrane (mole fraction) includes, but not limited to, preferably 30 - 80 %, and more preferably 40 - 70 %.

The liposome internally-including the present compound may contain sterols. The sterols includes cholesterol, β-sitosterol, stigmasterol, campesterol, brassicasterol, ergosterol, and fucosterol, and preferably cholesterol. The content of sterols per the whole component of the liposome membrane (mole fraction) includes, but not limited to, preferably 0 - 60 %, more preferably 10 - 50 %, and even more preferably 30 - 50 %.

The liposome internally-including the present compound may contain a polymer-modified lipid. The polymer-modified lipid means a lipid modified with polymer. The polymer-modified lipid is denoted by "lipid-polymer". The polymer part in polymer-modified lipid is preferably a hydrophilic polymer, and more preferably hydrophilic polymer where the polymer-terminal which is not bonded to lipid is alkoxylated. The polymer part in polymer-modified lipid is more preferably a hydrophilic polymer where the polymer-terminal which is not bonded to lipid is methoxylated, ethoxylated, or propoxylated. The polymer part in polymer-modified lipid is the most preferably a hydrophilic polymer where the polymer-terminal which is not bonded to lipid is methoxylated. The polymer part in polymer-modified lipid includes, but not limited to, for example, polyethylene glycol, polypropylene glycol, polyvinyl alcohol, polyvinylpyrrolidone, methoxypolyethylene glycol, methoxypolypropylene glycol, methoxypolyvinyl alcohol, methoxypolyvinylpyrrolidone, ethoxypolyethylene glycol, ethoxypolypropylene glycol, ethoxypolyvinyl alcohol, ethoxypolyvinylpyrrolidone, propoxypolyethylene glycol, propoxypolypropylene glycol, propoxypolyvinyl alcohol, and propoxypolyvinylpyrrolidone. The polymer part in polymer-modified lipid includes, preferably polyethylene glycol, methoxypolyethylene glycol, methoxypolypropylene glycol, ethoxypolyethylene glycol, ethoxypolypropylene glycol, propoxypolyethylene glycol, and propoxypolypropylene glycol. The polymer part in polymer-modified lipid includes, more preferably polyethylene glycol, methoxypolyethylene glycol, ethoxypolyethylene glycol, ethoxypolypropylene glycol, and propoxypolyethylene glycol. The polymer part in polymer-modified lipid includes, even more preferably polyethylene glycol and methoxypolyethylene glycol. The polymer part in polymer-modified lipid includes, the most preferably methoxypolyethylene glycol. The molecular weight of polymer part in polymer-modified lipid includes, but not limited to, for example, 100 - 10000 daltons, preferably 500 - 8000 daltons, more preferably 1000 - 7000 daltons, even more preferably 1500 - 5000 daltons, and the most preferably 1500 - 3000 daltons. The lipid part of polymer-modified lipid includes, but not limited to, for example, phosphatidylethanolamine and diacylglycerol. The lipid part of polymer-modified lipid includes, preferably phosphatidylethanolamine having C₁₄₋₁₈ saturated or unsaturated fatty acid residue and diacylglycerol having C₁₄₋₁₈ saturated or unsaturated fatty acid residue, more preferably phosphatidylethanolamine having C₁₄₋₁₈ saturated fatty acid residue and diacylglycerol having C₁₄₋₁₈ saturated fatty acid residue, and even more preferably phosphatidylethanolamine having palmitoyl group or stearoyl group and diacylglycerol having palmitoyl group or stearoyl group. The lipid part of polymer-modified lipid includes, the most preferably distearoylphosphatidylethanolamine.

The content of polymer-modified lipid per the whole component of the liposome membrane (mole fraction) includes, but not limited to, preferably 0 - 20 %, more preferably 1 - 10 %, and even more preferably 2 - 6 %.

The liposome internally-including the present compound may contain a pharmaceutically acceptable additive. The additive includes, for example, an inorganic acid, an inorganic acid salt, an organic acid, an organic acid salt, sugars, a buffering agent, an antioxidant, and polymers. The inorganic acid includes, for example, phosphoric acid, hydrochloric acid, and sulfuric acid. The inorganic acid salt includes, for example, disodium hydrogen phosphate, sodium chloride, ammonium sulfate, and magnesium sulfate. The organic acid includes, for example, citric acid, acetic acid, succinic acid, and tartaric acid. The organic acid salt includes, for example, sodium citrate, sodium acetate, disodium succinate, and sodium tartrate. The sugar includes, for example, glucose, sucrose, mannitol, sorbitol, and trehalose. The buffering agent includes, for example, L-arginine, L-histidine, trometamol (trishydroxymethylaminomethane, Tris), and a salt thereof. The antioxidant includes, for example, sodium sulfite, L-cysteine, sodium thioglycolate, sodium thiosulfate, ascorbic acid, and tocopherol. The polymers includes, for example, polyvinyl alcohol, polyvinylpyrrolidone, carboxy vinyl polymer, and carboxymethylcellulose sodium.

In the present oily suspension formulation, an antigen (tumor antigen or pathogen-derived antigen) and/or the compound of formula (1) or a pharmaceutically acceptable salt thereof may be contained in an oily ingredient, in solution state or dispersion state, or in the both state. The additive used in the present oily suspension formulation includes, for example, a buffering agent, a pH adjusting agent, a stabilizer, an isotonizing agent, animal or vegetable oil and fat, hydrocarbons, a fatty acid, fatty acid esters, a solubilizer and a solubilizing agent, wherein
the buffering agent includes phosphate and organic acid salt,
the pH adjusting agent includes hydrochloric acid and sodium hydroxide,
the stabilizer includes glycerin, propylene glycol, and sulfite,
the isotonizing agent includes sodium chloride, glucose, sucrose, and mannitol,
the animal or vegetable oil and fat includes olive oil, soybean oil, and liver oil,
the hydrocarbons includes liquid paraffin, squalene, and squalane,
the fatty acid includes oleic acid and myristic acid,
the fatty acid esters includes ethyl oleate, octyldodecyl myristate, cetyl 2-ethyl-hexanoate, isopropyl myristate, sucrose fatty acid ester, glycerin fatty acid ester, sorbitan fatty acid ester, and propylene glycol fatty acid ester,
the solubilizer or solubilizing agent includes glycerin, propylene glycol, macrogol, and ethanol.

The present hydrogel formulation includes, for example, a formulation prepared by dissolving or dispersing an antigen (tumor antigen or pathogen-derived antigen), and/or the compound of formula (1) or a pharmaceutically acceptable salt thereof in water and adding viscosity to the formulation. The additive used in the present hydrogel formulation includes, for example, purified water, water for injection, a buffering agent, a pH adjusting agent, a stabilizer, an isotonizing agent, a solubilizer, a solubilizing agent, and a thickener, wherein
the buffering agent includes phosphate and organic acid salt,
the pH adjusting agent includes hydrochloric acid and sodium hydroxide,
the stabilizer includes glycerin, propylene glycol, and sulfite,
the isotonizing agent includes sodium chloride, glucose, sucrose, and mannitol,
the solubilizer or solubilizing agent includes glycerin, propylene glycol, macrogol, and ethanol,
the thickener includes carmellose sodium, poloxamers, and povidones.

The compound of formula (1), or a pharmaceutically acceptable salt thereof, or a pharmaceutical composition of the present invention may be used in combination with further another medicament (also referred to as combination drug) besides the above tumor antigen.

In an embodiment, the compound of formula (1), or a pharmaceutically acceptable salt thereof, or a pharmaceutical composition of the present invention may be administered in combination with an "immunomodulator", besides the above-mentioned tumor antigen. As used herein, the term "immunomodulator" means any agent that controls transmission of costimulatory signals generated during T cell activation by antigen-presenting cells by interacting with molecules which are involved in the transmission of the costimulatory signals and are present on the antigen-presenting cells and/or T cells, as well as any agent that directly or indirectly controls function of molecules involved in establishment of immune tolerance (immunosuppression) in the immune system. Since a tumor antigen peptide is effective for increasing tumor-reactive CTLs in a tumor, it is potentially useful as an agent for coadministration with an immunomodulator, for lowering a necessary dose of an immunomodulator or reducing adverse event caused by an immumonodulator. Thus, the present disclosure provides, through the use of a WT1 antigen peptide in combination with an immumomodulator, patients with a therapy having improved efficacy and safety.

The "immunomodulator" can be an agent in the form of an antibody, a nucleic acid, a protein, a peptide, or a small molecules, but is not limited thereto. The "antibody" as the "immunomodulator" includes an antibody fragment. Examples of the antibody fragment include heavy and light chain variable regions of an antibody (VH and VL), F(ab')2, Fab', Fab, Fv, Fd, sdFv, and scFV. The "protein" as the "immunomodulator" means any protein other than antibodies. Examples of the "immunomodulator" include, for example, immune checkpoint inhibitors, costimulatory molecule agonists, immune activating agents, and small molecule inhibitors.

The "immune checkpoint inhibitor" inhibits immunosuppressive effect induced by cancer cells or antigen presenting cells. Examples of the immune checkpoint inhibitor include, but not limited to, agents against a molecule selected from the group consisting of: (1) CTLA-4 (e.g., ipilimumab and tremelimumab); (2) PD-1 (e.g., nivolumab, pembrolizumab, AMP-224, AMP-514 (MEDI0680), and pidilizumab (CT-011)); (3) LAG-3 (e.g., IMP-321 and BMS-986016); (4) BTLA; (5) KIR (e.g., IPH2101); (6) TIM-3 (e.g., LY3321367 and CA-327); (7) PD-L1 (e.g., durvalumab (MEDI4736), MPDL3280A, BMS-936559, avelumab (MSB0010718C), BMS-1001, BMS-1116, and CA-170,CA-327); (8) PD-L2; (9) B7-H3 (e.g., MGA-271); (10) B7-H4; (11) HVEM; (12) GAL9; (13) CD160; (14) VISTA (e.g., onvatilimab (JNJ-61610588), HMBD-002, and CA-170); (15) BTNL2; (16) TIGIT; (17) PVR; (18) BTN1A1; (19) BTN2A2; (20) BTN3A2 (Nat Rev Drug Discov. 2013; 12: 130-146; Nikkei Medical Cancer Review 2014; 9; Nat Rev Immunol. 2014; 14: 559-69); (21) CSF1-R; (22) VSIG-3; (23) CD112; (24) CD112R; and (25) CD96.

The "costimulatory molecule agonist" enhances T cell activation by transmission of an auxiliary signal via a costimulatory molecule on the T cells and/or antigen-presenting cells, and attenuates the immunosuppressive effect of cancer cells or antigen presenting cells. Examples of the costimulatory molecule agonist include, but not limited to, agents against a molecule selected from the group consisting of: (1) 4-1BB; (2) 4-1BB-L; (3) OX40; (4) OX40-L; (5) GITR; (6) CD28; (7) CD40; (8) CD40-L; (9) ICOS; (10) ICOS-L; (11) LIGHT; (12) CD27; and (13) DNAM-1.

The "immune activating agent" efficiently stimulates killer T cells in the lymph nodes by directly or indirectly activating immune cells such as T cells and dendritic cells. Examples of the immune activating agent include, but not limited to, Toll-like receptor (TLR) agonists, stimulator of interferon genes (STING) agonists, cytokines, and agents against heat shock protein (HSP).

Examples of the "Toll-like receptor (TLR) agonist" include, but not limited to, TLR1/2 agonists, TLR2 agonists, TLR3 agonists (e.g., PolyI:C), TLR4 agonists (e.g., S-type lipopolysaccharide, paclitaxel, lipid A, and monophosphoryl lipid A), TLR5 agonists (e.g., flagellin), TLR6/2 agonists (e.g., MALP-2), TLR7 agonist, TLR7/8 agonists (e.g., gardiquimod, imiquimod, loxoribine, and resiquimod (R848)), TLR7/9 agonists (e.g., hydroxychloroquine sulfate), TLR8 agonists (e.g., motolimod (VTX-2337)), TLR9 agonists (e.g., CpG-ODN), and TLR11 agonists (e.g., profilin).

Examples of the "cytokine" include, but not limited to, IL-1α, IL-1β, IL-2, IL-3, IL-4, IL-5, IL-6, IL-7, IL-8, IL-9, IL-10, IL-11, IL-12, IL-13, IL-14, IL-15, IL-16, IL-17, IL-18, interferon (INF)-α, INF-β, INF-γ, SCF, GM-CSF, G-CSF, M-CSF, erythropoietin, thrombopoietin, macrophage inflammatory protein (MIP), and monocyte chemoattractant protein (MCP).

Examples of the "heat shock protein (HSP)" include, but not limited to, HSP70, HSP90, HSP90α, HSP90β, HSP105, HSP72, and HSP40. Agents against a heat shock protein include HSP inhibitors. Examples of inhibitors to HSP90 include, but not limited to, tanespimycin (17-AAG), luminespib (AUY-922, NVP-AUY922), alvespimycin (17-DMAG) hydrochloride, ganetespib (STA-9090), BIIB021, onalespib (AT13387), geldanamycin, NVP-BEP800, SNX-2112 (PF-04928473), PF-4929113 (SNX-5422), KW-2478, XL888, VER155008, VER-50589, CH5138303, VER-49009, NMS-E973, PU-H71, HSP990 (NVP-HSP990), and KNK437.

Examples of the "small molecule inhibitor" include, but not limited to, histone deacetylase inhibitors, histone demethylase inhibitors, histone acetyltransferase inhibitors, histone methyltransferase inhibitors, DNA methyltransferase inhibitors, anthracycline antibiotics, platinum agents, MAPK inhibitors, β-catenin inhibitors, STAT3 inhibitors, NF-kB inhibitors, JAK inhibitors, mTOR inhibitors, IDO inhibitors, COX-2 inhibitors, CXCR4 inhibitors, and arginase inhibitors.

Examples of the "histone deacetylase inhibitor" include, but not limited to, vorinostat (SAHA, MK0683), entinostat (MS-275), panobinostat (LBH589), trichostatin A (TSA), mocetinostat (MGCD0103), BG45, BRD73954, belinostat (PXD101), romidepsin (FK228, depsipeptide), 4SC-202, HPOB, LMK-235, CAY10603, tasquinimod, TMP269, nexturastat A, rocilinostat (ACY-1215), RGFP966, RG2833 (RGFP109), scriptaid, tubastatin A, pracinostat (SB939), CUDC-101, M344, PCI-34051, dacinostat (LAQ824), tubastatin A hydrochloride, abexinostat (PCI-24781), CUDC-907, AR-42, sodium phenylbutyrate, resminostat, tubacin, quisinostat (JNJ-26481585) dihydrochloride, MC1568, givinostat (ITF2357), droxinostat, chidamide (C S055, HBI-8000), CHR-2485, CHR-3996, DAC-060, FRM-0334 (EVP-0334), MGCD-290, CXD-101 (AZD-9468), CG200745, arginine butyrate, sulforaphane, SHP-141, CUDC-907, YM753 (OBP-801), sodium valproate, apicidin, and CI994 (tacedinaline).

Examples of the "histone demethylase inhibitor" include, but not limited to, GSK J4 HCl, OG-L002, JIB-04, IOX1, SP2509, ORY-1001 (RG-6016), GSK J1, ML324, and GSK-LSD1 2HCl.

Examples of the "histone acetyltransferase inhibitor" include, but not limited to, C646, MG149, remodelin, and anacardic acid.

Examples of the "histone methyltransferase inhibitor" include, but not limited to, pinometostat (EPZ5676), EPZ005678, GSK343, BIX01294, tazemetostat (EPZ6438), 3-deazaneplanocin A (DZNeP) HCl, UNC1999, MM-102, SGC0946, entacapone, EPZ015666, UNC0379, EI1, MI-2 (menin-MLL inhibitor), MI-3 (menin-MLL inhibitor), PFI-2, GSK126, EPZ04777, BRD4770, GSK-2816126, and UNC0631.

Examples of the "DNA methyltransferase inhibitor" include, but not limited to, decitabine, azatidine, RG108, thioguanine, zebularine, SGI-110, CC-486, SGI-1027, lomeguatrib, and procainamide hydrochloride.

The "anthracycline antibiotic" is intercalated between DNA strands to inhibit DNA relaxation. Examples of the anthracycline antibiotic include, but not limited to, doxorubicin, liposomal doxorubicin, daunorubicin, pirarubicin, epirubicin, idarubicin, aclarubicin, amrubicin, aloin, and mitoxantrone.

Examples of the "platinum agents" include, but not limited to, cisplatin, carboplatin, miboplatin, nedaplatin, satraplatin (JM-126), oxaliplatin (ELOXATIN), triplatin tetranitrate, and DDS formulations thereof.

Examples of the "MAPK inhibitor" include, but not limited to, SB203580, doramapimod (BIRB796), SB202190 (FHPI), LY2228820, VX-702, SB239063, pexmetinib (ARRY-614), PH-797804, VX-745, and TAK-715.

Examples of the "β-catenin inhibitor" include, but not limited to, XAV-939, ICG-001, IWR-1-endo, Wnt-C59 (C59), LGK-974, KY02111, IWP-2, IWP-L6, WIKI4, and FH535.

Examples of the "STAT3 inhibitor" include, but not limited to, S3I-201, Stattic, niclosamide, nifuroxazide, napabucasin (BBI608), cryptotanshinone, HO-3867, WHI-P154, FLLL32, STA-21, WP1066, and SH-4-54.

Examples of the "NF-kB inhibitor" include, but not limited to, QNZ (EVP4593), sodium 4-aminosalicylate, JSH-23, phenethyl caffeate, sodium salicylate, andrographolide, and SC75741.

Examples of the "JAK inhibitor" include, but not limited to, ruxolitinib (INCB018424), tofacitinib (CP-690550) citrate, AZD1480, fedratinib (SAR302503, TG101348), AT9283, tyrphostin B42 (AG-490), momelotinib (CYT387), tofacitinib (CP-690550, tasocitinib), WP1066, TG101209, gandotinib

(LY2784544), NVP-BSK805 2HCl, baricitinib (LY3009104, INCB02850), AZ960, CEP-33779, pacritinib (SB1518), WHI-P154, XL019, S-ruxolitinib (INCB018424), ZM39923 HCl, decernotinib (VX-509), cerdulatinib (PRT062070, PRT2070), filgotinib (GLPG0634), FLLL32, peficitinib (ASP015K, JNJ-54781532), GLPG0634 analogue, Go6976, and Curcumol.

Examples of the "mTOR inhibitor" include, but not limited to, sirolimus (rapamycin), deforolimus (AP23573, MK-8669), everolimus (RAD-001), temsirolimus (CCI-779, NSC683864), zotarolimus (ABT-578), biolimus A9 (umirolimus), AZD8055, KU-0063794, voxtalisib (XL765, SAR245409), MHY1485, dactolisib (BEZ235, NVP-BEZ235), PI-103, and torkinib (PP242).

Examples of the "IDO inhibitor" include, but not limited to, NLG919, INCB024360 analog, indoximod (NLG-8189), and epacadostat (INCB024360).

Examples of the "COX-2 inhibitor" include, but not limited to, valdecoxib, rofecoxib, carprofen, celecoxib, lumiracoxib, tolfenamic acid, nimesulide, niflumic acid, asaraldehyde, lornoxicam, sodium meclofenamate, amfenac sodium hydrate, diclofenac sodium, ketoprofen, ketorolac, naproxen sodium, indomethacin, ibuprofen, aspirin, mefenamic acid, bromfenac sodium, oxaprozin, zaltoprofen, and nepafenac.

Examples of the "CXCR4 inhibitor" include, but not limited to, WZ811, plerixafor (AMD3100), and plerixafor 8HCl (AMD3100 8HCl).

The compound of formula (1), or a pharmaceutically acceptable salt thereof, or the composition as described herein may also be used in combination with one or more drugs selected from the group consisting of "hormone therapy agent", "immunotherapeutic agent", "biopharmaceutical", "cell growth factor", "cell growth factor inhibitor", "cell growth factor receptor inhibitor", "radiotherapeutic agent", "auxiliary agent", and "chemotherapeutic agent". For example, one to five drugs, one to three drugs, or one drug selected from the above group of drugs may be used in combination with the peptide or the compound of formula (1), or a pharmaceutically acceptable salt thereof, or a combination thereof as described herein.

Examples of the "hormone therapy agent" include adrenal cortical hormone agents (e.g., steroidal anti-inflammatory agents, estrogen preparations, progesterone preparations, and androgen preparations), anti-estrogen agents, estrogen-controlling agents, estrogen synthesis inhibitors, antiandrogen agents, androgen-controlling agents, androgen synthesis inhibitors, LH-RH agonist preparations, LH-RH antagonist preparations, aromatase inhibitors, steroidlactonase inhibitors, contraceptive pills, retinoids, and agents which delay metabolism of a retinoid.

Examples of the "hormone therapy agent" include fosfestrol, diethylstilbestrol, fluoxymesterol, chlorotrianisene, methyl testosterone, medroxyprogesterone acetate, megestrol acetate, chlormadinone acetate, cyproterone acetate, danazol, allylestrenol, gestrinone, mepartricin, raloxifene, ormeloxifene, levormeloxifene, tamoxifen citrate, toremifene citrate, iodoxyfene, pill formulations, mepitiostane, testololactone, aminoglutethimide, goserelin acetate, buserelin, leuprorelin, leuprolide, droloxifene, epitiostanol, ethinylestradiol sulfonate, estramustine, fadrozole hydrochloride, anastrozole, terorazole, ketoconazole, letrozole, exemestane, vorozole, formestane, exemestane, flutamide, bicalutamide, nilutamide, enzalutamide, mifepristone, finasteride, dexamethasone, prednisolone, betamethasone, triamcinolone, abiraterone, liarozole, bexarotene, and DN101.

Examples of the "immunotherapeutic agent" include picibanil, krestin, sizofiran, lentinan, ubenimex, interferon (IL)-α, interferon (IL)-β, interferon (IL)-γ, interleukin, macrophage colony stimulating factor, granulocyte-colony stimulating factor, erythropoietin, lymphotoxin, BCG vaccine, *Corynebacterium parvum,* levamisole, polysaccharide K, procodazole, anti-CTLA4 antibody, anti-PD-1 antibody, and TLR agonists (e.g., TLR7 agonists, TLR8 agonists, TLR9 agonists).

Examples of the "biopharmaceutical" include, but not limited to, interleukin-2 (aldesleukin), interferon-α, interferon-β, interferon-γ, erythropoietin (EPO), granulocyte-colony stimulating factor (filgrastim), granulocyte-macrophage-colony stimulating factor (sargramostim), IL13-PE38QQR, Bacille Calmette-Guerin, levamisole, octreotide, CPG7909, Provenge, GVAX, Myvax, Favld, lenalidomide, trastuzumab, rituximab, gemtuzumab ozogamicin, alemtuzumab, endostatin, ibritumomab tiuxetan, tositumomab, cetuximab, zanolimumab, ofatumumab, HGS-ETR1, pertuzumab, M200, SGN-30, matuzumab, adecatumumab, denosumab, zalutumumab, MDX-060, nimotuzumab, MORAb-003, Vitaxin, MDX-101, MDX-010, DPC4 antibodies, NF-1 antibodies, NF-2 antibodies, Rb antibodies, p53 antibodies, WT1 antibodies, BRCA1 antibodies, BRCA2 antibodies, ganglioside (GM2), prostate specific antigens (PSA), α-fetoprotein (AFP), carcinoembryonic antigens (CEA), melanoma-associated antigens (MART-1, gap100, MAGE 1,3 tyrosine), papilloma virus E6 and E7 fragments, and DDS formulations thereof.

Regarding the "cell growth factor", "cell growth factor inhibitor" and "cell growth factor receptor inhibitor", cell growth factor may be any agent that promotes cell proliferation. For example, a cell growth factor may be a peptide having a molecular weight of not more than 20,000 which can bind to a receptor to function at a low concentration.

Examples of the "cell growth factor" include, but not limited to, epidermal growth factor (EGF), insulin-like growth factor (IGF (e.g., insulin, IGF-1, and IGF-2)), transforming growth factor (TGF (e.g., TGF-α and TGF-β)), nerve growth factor (NGF), brain-derived neurotrophic factor (BDNF), vascular endothelial growth factor (VEGF), colony stimulating factor (CSF (e.g., granulocyte-colony stimulating factor (G-CSF)), granulocyte-macrophage-colony stimulating factor (GM-CSF)), platelet-derived growth factor (PDGF), erythropoietin (EPO), fibroblast growth factor (FGF (e.g., acidic FGF, basic FGF, keratinocyte growth factor (KGK), and FGF-10)), hepatocyte growth factor (HGF), heregulin, and angiopoietin. The term "cell growth factor" is synonymous with the term "growth factor".

Examples of the "cell growth factor inhibitor" include, but not limited to, epidermal growth factor inhibitors (EGF inhibitors), insulin-like growth factor inhibitors (IGF inhibitors), nerve growth factor inhibitors (NGF inhibitors), brain-derived neurotrophic factor inhibitors (BDNF inhibitors), vascular endothelial cell growth factor inhibitors (VEGF inhibitors), colony stimulating factor inhibitors (CSF inhibitors), platelet-derived growth factor inhibitors (PDGF inhibitors), erythropoietin inhibitors (EPO inhibitors), fibroblast growth factor inhibitors (FGF inhibitors), hepatocyte growth factor inhibitors (HGF inhibitors), heregulin inhibitors, and angiopoietin inhibitors. The term "cell growth factor inhibitor" is synonymous with the term "growth factor inhibitor".

Examples of the "cell growth factor receptor inhibitor" include, but not limited to, epidermal growth factor receptor inhibitors (EGFR inhibitors), insulin-like growth factor receptor inhibitors (IGFR inhibitors), nerve growth factor receptor inhibitors (NGFR inhibitors), brain-derived neurotrophic factor receptor inhibitors (BDNFR inhibitors), vascular endothelial cell growth factor receptor inhibitors (VEGFR inhibitors), colony stimulating factor inhibitors (CSF inhibitors), platelet-derived growth factor receptor inhibitors (PDGFR inhibitors), erythropoietin receptor inhibitors (EPOR inhibitors), fibroblast growth factor receptor inhibitors (FGFR inhibitors), hepatocyte growth factor receptor inhibitors (HGFR inhibitors), heregulin receptor inhibitors, and angiopoietin receptor inhibitors. The term "cell growth factor receptor inhibitor" is synonymous with the term "growth factor receptor inhibitor".

Examples of the "radiotherapeutic agent" include, but not limited to, radioactive materials and radiosensitizers.

The "auxiliary agent" is an agent used together with an anticancer agent for suppressing a side effect or vomiting caused by the anticancer agent. Examples of the "auxiliary agent" include, but not limited to, aprepitant, ondansetron, lorazepam, dexamethasone, diphenhydramine, ranitidine, cimetidine, ranitidine, famotidine, cimetidine, Procrit, epoetin alfa, filgrastim, oprelvekin, leucovorin, and granulocyte-macrophage-colony stimulating factor (GM-CSF).

Examples of the "chemotherapeutic agent" include, but not limited to, alkylating agents, platinum agents, antimetabolites, topoisomerase inhibitors, DNA intercalators, antimitotic agents, antitumor antibiotics, plant-derived anticancer agents, epigenetic drugs, immunomodulators, molecular targeted drugs, angiogenesis inhibitors, and other chemotherapeutic agents. Some typical examples of chemotherapeutic agent are listed below.

Examples of the "alkylating agent" include, but not limited to, nitrogen mustard, nitrogen mustard N-oxide hydrochloride, chlorambucil, cyclophosphamide, ifosfamide, thiotepa, carboquone, improsulfan tosylate, busulfan, nimustine hydrochloride, mitobronitol, melphalan, dacarbazine, procarbazine, ranimustine, estramustine sodium phosphate, triethylenemelamine, carmustine, lomustine, streptozocin, pipobroman, etoglucid, altretamine, ambamustine, dibrospidium hydrochloride, fotemustine, prednimustine, bendamustine, uramustine, semustine, pumitepa, ribomustin, temozolomide, treosulfan, trofosfamide, zinostatin stimalamer, adozelesin, cystemustine, bizelesin, mechlorethamine, uracil mustard, streptozocin, trabectedin, becaterin, chlormethine, mannosulfan, triaziquone, procarbazine, canfosfamide, nitrosoureas, and DDS formulations thereof.

Examples of the "platinum agents" include, but not limited to, cisplatin, carboplatin, miboplatin, nedaplatin, satraplatin, oxaliplatin, triplatin tetranitrate, and DDS formulations thereof.

Examples of the "antimetabolite" include, but not limited to, antifolates, pyrimidine metabolism inhibitors, purine metabolism inhibitors, ribonucleotide reductase inhibitors, and nucleotide analogs.

Examples of the "antimetabolite" include, but not limited to, mercaptopurine, 6-mercaptopurine riboside, thioinosine, methotrexate, pemetrexed, eoshitabin, enocitabine, cytarabine, cytarabine ocfosfate, ancitabine hydrochloride, 5-FU agents (e.g., fluorouracil, Carzonal, Bennan, Lunachol, Lunapon, tegafur, tegafur-uracil, tegafurgimeracil-oteracil potassium (TS-1), UFT, doxifluridine, carmofur, gallocitabine, emitefur, and capecitabine), aminopterin, nelarabine, leucovorin calcium, Tabloid, butocine, folinate calcium, levofolinate calcium, cladribine, emitefur, fludarabine, gemcitabine, hydroxycarbamide, pentostatin, piritrexim, idoxuridine, mitoguazone, tiazofurine, ambamustine, bendamustine, floxuridine, nelarabine, leucovorin, hydroxyurea, thioguanine, asparaginase, bortezomib, raltitrexed, clofarabine, enocitabine, sapacitabine, azacytidine, sulfadiazine, sulfamethoxazole, trimethoprim, Liproxstatin-1, D4476, Xanthohumol, Epacadostat (INCB024360), Vidofludimus, P7C3, GMX1778 (CHS828), NCT-501, SW033291, Ro61-8048, and DDS formulations thereof.

Examples of the "topoisomerase inhibitor" include, but not limited to, doxorubicin, daunorubicin, epirubicin, idarubicin, anthracenedione, mitoxantrone, mitomycin C, bleomycin, dactinomycin, plicatomycin, irinotecan, camptothecin, rubitecan, belotecan, etoposide, teniposide, topotecan, amsacrine, and DDS formulations thereof.

Examples of the "DNA intercalator" include, but not limited to, proflavine, doxorubicin (adriamycin), daunorubicin, dactinomycin, thalidomide, and DDS formulations thereof.

Examples of the "antimitotic agent" include, but not limited to, paclitaxel, paclitaxel derivatives (e.g., DHA paclitaxel, paclitaxel polyglutamate, nab-paclitaxel, micellar paclitaxel, 7α-glucosyloxyacetylpaclitaxel, and BMS-275183), docetaxel, vinorelbine, vincristine, vinblastine, vindesine, vinzolidine, etoposide, teniposide, ixabepilone, larotaxel, ortataxel, tesetaxel, ispinesib, colchicine, vinflunine, and DDS formulations thereof.

Examples of the "antitumor antibiotic" include, but not limited to, actinomycin D, actinomycin C, mitomycin C, chromomycin A3, mithramycin A, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, amrubicin hydrochloride, neocarzinostatin, zinostatin stimalamer, mithramycin, sarkomycin, carzinophilin, mitotane, zorubicin hydrochloride, mitoxantrone hydrochloride, idarubicin hydrochloride, liposomal doxorubicin, and DDS formulations thereof.

Examples of the "plant-derived anticancer agent" include, but not limited to, irinotecan, nogitecan, etoposide, etoposide phosphate, eribulin, sobuzoxane, vinblastine sulfate, vincristine sulfate, vindesine sulfate, teniposide, paclitaxel, paclitaxel injection, docetaxel, DJ-927, vinorelbine, topotecan, and DDS formulations thereof.

Examples of the "epigenetic drug" include, but not limited to, DNA methylation inhibitors, histone deacetylase (HDAC) inhibitors, DNA methyl transferase (DNMT) inhibitors, histone deacetylase activators, histone demethylase inhibitors, and methylated nucleotides.

Specific examples of the "epigenetic drug" include, but not limited to, vorinostat, belinostat, mocetinostat (MGCD0103), entinostat (SNDX-275), romidepsin, azacytidine, decitabine, GSK2879552 2Hl, SGC707, ORY-1001 (RG-6016), PFI-4, SirReal2, GSK2801, CPI-360, GSK503, AMI-1, CPI-169, and DDS formulations thereof.

Examples of the "immunomodulator" include, but not limited to, thalidomide, lenalidomide, pomalidomide, and DDS formulations thereof.

The "molecular targeted drug" can be a small molecules or an antibody. Examples of the "molecular targeted drug" include, but not limited to, kinase inhibitors, proteasome inhibitors, monoclonal antibodies, mTOR inhibitors, TNF inhibitors, and T-cell inhibitors.

Examples of the "kinase inhibitor" include, but not limited to, tyrosine kinase inhibitors, serine/threonine kinase inhibitors, Raf kinase inhibitors, cyclin-dependent kinase (CDK) inhibitors, and mitogen-activated protein kinase (MEK) inhibitors.

Specific examples of the "kinase inhibitor" include, but not limited to, imatinib, gefitinib, erlotinib, afatinib, dasatinib, bosutinib, vandetanib, sunitinib, axitinib, pazopanib, lenvatinib, lapatinib, nintedanib, nilotinib, crizotinib, ceritinib, alectinib, ruxolitinib, tofacitinib, ibrutinib, sorafenib, vemurafenib, dabrafenib, palbociclib, trametinib, regorafenib, cedivanib, lestaurtinib, bandetinib, vatalanib, seliciclib, tivantinib, canertinib, pelitinib, tesevatinib, cediranib, motesanib, midostaurin, foretinib, cabozantinib, selumetinib, neratinib, volasertib, saracatinib, enzastaurin, tandutinib, semaxanib, alvocidib, ICR-62, AEE788, PD0325901, PD153035, TK787, amcasertib (BBI503), E6201, E7050, and DDS formulations thereof.

Examples of the "proteasome inhibitor" include, but not limited to, bortezomib, carfilzomib, and DDS formulations thereof.

Examples of the "monoclonal antibody" include, but not limited to, anti-CD22 antibodies, anti-CD20 antibodies, anti-CD25 antibodies, anti-CD30 antibodies, anti-CD33 antibodies, anti-CD5 antibodies, anti-CD52 antibodies, antiepidermal growth factor receptor antibodies (EGFR antibodies), anti-vascular endothelial cell growth factor antibodies (VEGF antibodies), anti-TNF-a antibodies, anti-IL-1 receptor antibodies, anti-IL-2 receptor antibodies, anti-IL-5 receptor antibodies, anti-IL-6 receptor antibodies, anti-HER2 antibodies, anti-IgE antibodies, anti-IgG antibodies, anti-RS virus antibodies, anti-CCR4 antibodies, anti-cytotoxic T lymphocyte-associated antigen 4 (CTLA-4, CD152) antibodies, anti-PD-1 antibodies, anti-receptor activator of nuclear factor κB ligand (RANKL) antibodies, anti-c-Met antibodies, and anti-CXCR4 antibodies.

Specific examples of the "monoclonal antibody" include, but not limited to, ibritumomab tiuxetan, rituximab, cetuximab, infliximab, basiliximab, brentuximab vedotin, tocilizumab, trastuzumab, bevacizumab, omalizumab, mepolizumab, gemtuzumab, ozogamicin, palivizumab, ranibizumab, certolizumab, ocrelizumab, mogamulizumab, eculizumab, pertuzumab, alemtuzumab, inotuzumab, panitumumab, ofatumumab, golimumab, adalimumab, ramucirumab, nivolumab, anakinra, denosumab, ipilimumab, pembrolizumab, matuzumab, farletuzumab, MORAb-004, MORA-b009, and DDS formulations thereof.

Examples of the "mTOR inhibitor" include, but not limited to, everolimus (RAD001), rapamycin (sirolimus), AZD8055, temsirolimus (CCI-779, NSC683864), KU-0063794, voxtalisib (XL-765, SAR245409), MHY1485, dactolisib (BEZ235), PI-103, torkinib (PP242), ridaforolimus (deforolimus, MK-8669), INK-128 (MLN0128), Torin1, omipalisib (GSK2126458, GSK458), OSI-027, PF-04691502, apitolisib (GDC-0980, RG7422), GSK1059615, gedatolisib (PF-05212384, PKI-587), WYE-132, PP121, WYE-354, AZD2014, Torin2, WYE-687, CH5132799, WAY-600, ETP-46464, GDC-0349, XL388, zotarolimus (ABT-578), tacrolimus (FK506), BGT226 (NVP-BGT226), Palomid 529 (P529), chrysophanic acid, and DDS formulations thereof.

Examples of the "TNF inhibitor" include, but not limited to, etanercept, lenalidomide (CC-5013), pomalidomide, thalidomide, necrostatin-1, and QNZ (EVP4593).

Examples of the "T-cell inhibitor" include, but not limited to, abatacept.

Examples of the "angiogenesis inhibitor" include, but not limited to, CM101, IFN-α, IL-12, platelet factor-4, suramin, semaxanib, thrombospondin, VEGFR antagonists, combinations of an angiostatic steroid and heparin, cartilage-derived angiogenesis inhibitors, matrix metalloproteinase inhibitors, batimastat, marimastat, angiostatin, endostatin, 2-methoxyestradiol, tecogalan, thrombospondin, αVβ3 inhibitors, linomide, ADH-1, E7820, and DDS formulations thereof.

Examples of the "other chemotherapeutic agent" include, but not limited to, finasteride, sobuzoxane, obatoclax, efaproxiral, tipifarnib, and lonafarnib.

The pharmaceutical composition of the present invention may further contain other additives, and examples of such additives include surfactant, antioxidants, preservatives, and soothing agents.

The compound of formula (1) or a pharmaceutically acceptable salt thereof may be administered simultaneously with or at any interval before or after the antigenic substance (immunogen) in a unit dose ranging from generally 5 to 5000 mg/m² of body surface area, i.e., about 0.1 ng/kg to 100 mg/kg, which provides an effective dose for vaccine adjuvant. The unit dosage form for injections generally contains, for example, 1 ng to 250 mg of the active ingredient, and preferably, used at a dose ranging from 1 ng to 50 mg/kg of the active ingredient per day. However, the daily dose may vary depending on the host to be treated, the route of administration and the severity of the disease being treated. Thus, the optimal dose can be determined by a practitioner who treats individual patient or warm-blooded animal.

The term "treatment" as used herein means alleviating some or all of the symptoms of disease, in whole or in part, or preventing or delaying the progression of disease.

The term "prevention" as used herein means primary prevention of disease (prevention of onset of disease) or secondary prevention of disease (prevention of relapse in a patient whose symptom has been alleviated or disease has been cured after the onset of the disease, prevention of recurrence).

Since the compound of the present invention or a pharmaceutically acceptable salt thereof has an immune adjuvant activity *in vitro* or *in vivo,* it is useful as a vaccine adjuvant for maintaining or enhancing the immunogenicity of the antigen (tumor antigen or pathogen-derived antigen).

The compound of the present invention or a pharmaceutically acceptable salt thereof has an adjuvant activity for cellular immunity *in vitro* or *in vivo,* and thus it is useful as a vaccine adjuvant for maintaining or enhancing the immunogenicity of tumor antigen.

The compound of the present invention or a pharmaceutically acceptable salt thereof can be used for maintaining or enhancing the effect of an immunostimulant for treating or preventing a disease, that is a substance inducing an antigen (tumor antigen or pathogen-derived antigen)-specific immune reaction.

The pharmaceutical composition comprising the compound of the present invention or a pharmaceutically acceptable salt thereof, and a substance enhancing the specific immune response for tumor antigen or pathogen (also referred to as tumor antigen or pathogen-derived antigen) is also included in one embodiment of the present invention. The tumor antigen includes, but not limited to, an antigen protein or an antigen peptide (partial peptide) derived from said antigen protein, a tumor antigen protein or a tumor antigen peptide (partial peptide) derived from said tumor antigen protein, or a complex thereof with a carrier.

In a specific embodiment of the present invention, the present compound or a pharmaceutically acceptable salt thereof can treat or prevent cancer by the administration with a tumor antigen protein or a tumor antigen peptide for cancer immunotherapy. The cancer includes, for example, leukemia, myelodysplastic syndrome, multiple myeloma, malignant lymphoma, stomach cancer, colon cancer, lung cancer, breast cancer, germ cell cancer, liver cancer, skin cancer, bladder cancer, prostate cancer, uterine cancer, cervical cancer, ovarian cancer, brain tumor, bone cancer, pancreatic cancer, head and neck cancer, skin or intraorbital malignant melanoma, rectal cancer, anal cancer, testicular cancer, fallopian tube carcinoma, endometrial carcinoma, uterocervical carcinoma, vaginal carcinoma, vulval carcinoma, Hodgkin's disease, non-Hodgkin's lymphoma, esophageal cancer, small intestinal cancer, endocrine system cancer, thyroid cancer, parathyroid cancer, adrenal cancer, soft tissue sarcoma, urethral cancer, penile cancer, acute myeloid leukemia, chronic myeloid leukemia, acute lymphoblastic leukemia, chronic or acute leukemia including chronic lymphocytic leukemia, children solid cancer, lymphocytic lymphoma, renal/ureter cancer, renal pelvic carcinoma, central nervous system (CNS) tumor, primary CNS lymphoma, tumor angiogenesis, spinal tumor, pontine glioma, pituitary adenoma, Kaposi's sarcoma, squamous cell carcinoma, planocellular carcinoma, T-cell lymphoma, polytypic glioblastoma, malignant melanoma, non-small-cell lung cancer, renal cell cancer, and asbestos-induced cancer. The treatment or prevention of cancer includes preventing metastatic disease and tumor recurrence, and preventing and treating paraneoplastic syndrome.

In a specific embodiment, the compound of the present invention or a pharmaceutically acceptable salt thereof, by administering in combination with an active ingredient of a vaccine for preventing infectious diseases, can prevent various infectious diseases such as genital wart, common wart, plantar wart, hepatitis B, hepatitis C, herpes simplex virus, molluscum contagiosum, smallpox, human immunodeficiency virus (HIV), human papilloma virus (HPV), RS virus, norovirus, cytomegalovirus (CMV), varicella zoster virus (VZV), rhinovirus, adenovirus, coronavirus, influenza, and parainfluenza; bacterial diseases such as tuberculosis, mycobacterium avium, and Hansen's disease; infections such as mycosis, chlamydia, Candida, Aspergillus, cryptococcal meningitis, Pneumocystis carini, cryptosporidiosis, histoplasmosis, toxoplasmosis, malaria, Trypanosoma infection, and leishmaniasis. Examples of the active ingredient of the vaccine for preventing infectious include, but not limited to, substances derived from microorganisms/pathogens including bacteria, fungi, protozoa, and viruses which cause infectious diseases, such as antigenic protein, antigen peptide (partial peptide) from said antigenic protein, polysaccharide, lipid, and a combination thereof or a combination of the substance derived from said microorganisms/pathogen and a carrier.

Examples of the viral antigenic peptide derived from the viral antigen include, but not limited to, influenza matrix protein peptide 58-66 (Jager E et al., Int. J. Cancer 67: 54 (1996)), HPV16 E7 peptide 86-93 (van Driel WJ et al., Eur. J. Cancer 35:946 (1999)), HPV E7 peptide 12-20 (Scheibenbogen C et al., J. Immunother 23: 275 (2000)), HPV16 E7 peptide 11-20 (Smith JWI et al., J. Clin. Oncol. 21: 1562 (2003)), HSV2 gD (Berman PW et al., Science 227: 1490 (1985)), CMV gB (Frey SE et al., Infect Dis. 180: 1700 (1999), Gonczol E. et al., Exp. Opin. Biol. Ther. 1: 401 (2001)), and CMV pp65 (Rosa CL et al., Blood 100: 3681 (2002), Gonczol E. et al., Exp. Opin. Biol. Ther. 1: 401 (2001)).

The carrier as used herein is a substance, such as protein and lipid, to which an antigenic protein or an antigenic peptide is bound chemically and/or physically, and examples include, but not limited to, CRM 197 (Vaccine. 2013 Oct 1; 31(42):4827-33), KLH (Cancer Immunol Immunother. 2003 Oct; 52(10):608-16), virus-like particles (PLoS ONE 5(3): e9809) and liposomes (J Liposome Res. 2004; 14(3-4):175-89).

The antigenic protein may be prepared by cloning cDNA, which encodes the antigenic protein, and expression in a host cell, according to a textbook such as Molecular Cloning 2nd ed., Cold Spring Harbor Laboratory Press (1989).

The synthesis of the antigenic peptide can be carried out according to a method generally used in peptide chemistry, for example, as described in literatures (Peptide Synthesis, Interscience, New York, 1966; The Proteins, Vol. 2, Academic Press Inc., New York, 1976).

In an embodiment, the present invention further provides a kit comprising:
a) a compound of the formula (1) or a pharmaceutically acceptable salt thereof, or a pharmaceutical composition comprising a compound of the formula (1) or a pharmaceutically acceptable salt thereof; and
b) an antigen (tumor antigen or pathogen-derived antigen) or a pharmaceutical composition comprising an antigen (tumor antigen or pathogen-derived antigen).

The antigen is not limited so long as it is an antigen that may be used as an active ingredient of vaccines, which includes antigenic proteins as mentioned above, antigenic peptides (partial peptides) derived from such antigenic proteins, and a complex thereof with a carrier.

In an embodiment, the present invention provides a kit comprising:
a) a compound of the formula (1) or a pharmaceutically acceptable salt thereof, or a pharmaceutical composition comprising a compound of the formula (1) or a pharmaceutically acceptable salt thereof; and
b) a tumor antigen or a pharmaceutical composition comprising a tumor antigen.

The tumor antigen herein should not be limited as long as the tumor antigen can be used as an active ingredient for a cancer vaccine, which includes the above-mention tumor antigen protein or a tumor antigen peptide (partial peptide) derived from said antigen protein, and further a complex thereof with a carrier.

In an embodiment, the present invention provides a kit comprising:
a) a compound of the formula (1) or a pharmaceutically acceptable salt thereof, or a pharmaceutical composition comprising a compound of the formula (1) or a pharmaceutically acceptable salt thereof; and
b) a pathogen-derived antigen or a pharmaceutical composition comprising a pathogen-derived antigen.

The pathogen-derived antigen herein should not be limited as long as the pathogen-derived antigen can be used as an active ingredient for an infective vaccine, which includes the above-mention pathogen-derived antigen protein or a pathogen-derived antigen peptide (partial peptide) derived from said pathogen-derived antigen protein, and further a complex thereof with a carrier.

In one embodiment of the present invention, the present invention provides use of a compound of the formula (1) or a pharmaceutically acceptable salt thereof in the preparation of a vaccine adjuvant.

Further in one embodiment of the present invention, the present invention provides use of a compound of the formula (1) or a pharmaceutically acceptable salt thereof as a vaccine adjuvant in the preparation of a vaccine for treating cancer or infection.

In one embodiment of the present invention, the present invention provides use of a compound of the formula (1) or a pharmaceutically acceptable salt thereof in the preparation of a vaccine adjuvant for a cancer vaccine.

Further in one embodiment of the present invention, the present invention provides use of a compound of the formula (1) or a pharmaceutically acceptable salt thereof as a vaccine adjuvant in the preparation of a cancer vaccine for treating cancer.

In one embodiment of the present invention, there is provided a use of a compound of the formula (1), or a pharmaceutically acceptable salt thereof, for the manufacture of a vaccine adjuvant for infection vaccine.

In one embodiment of the present invention, there is provided a use of a compound of the formula (I) as defined above, or a pharmaceutically acceptable salt thereof, as a vaccine adjuvant in the manufacture of an infection vaccine for the treatment of an infection.

Further, one embodiment of the present invention provides a method for the treatment or prevention of cancer or infection, or the prevention of the progress thereof, comprising a step of administering a compound of the formula (I) as defined above, or a pharmaceutically acceptable salt thereof, together with an antigen (tumor antigen or pathogen-derived antigen), to a patient.

One embodiment of the present invention provides a method for the treatment or prevention of cancer, or the prevention of the progress thereof, comprising a step of administering a compound of the formula (I) as defined above, or a pharmaceutically acceptable salt thereof, together with a tumor antigen, to a patient.

One embodiment of the present invention provides a method for the treatment or prevention of infection, or the prevention of the progress thereof, comprising a step of administering a compound of the formula (I) as defined above, or a pharmaceutically acceptable salt thereof, together with a pathogen-derived antigen, to a patient.

The pharmaceutical composition of the present invention may comprise a compound disclosed in Non-Patent Literature 6 or Non-Patent Literature 7, besides the compound of formula (1) .

A compound disclosed in Non-Patent Literature 6 or Non-Patent Literature 7, or a pharmaceutically acceptable salt thereof, or a pharmaceutical composition comprising the compound or the pharmaceutically acceptable salt may be used as a vaccine adjuvant of a tumor antigen.

### EXAMPLES

The present invention will be further described with reference to the following examples which should not be regarded as limiting in any respect.

- Fmoc:: 9-fluorenylmethyloxycarbonyl
- Boc:: tert-butoxycarbonyl
- Alko:: p-alkoxybenzyl alcohol
- PEG:: polyethylene glycol
- tBu:: tert-butyl
- HBTU:: O-(benzotriazol-1-yl)-N,N,N',N'-tetramethyluronium hexafluorophosphate
- DIPEA:: N,N-diisopropylethylamine
- DMF:: N,N-dimethylformamide
- NMP:: N-methyl-2-pyrrolidone
- TFA:: trifluoroacetic acid
- TIS:: triisopropylsilane
- THF:: tetrahydrofuran
- TBS:: tert-butyldimethylsilyl group
- TBDPS:: tert-butyldiphenylsilyl group
- TBAF:: tetrabutylammonium fluoride

The analysis conditions of high performance liquid chromatograph-mass spectrometer (LCMS) are shown below.

### LCMS Condition A

MS detector: LCMS-IT-TOF
HPLC: Shimadzu Nexera X2 LC 30AD
Column: Kinetex 1.7 µ C18 100A New column 50 × 2.1 mm
Flow rate: 1.2 ml/min
Wave length: 254/220 nm
Mobile phase:
A: 0.1 % formic acid/water
B: acetonitrile

Time program:

| | |
|---|---|
| Step | Time (min) |
| 1 | 0.01-1.40 A:B = 90:10 - 5:95 |
| 2 | 1.40-1.60 A:B = 5:95 |
| 3 | 1.61-2.00 A:B = 99:1 |

### LCMS Condition B

MS detector: ACQUITY^{™} SQ detecter (Waters)
HPLC: ACQUITY^{™} system
Column: Waters ACQUITY^{™} UPLC BEH C18 (1.7 µm, 2.1 mm × 30 mm)
Flow rate: 0.8 ml/min
Wave length: 254/220 nm
Mobile phase:
   A: 0.06 % formic acid/acetonitrile
   B: 0.06 % formic acid/water
Time program: 0.0-1.30 A:B = 2:98 - 96:4
Column temperature: 25°C

### Reference example 1

### Synthesis of a peptide consisting of amino acid sequence: RMFPNAPYL (Arg-Met-Phe-Pro-Asn-Ala-Pro-Tyr-Leu)(SEQ ID NO: 1)

From 1.00 g of (Fmoc-Lys(Boc)-Alko-PEG Resin) (WATANABE CHEMICAL INDUSTRIES, LTD.; 0.23 mmol/g, 0.23 mmol) as a starting material, the peptide chain was elongated by solid-phase synthesis of Fmoc/tBu method. The solid-phase synthesis was done with CS336X peptide synthesizer (CSBio), and Fmoc group was deprotected by the treatment with 20 % piperidine in DMF for 5 minutes or 20 minutes. The coupling of the protected amino acid to the resin compound was done by reacting the resin compound with a solution of 1.05 mmol of the protected amino acid, 1 mmol of HBTU, and 2 mmol of DIPEA in DMF for one hour. The obtained resin was washed with DMF and ether and dried *in vacuo* to give a peptide resin. To the peptide resin was added 10 mL of a mixture of TFA/water/TIS (volume ratio: 94/2.5/2.5), and the mixture was shaken at room temperature for 2 hours. The resin was removed by filtration, and the reaction solution was concentrated under reduced pressure. The reaction solution was cooled at ice temperature, and diethyl ether (50 mL) was added thereto. The resulting precipitate was collected on a filter, washed with ether, and dried *in vacuo* to give a crude peptide. The obtained crude peptide was dissolved in a mixture of 20 % acetic acid/water and acetonitrile (volume ratio: 1/1), and purified according to the condition shown below to give trifluoroacetate of RMFPNAPYL (Arg-Met-Phe-Pro-Asn-Ala-Pro-Tyr-Leu) (SEQ ID NO: 1) (0.16 g). The obtained trifluoroacetate was converted to its acetate in a common manner, which was evaluated.
Mass spectrometry: m/z = 554.73 [M+2H]⁺², Retention time: 0.82 min (LCMS Condition A)
Purification condition
HPLC system: High throughput HPLC preparative system (Gilson)
Column: YMC ODS-A 3 cmϕ × 25 cm, 10 µm
Eluate 1: 0.1 % TFA/water
Eluate 2: 0.035 % TFA/acetonitrile
Flow rate: 20 mL/min
Gradient method:

| Time (min) | Concentration of Eluate 2 (%) |
|---|---|
| 0 | 10 |
| 25 | 50 |

According to the method described in Reference example 1, the peptides shown in Table 1 were prepared as their trifluoroacetate from each corresponding starting material. These compounds were dealt as reference examples since they are not within the present compounds. Reference example 3 was converted to its acetate in a common manner, which was evaluated as follows.

| Table 1 | | | |
|---|---|---|---|
| Reference example No. | SEQ ID No. | Amino acid sequence and structure | LCMS condition A (m/z, Retention time (min)) |
| 2 | 2 | CYTWNQMNL | 586.69 [M+2H]⁺², 0.87 |
| 3 | 3 | WAPVLDFAPPGASAYGSL | 910.30 [M+2H]⁺², 0.95 |
| 4 | 4 | KRYFKLSHLQMHSRKH | 420.2 [M+5H]⁺⁵, 0.64 |
| 5 | 5 | VLDFAPPGA | 884.4 [M-H]⁻, 0.75 |
| 6 | 6 | VLQELNVTV | 507.8 [M+2H]⁺², 1.14 |
| 7 | 7 | GLYDGMEHL | 517.7 [M+2H]⁺², 1.12 |
| 8 | 8 | KIFGSLAFL | 498.2 [M+2H]⁺², 1.34 |

According to the method described in WO 2014/157692, the compound shown in Table 2 (wherein the bond between C-C is disulfide bond) was prepared as its trifluoroacetate. The compound was dealt as a reference example since it is not within the present compounds.

| Table 2 | | | |
|---|---|---|---|
| Reference example No. | Formula No. | Structure | LCMS condition A (m/z, Retention time (min)) |
| 9 | 4 | 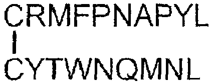 | 794.60 [M+3H]⁺³, 0.88 |

### Reference example 10

### Preparation of N-2,2,3,3-pentamethyl-4,7,10,13,16-pentaoxa-3-silaoctadecane-18-amine

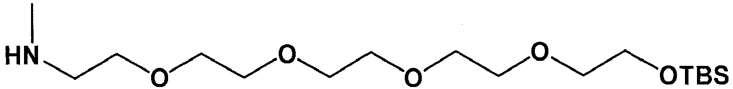

### Step 1

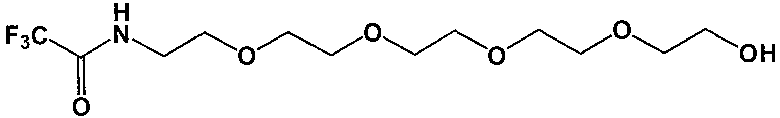

To a solution of 14-amino-3,6,9,12-tetraoxatetradecan-1-ol (1.60 g) which is a known compound in THF (25 mL) were added triethylamine (4.7 mL) and ethyl trifluoroacetate (2.4 mL), and the solution was stirred at room temperature for 2 hours. The reaction solution was concentrated under reduced pressure, and the obtained crude product was purified by silica gel chromatography (mobile phase: chloroform/methanol) to give 2,2,2-trifluoro-N-(14-hydroxy-3,6,9,12-tetraoxatetradecan-1-yl)acetamide (1.00 g).
m/z = 334 [M+H]⁺, Rt = 0.507 (LCMS Condition B)

### Step 2

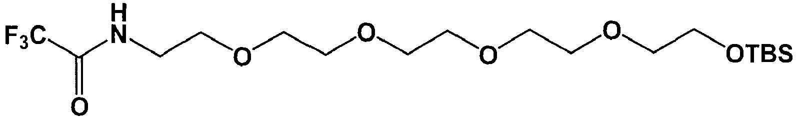

To a solution of the compound (3.91 g) prepared in Reference example 10 (Step 1) in DMF (20 mL) were added triethylamine (4.90 mL) and tert-butyldimethylchlorosilane (3.54 g), and the solution was stirred at room temperature for 2 hours. The reaction solution was diluted with ethyl acetate, washed with water and brine, dried over sodium sulfate, filtered, and concentrated under reduced pressure. The obtained crude product was purified by silica gel chromatography (mobile phase: hexane/ethyl acetate) to give 2,2,2-trifluoro-N-(2,2,3,3-tetramethyl-4,7,10,13,16-pentaoxa-3-silaoctadecan-18-yl)acetamide (3.70 g).
m/z = 448 [M+H]⁺, Rt = 1.153 (LCMS Condition B)

### Step 3

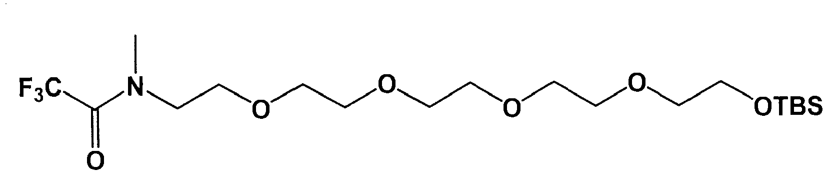

To a solution of the compound (4.44 g) prepared in Reference example 10 (Step 2) in DMF (20 mL) were added cesium carbonate (6.46 g) and methyl iodide (1.6 g), and the solution was stirred at room temperature for 2 hours. The reaction solution was diluted with ethyl acetate, washed with water and brine, dried over sodium sulfate, filtered, and concentrated under reduced pressure. The obtained crude product was purified by silica gel chromatography (mobile phase: hexane/ethyl acetate) to give 2,2,2-trifluoro-N-methyl-N-(2,2,3,3-tetramethyl-4,7,10,13,16-pentaoxa-3-silaoctadecan-18-yl)acetamide (3.31 g).
m/z = 463 [M+H]⁺, Rt = 1.210 (LCMS Condition B)

### Step 4

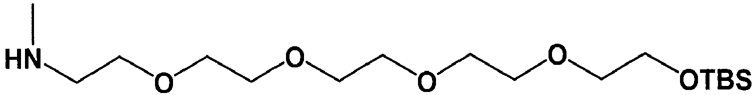

To a solution of the compound (117 mg) prepared in Reference example 10 (Step 3) in methanol (5 mL) was added potassium carbonate (70 mg), and the solution was stirred at room temperature for 5 hours. The reaction solution was concentrated, and the obtained crude product was purified by silica gel chromatography (mobile phase: chloroform/methanol) to give N-2,2,3,3-pentamethyl-4,7,10,13,16-pentaoxa-3-silaoctadecane-18-amine (67 mg).
m/z = 366 [M+H⁺], Rt = 0.718 (LCMS Condition B)

### Reference example 11

### Preparation of N,2,2-trimethyl-3,3-diphenyl-4,7,10,13,16-pentaoxa-3-silaoctadecane-18-amine

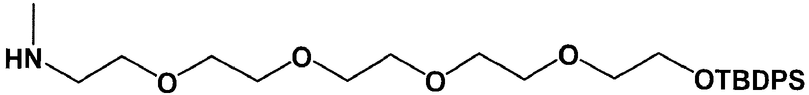

The title compound was prepared according to the process of Reference example 10.
m/z = 490 [M+H]⁺, Rt = 0.953 (LCMS Condition B)

### Example 1

### Preparation of 6-amino-2-butoxy-9-{[5-(16-hydroxy-2-methyl-5, 8, 11, 14-tetraoxa-2-azahexadecan-1-yl)pyridin-2-yl]methyl}-7,9-dihydro-8H-purin-8-one·trifluoroacetate

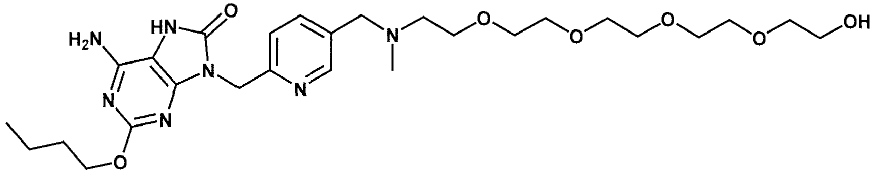

### Step 1

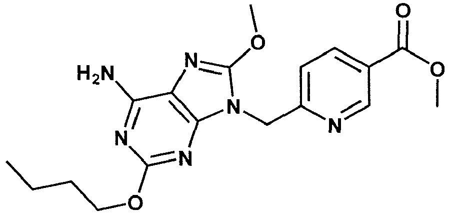

To a solution of 2-butoxy-8-methoxy-9H-purine-6-amine·trifluoroacetate (1.20 g) which is a known compound in DMF (15mL) were added methyl 6-(chloromethyl)pyridine-3-carboxylate (633 mg) and potassium carbonate (1.41 g) , and the solution was stirred at room temperature. Water was added to the reaction solution, and the mixture was extracted with chloroform, dried over magnesium sulfate, filtered, and concentrated under reduced pressure. The obtained crude product was purified by silica gel chromatography (mobile phase: chloroform/methanol) to give methyl 6-[(6-amino-2-butoxy-8-methoxy-9H-purin-9-yl)methyl]pyridine-3-carboxylate (412 mg).
m/z = 387 [M+H]⁺, Rt = 0.732 (LCMS Condition B)

### Step 2

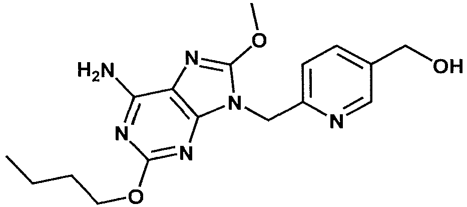

To a solution of the compound (300 mg) prepared in Example 1 (Step 1) in THF (30 mL) was added lithium aluminium hydride (32.4 mg) at 0°C, and the mixture was stirred. The reaction mixture was treated with water and 2 mol/L aqueous sodium hydroxide, and the precipitate was filtered off with Celite. The filtrate was concentrated, and the obtained residue was purified by silica gel chromatography (mobile phase: chloroform/methanol) to give {6-[(6-amino-2-butoxy-8-methoxy-9H-purin-9-yl)methyl]pyridin-3-yl}methanol (200 mg) .
m/z = 359 [M+H]⁺, Rt = 0.611 (LCMS Condition B)

### Step 3

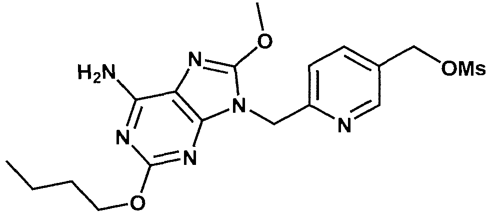

To a solution of the compound (100 mg) prepared in Example 1 (Step 2) in NMP (2 mL) were added triethylamine (0.35 mL) and methanesulfonyl chloride (0.65 mL) at 0°C, and the mixture was stirred for 3 hours. Methanesulfonyl chloride (0.01 mL) was added to the reaction mixture, and the mixture was stirred for 30 minutes. To the reaction solution was added ice water, and the mixture was extracted with ethyl acetate. The organic layer was dried over magnesium sulfate, filtered, and concentrated under reduced pressure to give {6-[(6-amino-2-butoxy-8-methoxy-9H-purin-9-yl)methyl]pyridin-3-yl}methyl methanesulfonate as a crude product in NMP. The obtained product was used in the next reaction without further purification.
m/z = 437 [M+H]⁺, Rt = 0.715 (LCMS Condition B)

### Step 4

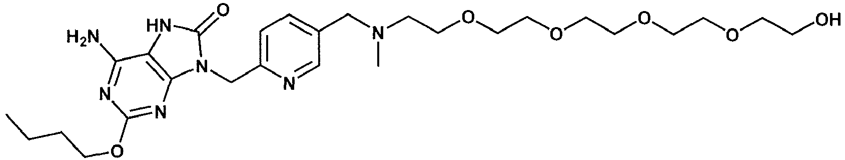

To a solution of the compound prepared in Example 1 (Step 3) in NMP were added the compound (102 mg) of Reference example 10, potassium carbonate (0.039 g), and potassium iodide (0.046 g), and the mixture was stirred at room temperature for 4 hours. The reaction solution was diluted with methanol (3 mL), and 4 mol/L hydrochloric acid in ethyl acetate (2 mL) was added thereto. The mixture was stirred at room temperature for one hour. The reaction solution was concentrated and the residue was purified by reversed-phase HPLC to give 6-amino-2-butoxy-9-{[5-(16-hydroxy-2-methyl-5, 8, 11, 14-tetraoxa-2-azahexadecan-1-yl)pyridin-2-yl]methyl}-7,9-dihydro-8H-purin-8-one·trifluoroacetate (0.09 g).
m/z = 290 [M+2H]⁺², Rt = 0.632 (LCMS Condition B)
¹H-NMR (DMSO-d6): δ 10.03 (s, 1H), 9.66 (s, 1H), 8.58 (d, J = 2.0 Hz, 1H), 7.90 (dd, J = 8.0 Hz, 2.4 Hz, 1H), 7.32 (d, J = 8.0 Hz, 1H), 6.50 (s, 2H), 5.00 (s, 2H), 4.42 (d, J = 13.2 Hz, 1H), 4.28 (d = 13.2 Hz, 1H), 4.06 (t, J = 6.8 Hz, 2H), 3.75 (t, J = 5.2 Hz, 2H), 3.70-3.50 (m, 16H), 3.38 (t, J = 6.8 Hz, 2H), 2.72 (d, J = 4.4 Hz, 3H), 1.60-1.52 (m, 2H), 1.37-1.27 (m, 2H), 0.86 (t, J = 7.6 Hz, 3H)

### Example 2

### Preparation of 6-amino-2-butoxy-9-{[6-(16-hydroxy-2-methyl-5, 8, 11, 14-tetraoxa-2-azahexadecan-1-yl)pyridin-3-yl]methyl}-7,9-dihydro-8H-purin-8-one·trifluoroacetate

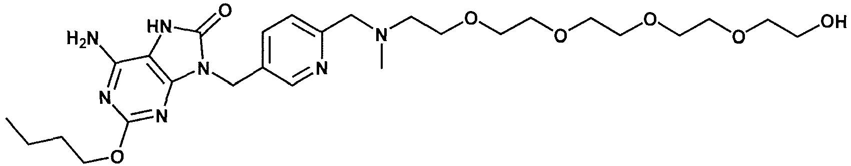

### Step 1

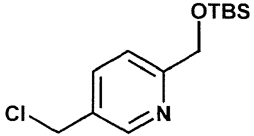

Using [5-(chloromethyl)pyridin-2-yl]methanol which is a known compound as a starting material, 2-{[(tert-butyldimethylsilyl)oxy]methyl}-5-(chloromethyl)pyridine was prepared according to similar reaction and treatment to the procedure of Step 2 in Reference example 10.
m/z = 272 [M+2H]⁺², Rt = 1.23 (LCMS Condition B)

### Step 2

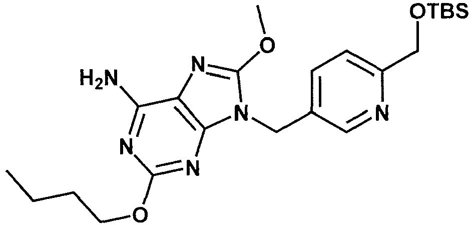

2-Butoxy-9-{[6-({[tert-butyl(dimethyl)silyl]oxy}methyl)pyridin-3-yl]methyl}-8-methoxy-9H-purine-6-amine was prepared according to similar reaction and treatment to the procedure of Step 1 in Example 1.
m/z = 237 [M+2H]⁺², Rt = 1.33 (LCMS Condition A)

### Step 3

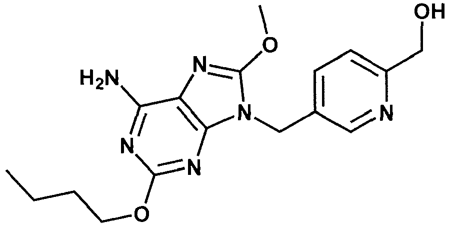

To a solution of the compound (994 mg) prepared in Example 2 (Step 2) in THF (20 mL) was added TBAF (1 M THF solution, 4 mL) at room temperature, and the solution was stirred for 6 hours. The reaction solution was concentrated, and purified by silica gel column chromatography (mobile phase: chloroform/methanol) to give {5-[(6-amino-2-butoxy-8-methoxy-7,8-dihydro-9H-purin-9-yl)methyl]pyridin-2-yl}methanol (710 mg).
m/z = 359 [M+H]⁺, Rt = 0.571 (LCMS Condition B)

### Step 4

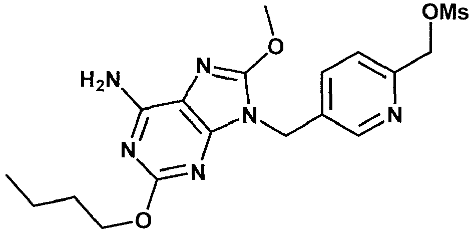

Using the compound prepared in Example 2 (Step 3) as a starting compound, {5-[(6-amino-2-butoxy-8-methoxy-9H-purin-9-yl)methyl]pyridin-2-yl}methyl methanesulfonate was prepared according to similar reaction to the procedure of Step 3 in Example 1. The obtained compound was used in the next reaction without further purification.

### Step 5

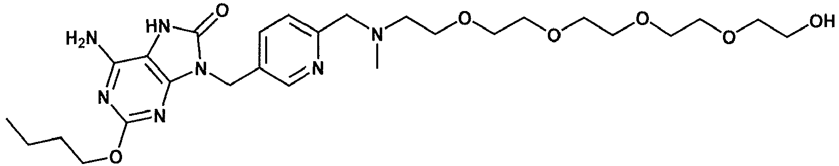

Using the compound prepared in Example 2 (Step 4) as a starting compound, 6-amino-2-butoxy-9-{[6-(16-hydroxy-2-methyl-5, 8, 11, 14-tetraoxa-2-azahexadecan-1-yl)pyridin-3-yl]methyl}-7,9-dihydro-8H-purin-8-one·trifluoroacetate was prepared according to similar reaction to the procedure of Step 4 in Example 1.
m/z = 290 [M+H]⁺, Rt = 0.611 (LCMS Condition B)
¹H-NMR (CD₃OD-d4): δ 8.75 (d, J = 1.8 Hz, 1H), 7.93 (dd, J = 2.4, 7.9 Hz, 1H), 7.48 (d, J = 7.9 Hz, 1H), 5.07 (s, 2H), 4.54 (s, 2H), 4.29 (t, J = 4.9 Hz, 2H), 3.85 (t, J = 4.9 Hz, 2H), 3.66-3.56 (m, 14H), 3.52-3.47 (m, 2H), 3.45-3.40 (m, 2H), 2.96 (s, 3H), 1.73 (dt, J = 15.6, 5.9 Hz, 2H), 1.48 (dt, J = 14.9, 7.9 Hz, 2H), 0.98 (t, J = 7.2 Hz, 3H)

### Reference examples 12 - 14

The compounds in Table 3 below were prepared from each corresponding starting compound according to similar reaction and treatment to the procedure of Step 4 in Example 1.

| Table 3 | | |
|---|---|---|
| Reference example No. | Structure | LCMS (LCMS Condition B) |
| 12 | 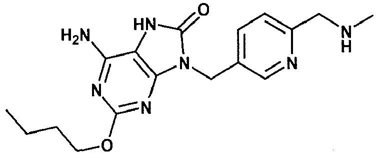 | 179[M+2H]⁺², Rt = 0.56 |
| 13 | 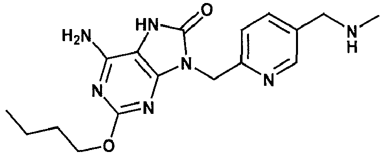 | 179[M+2H]⁺², Rt = 0.56 |
| 14 | 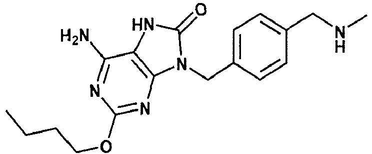 | 179[M+2H]⁺², Rt = 0.54 |

### Examples 3 - 4

The compounds in Table 4 below were prepared from each known compound according to similar reaction and treatment to the procedure of Example 1.

| Table 4 | | |
|---|---|---|
| Example No. | Structure | LCMS (LCMS Condition B) |
| 3 | 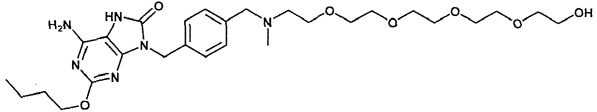 | m/z = 289 [M+2H]⁺², Rt = 0.613 |
| 4 | 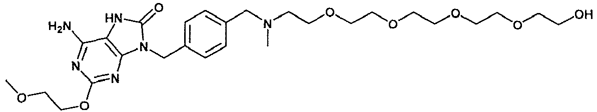 | m/z = 579 [M+H] ⁺, Rt = 0.458 |

### Example 5

The compound in Table 5 below was prepared from the corresponding starting compound according to similar reaction and treatment to the procedure described in Tetrahedron Letters Volume 56, Issue 2, 8 January 2015, Pages 458-460.

| Table 5 | | |
|---|---|---|
| Example No. | Structure | LCMS (LCMS Condition B) |
| 5 | 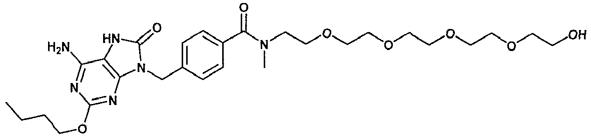 | m/z = 592 [M+2H] ⁺², Rt = 0.715 |

### Reference example 15

### N,2,2,3,3-Pentamethyl-4,7,10,13,16,19,22,25,28,31-decaoxa-3-silatritriacontane-33-amine

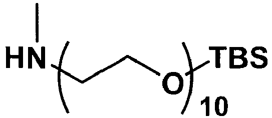

The title compound was prepared according to the process of Reference example 10.
m/z = 587 [M+H]⁺, Rt = 0.865 (LCMS Condition B)

### Reference example 16

### N,2,2,3,3-Pentamethyl-4,7,10,13,16,19,22,25,28,31,34,37,40,43,46,49,52,55,58,61,6 4, 67, 70, 73-tetracosaoxa-3-silapentaheptacontane-75-amine

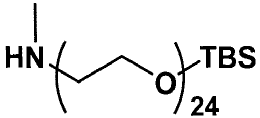

The title compound was prepared according to the process of Reference example 10.
m/z = 402 [M+3H]⁺³, Rt = 0.946 (LCMS Condition B)

### Reference example 17

### N,2,2,3,3-Pentamethyl-4,7,10,13,16,19,22,25,28,31,34,37,40,43,46,49,52,55,58,61,6 4,67,70,73,76,79,82,85,88,91,94,97,100,103,106,109-hexatriacontaoxa-3-silahendecahectane-111-amine

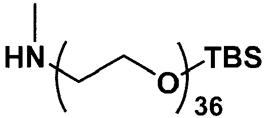

The title compound was prepared according to the process of Reference example 10.
m/z = 866 [M+2H]⁺², Rt = 0.948 (LCMS Condition B)

### Example 6

### 6-Amino-2-butoxy-9-({6-[13-hydroxy-2-(2-{2-[2-(2-hydroxyethoxy)ethoxy]ethoxy}ethyl)-5,8,11-trioxa-2-azatridecan-1-yl]pyridin-3-yl]methyl)-7,9-dihydro-8H-purin-8-one

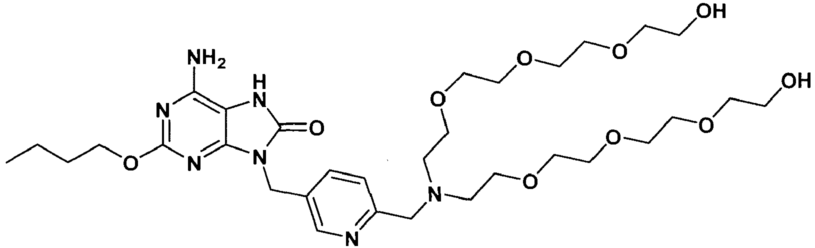

The title compound was prepared from the known compound according to similar reaction and treatment to the procedure of Example 2.
m/z = 697 [M+H]⁺, Rt = 0.601 (LCMS Condition B)
¹H-NMR (DMSO-d6): δ 10.45 (1H, s), 8.64 (1H, d, J = 2.3 Hz), 7.81 (1H, dd, J = 8.0, 2.1 Hz), 7.54 (1H, d, J = 8.2 Hz), 4.94 (2H, s), 4.57 (2H, s), 4.16 (2H, t, J = 6.6 Hz), 3.78 (4H, t, J = 4.8 Hz), 3.55-3.35 (32H, m), 1.66-1.59 (2H, m), 1.37 (2H, m), 0.94-0.88 (3H, t, J = 7.3 Hz).

### Example 7

### 6-Amino-2-butoxy-9-{[6-(31-hydroxy-2-methyl-5,8,11,14,17,20,23,26,29-nonaoxa-2-azahentriacontan-1-yl)pyridin-3-yl]methyl}-7,9-dihydro-8H-purin-8-one

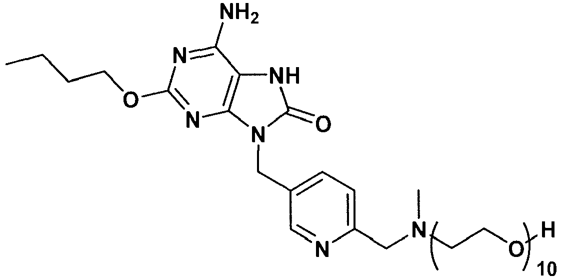

The title compound was prepared from Reference example 15 according to similar reaction and treatment to the procedure of Example 2.
m/z = 799 [M+H]⁺, Rt = 0.634 (LCMS Condition B)
¹H-NMR (CDCl₃) : δ 9.27 (1H, s), 8.66 (1H, s), 7.76 (1H, d, J = 8.2 Hz), 7.44 (1H, d, J = 8.2 Hz), 5.71 (2H, s), 4.97 (2H, s), 4.25 (2H, t, J = 6.6 Hz), 3.74-3.45 (40H, m), 2.58 (2H, t, J = 5.5 Hz), 2.32 (3H, s), 1.73 (3H, m), 1.46 (2H, m), 0.94 (3H, t, J = 7.3 Hz).

### Example 8

### 6-Amino-2-butoxy-9-{[6-(73-hydroxy-2-methyl-5,8,11,14,17,20,23,26,29,32,35,38,41,44,47,50,53,56,59,62,6 5, 68, 71-tricosaoxa-2-azatriheptacontan-1-yl)pyridin-3-yl]methyl}-7,9-dihydro-8H-purin-8-one

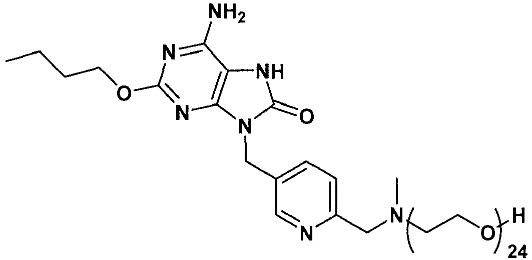

The title compound was prepared from Reference example 16 according to similar reaction and treatment to the procedure of Example 2.
m/z = 708 [M+2H]⁺², Rt = 0.701 (LCMS Condition B)
¹H-NMR (CDCl₃): δ 9.26 (1H, s), 8.64 (1H, s), 7.77 (1H, d, J = 8.2 Hz), 7.40 (1H, d, J = 7.9 Hz), 5.63 (2H, s), 4.98 (2H, s), 4.23 (2H, t, J = 6.7 Hz), 3.77-3.45 (97H, m), 2.67 (2H, s), 2.35 (3H, s), 1.73 (2H, m), 1.46 (2H, m), 0.94 (3H, t, J = 7.3 Hz).

### Example 9

### 6-Amino-2-butoxy-9-{[6-(109-hydroxy-2-methyl-5,8,11,14,17,20,23,26,29,32,35,38,41,44,47,50,53,56,59,62,6 5,68,71,74,77,80,83,86,89,92,95,98,101,104,107-pentatriacontaoxa-2-azanonahectan-1-yl)pyridin-3-yl]methyl}-7,9-dihydro-8H-purin-8-one

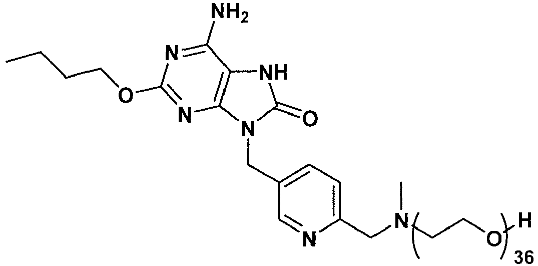

The title compound was prepared from Reference example 17 according to similar reaction and treatment to the procedure of Example 2.
m/z = 649 [M+3H]⁺³, Rt = 0.732 (LCMS Condition B)
¹H-NMR (CDCl₃) : δ 9.19 (1H, s), 8.65 (1H, d, J = 2.4 Hz), 7.76 (1H, d, J = 8.2 Hz), 7.41 (1H, d, J = 7.9 Hz), 5.57 (2H, s), 4.97 (2H, s), 4.23 (2H, t, J = 6.4 Hz), 3.79-3.46 (144H, m), 2.58 (2H, t, J = 5.5 Hz), 2.30 (3H, s), 1.76-1.69 (2H, m), 1.46 (2H, m), 0.94 (3H, t, J = 7.3 Hz).

### Test 1

### Human TLR7 reporter gene assay

TLR7/NF-κB/SEAPorter^{™} HEK293 cell line (Imgenex Corporation) is a stably co-transfected cell line which expresses full-length human TLR7 and secretory alkaline phosphatase (SEAP) reporter gene under the transcriptional regulation of an NF-κB response element. The TLR7 expression of the cell line has been already tested by flow cytometry. Transfectants with stable expression were selected using the antibiotic blasticidin and Geneticin. TLR signaling leads to the translocation of NF-κB and the activation of the promoter results in expression of the SEAP gene. TLR7-specific activation was assessed by determining the level of SEAP produced following overnight incubation of the cells at 37°C with each compound prepared in Examples and Reference examples in the presence of 0.1 % (v/v) dimethylsulfoxide (DMSO). The human TLR7 activity for the present compound was assessed by human TLR7 reporter gene assay, and the results are shown in Tables 6 and 7 as the compound concentration which produced half of the maximal level of SEAP induction (EC₅₀) .

| Table 6 | |
|---|---|
| Example No. | EC₅₀ (nM) |
| 1 | 150 |
| 2 | 774 |
| 3 | 24 |
| 4 | 5298 |

| Table 7 | |
|---|---|
| Reference example No. | EC₅₀ (nM) |
| 12 | 578 |
| 13 | 82 |
| 14 | 12 |

### Test 2

### Mouse TLR7 reporter gene assay

HEK-Blue^{™} mTLR7 cell line (Invivogen) is a stably co-transfected cell line which expresses full-length mouse TLR7 and secretory SEAP reporter gene under the transcriptional regulation of an NF-κB response element. The TLR7 expression of the cell line has been already tested by RT-PCR. Transfectants with stable expression were selected using the antibiotic blasticidin and Zeocin. TLR signaling leads to the translocation of NF-κB and the activation of the promoter results in expression of the SEAP gene. TLR7-specific activation was assessed by determining the level of SEAP produced following overnight incubation of the cells at 37°C with each compound prepared in Examples and Reference examples in the presence of 0.1 % (v/v) DMSO. The mouse TLR7 activity for the present compound was assessed by mouse TLR7 reporter gene assay, and the results are shown in Tables 8 and 9 as the compound concentration which produced half of the maximal level of SEAP induction (EC₅₀).

| Table 8 | |
|---|---|
| Example No. | EC₅₀ (nM) |
| 1 | 1449 |
| 2 | 510 |
| 3 | 42 |

| Table 9 | |
|---|---|
| Reference example No. | EC₅₀ (nM) |
| 12 | 251 |
| 13 | 279 |
| 14 | 23 |

The results in Tests 1 and 2 suggest that the Example compounds and the Reference example compounds of the present invention can both act as human and mouse TLR7 agonists.

### Test 3

### Evaluation of action for activating immune cell with human peripheral blood mononuclear cell

Cryopreserved peripheral blood mononuclear cells (PBMCs) of adult (C.T.L) were reconstituted, suspended in a culture medium which comprises AIM V medium (Life Technologies) supplemented with 10 % human serum (biowest) and 1 % MEM Non-Essential Amino Acids Solution (100×) (Life Technologies), and seeded on a U-bottom 96 well plate at 4 × 10⁵ cells/well. The compound of the present invention was added in the presence of 0.1 % (v/v) DMSO to the wells at a final concentration of 200 nmol/L. The plate was incubated at 37°C under an atmosphere of 5 % CO₂. One day after the incubation, the culture supernatant was collected. The concentration of cytokine and chemokine (GM-CSF, IFNα2, IFNγ, IL-12p40, IL-1β, IL-6, IP-10, and TNFα) which were included in each culture supernatant was measured with Milliplex Human Cytokine/Chemokine Magnetic Bead Panel kit (Millipore) and Luminex system (Luminex) in accordance with the manufacturer's protocol.

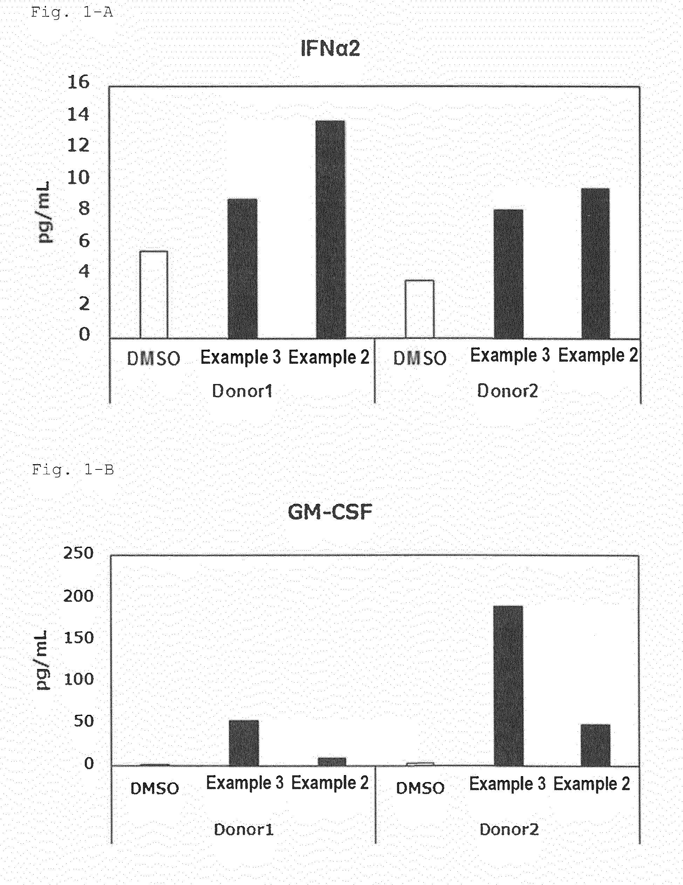

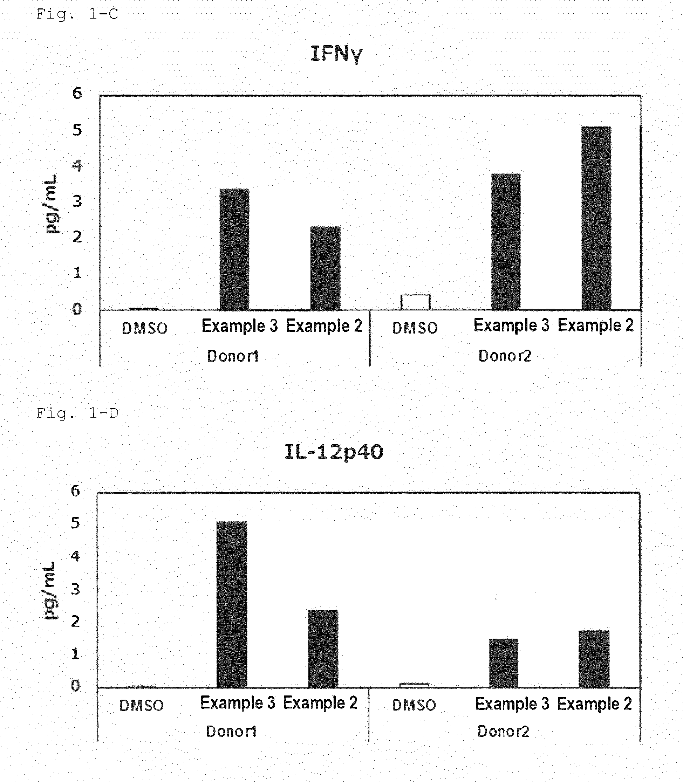

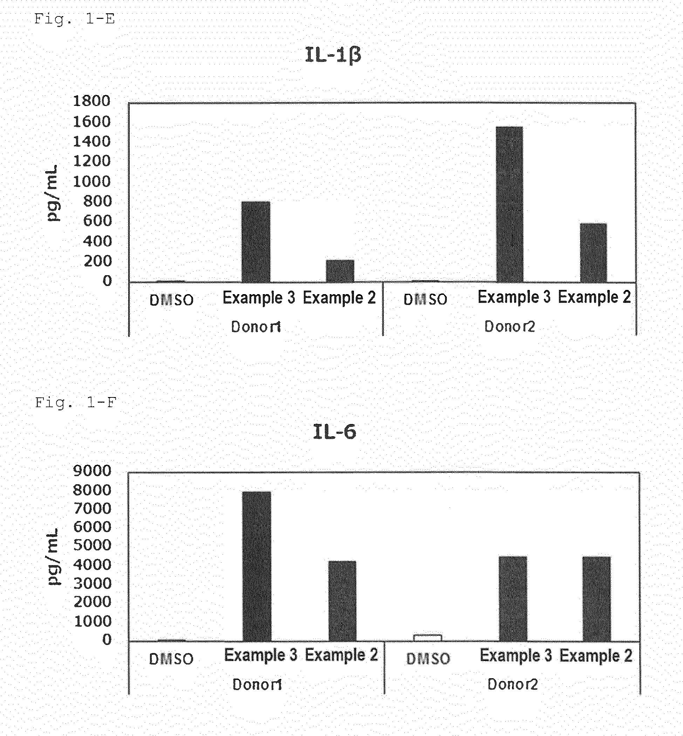

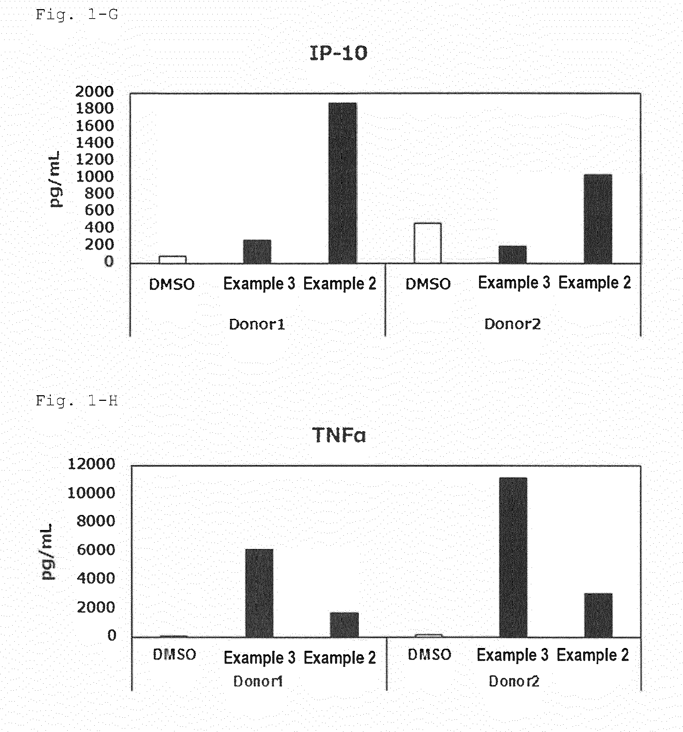

The results of the test wherein PBMCs derived from two different donors were used were shown in Fig. 1-A to Fig. 1-I. The white bar indicates the mean number of the concentration of cytokine and chemokine in the culture supernatant from three wells to which only DMSO was added, and the black bar indicates the mean number of the concentration of cytokine and chemokine in the culture supernatant from three wells to which the present compound was added. The compound prepared in Example 2 or Example 3 made the concentration of cytokine and chemokine higher, compared with the case of only DMSO. These results show that the present compound has an action for activating human immune cell.

### Test 4

### Evaluation of action for activating mouse bone marrow-derived dendritic cell

Myeloid cells were collected from the femur and tibia of HLA-A^{∗}02:01 transgenic mouse (C57BL/6CrHLA-A2.1DR1), and then suspended in RPMI 1640 medium with 10 % fetal bovine serum (FBS) and 10 ng/mL of GM-CSF. The dendritic cells in the myeloid cells were incubated at 37°C under an atmosphere of 5 % CO₂ for induction of differentiation. Seven days after the incubation, the cells were collected. After observing the expression of CD11c on the cell surface by flow cytometry, the cells were cryopreserved. Cryopreserved mouse bone marrow-derived dendritic cells were reconstituted, suspended in RPMI 1640 medium with 10 % FBS, and seeded on a U-bottom 96 well plate at 5 × 10⁵ cells/well. The compound prepared in Example 3 was added in the presence of 0.1 % (v/v) DMSO to the wells at a final concentration of 200 nmol/L. The plate was incubated at 37°C under an atmosphere of 5 % CO₂. One day after the incubation, the culture supernatant was collected. The concentration of cytokine and chemokine (IL-1β, IL-6, IL-12p40, IL-12p70, IP-10, MIP-1α, MIP-1β, Rantes, and TNFα) which were included in each culture supernatant was measured with Milliplex Mouse Cytokine/Chemokine Magnetic Bead Panel kit (Millipore) and Luminex system in accordance with the manufacturer's protocol.

The results are shown in Fig. 2-A to Fig. 2-I. The white bar indicates the mean number of the concentration of cytokine and chemokine in the culture supernatant from three wells to which only DMSO was added, and the black bar indicates the mean number of the concentration of cytokine and chemokine in the culture supernatant from three wells to which the compound prepared in Example 3 was added. The compound prepared in Example 3 made the yield of cytokine and chemokine higher, compared with the case of only DMSO. These results show that the present compound has an action for activating dendritic cell.

### Test 5

### Evaluation of action for activating immune cell with mouse splenocyte

Splenocyte was derived from the spleen of a HLA-A^{∗}02:01 transgenic mouse, and cryopreserved. Cryopreserved mouse splenocyte was reconstituted, suspended in RPMI 1640 medium with 10 % FBS, and seeded on a U-bottom 96 well plate at 4 × 10⁵ cells/well. The compound prepared in Example 3 was added in the presence of 0.1 % (v/v) DMSO to the wells at a final concentration of 200 nmol/L. The plate was incubated at 37°C under an atmosphere of 5 % CO₂. One day after the incubation, the culture supernatant was collected. The concentration of cytokine and chemokine (GM-CSF, IFNγ, IL-6, IP-10, MCP-1, MIP-1α, MIP-1β, Rantes, and TNFα) which were included in each culture supernatant was measured with Milliplex Mouse Cytokine/Chemokine Magnetic Bead Panel kit and Luminex system in accordance with the manufacturer's protocol.

The results are shown in Fig. 3-A to Fig. 3-I. The white bar indicates the mean number of the concentration of cytokine and chemokine in the culture supernatant from three wells to which only DMSO was added, and the black bar indicates the mean number of the concentration of cytokine and chemokine in the culture supernatant from three wells to which the compound prepared in Example 3 was added. The compound prepared in Example 3 made the yield of cytokine and chemokine higher, compared with the case of only DMSO. These results show that the present compound has an action for activating mouse immune cell.

### Test 6

### Evaluation of In vivo adjuvant activity in HLA-A^{∗}02:01 transgenic mouse

The *in vivo* adjuvant activity of the compound of Example 3 and the compound of Reference example 14 was evaluated in the following procedure. To a cocktail vaccine comprising Compound of formula 4 prepared in Reference example 9 and Peptide SEQ ID No. 3 prepared in Reference example 3 with a preliminarily-emulsified composition (hereinafter, referred to as "vaccine a") was added the compound prepared in Example 3 or the compound prepared in Reference example 14 respectively to prepare each vaccine. The vaccine was administered to a HLA-A^{∗}02:01 transgenic mouse, and the adjuvant activity of each vaccine was evaluated by the method of testing antigen-specific cytotoxic T-lymphocyte (CTL) induction. The RMFPNAPYL (SEQ ID No. 1) in Compound of formula 4 and VLDFAPPGA (SEQ ID No. 5) in Peptide SEQ ID No.3 correspond to antigen peptides derived from an HLA-A^{∗}02:01-restricted WT1 protein.

HLA-A^{∗}02:01 transgenic mouse (C57BL/6CrHLA-A2.1DR1) lacks mouse MHC, and instead expresses a chimeric HLA of a human MHC (HLA-A^{∗}02:01) and a mouse MHC (H-2D^{b}), and HLA-DRB1^{∗}01:01. The mouse can evaluate the pharmacological activity of immunotherapeutic agents including a peptide which induces CTLs in HLA-A^{∗}02:01-positive human (Eur J Immunol. 2004; 34: 3060-9). In addition, the mouse can also evaluate the induction of a helper peptide which can bind to a human HLA-DRB1^{∗}01:01 to induce a helper T-cell, and the enhancing effect for inducing CTLs.

The character for inducing CTLs specific to the antigen peptide (SEQ ID No. 1 or SEQ ID No. 5) by vaccine a was evaluated by measuring whether IFNγ can be produced upon stimulation of splenocytes from the mouse with the peptide. In addition, the character for exerting in vivo adjuvant activity by the compound in Example 3 or the compound in Reference example 14 was evaluated by comparing the number of CTLs induced by vaccine a and the number of CTLs induced by the vaccine prepared by adding the compound prepared in Example 3 or the compound in Reference example 14 to vaccine a, and checking presence or absence of the increase on the number.

Specifically, the *in vivo* adjuvant activity was evaluated as follows.

0.312 g of sodium dihydrogen phosphate dihydrate was dissolved in 80 g of water for injection. 14.0 g of ethyl oleate, 14.0 g of octyldodecyl myristate, 2.0 g of sorbitan monooleate, 2.8 g of glyceryl monooleate, 0.4 g of polyoxyethylene hydrogenated castor oil 20, and 0.4 g of glycerin were mixed. 2.354 mL of the mixture (corresponding to 2.077 g) was put into a test tube, and 0.396 mL of the aqueous sodium dihydrogen phosphate (corresponding to 0.396 g) was gradually added into the test tube which was being stirred with a mixer (ULTRA-TURRAX T10, IKA, or Touch Mixer MT-51, Yamato Scientific) to emulsify the mixture in the test tube. The obtained emulsion was referred to as preliminary emulsified composition. The amount of the preparation was adjusted as appropriate.

Compound of formula 4 and Peptide SEQ ID No. 3 were dissolved in DMSO, and then diluted with water for injection to concentrations of Compound of formula 4 of 3 mg/ml and Peptide of SEQ ID No. 3 of 2.25 mg/ml. The diluted peptide solution was mixed and emulsified with an equal volume of the above preliminary emulsified composition to prepare vaccine a. Vaccine a was injected to mice intradermally at the tail base area in an amount for administering 300 µg of Compound of formula 4 per body and 225 µg of Peptide SEQ ID No. 3 per body. Or, in the step of preparing vaccine a, the compound prepared in Example 3 was added to the diluted peptide solution to prepare a vaccine, and the prepared vaccine was injected to mice intradermally at the tail base area in an amount for administering 300 µg of Compound of formula 4 per body, 225 µg of Peptide SEQ ID No. 3 per body, and 32.5 ng of the compound prepared in Example 3 per body. Or, in the step of preparing vaccine a, the compound prepared in Reference example 14 was added to the diluted peptide solution to prepare a vaccine, and the prepared vaccine was injected to mice intradermally at the tail base area in an amount for administering 300 µg of Compound of formula 4 per body, 225 µg of Peptide SEQ ID No. 3 per body, and 17.5 ng of the compound prepared in Reference example 14 per body. One week later, the mice were sacrificed with CO₂ gas. Splenocytes were harvested from spleens removed from the mice. For detecting IFNγ-producing splenocytes, an IFNγ ELISPOT assay kit (BD) was used. In particular, an ELISPOT plate was treated with an anti-mouse-IFNy antibody on the day before preparation of the splenocyte samples. On the next day, the plate was blocked by treatment with an RPMI 1640 medium with 10 % FBS. To the blocked ELISPOT plate, the prepared splenocyte samples were added at 1.25 × 10⁵ cells/well. Peptide (SEQ ID No. 1 or SEQ ID No. 5) was added in the presence of 0.1 % (v/v) DMSO to the splenocyte-containing wells at a final concentration of 10 µg/ml. The peptide-added splenocyte was incubated overnight at 37°C under an atmosphere of 5 % CO₂ to re-stimulate the peptide *in vitro.* Then, after removal of the supernatant from the wells, the ELISPOT plate was subjected to treatment for cell staining in accordance with the manufacturer's protocol. Stained spots were counted on ImmunoSpot Analyzer (C.T.L.).

Fig 4 and Fig. 5 show results from the IFNγ ELISPOT assay using the HLA-A^{∗}02:01 transgenic mouse. The scale on the vertical axis of the graphs of Fig. 4 and Fig. 5 indicates the mean number of cells which produced IFNγ in response to the stimulation with the added cells, from each three mice per group. The vaccine administered to the mice is indicated on the horizontal axis. The black bars and the white bars in Fig. 4 show the results that the splenocyte from the HLA-A^{∗}02:01 transgenic mouse was incubated in the presence or absence of Peptide SEQ ID No. 1, respectively. Thus, the difference in the cell count between the black bar and the white bar shows the count of IFNγ-producing cells specific for Peptide SEQ ID No. 1 which was induced in the mouse's body by the administration of the vaccine, i.e., CTL count. The counts of the white bars in Fig. 4 were almost-imperceptible. It suggests that the splenocyte of the mouse reacted very little in the absence of the desired peptide. The results of the present test showed that vaccine a induced CTLs responsive to Peptide SEQ ID No. 1 in the HLA-A^{∗}02:01 transgenic mouse. And, the CTL counts increased by adding the compound prepared in Example 3 or the compound prepared in Reference example 14 to vaccine a. In addition, the increase of the CTL counts was more in the case of adding the compound prepared in Example 3 to cocktail vaccine b, compared with the case of the compound prepared in Reference example 14.

In addition, the black bars and the white bars in Fig. 5 show the results that the splenocyte from the HLA-A^{∗}02:01 transgenic mouse was incubated in the presence or absence of Peptide SEQ ID No. 5, respectively. Thus, the difference in the cell count between the black bar and the white bar shows the count of IFNγ-producing cells specific for Peptide SEQ ID No. 5 which was induced in the mouse's body by the administration of the vaccine, i.e., CTL count. The counts of the white bars in Fig. 5 were almost-imperceptible. It suggests that the splenocyte of the mouse reacted very little in the absence of the desired peptide. The results of the present test showed that vaccine a induced CTLs responsive to Peptide SEQ ID No. 5 in the HLA-A^{∗}02:01 transgenic mouse. And, the CTL counts increased by adding the compound prepared in Example 3 or the compound prepared in Reference example 14 to vaccine a. In addition, the increase of the CTL counts was more in the case of adding the compound prepared in Example 3 to cocktail vaccine b, compared with the case of the compound prepared in Reference example 14.

The above results show that the induced CTL count increases by adding the compound prepared in Example 3 to the vaccine, and strongly suggest that the compound prepared in Example 3 has *in vivo* adjuvant activity. In addition, the results also show that the effect of increasing CTLs with the compound prepared in Example 3 is higher, compared with the case of the compound prepared in Reference example 14 which has no PEG structure.

### Test 7

### Evaluation of in vivo adjuvant activity in HLA-A^{∗}02:01 transgenic mouse

The *in vivo* adjuvant activity of the compound prepared in Example 2 and the compound prepared in Reference example 12 was evaluated in the following procedure. To vaccine a was added the compound prepared in Example 2 or the compound prepared in Reference example 12 to prepare each vaccine, and the vaccine was administered to a HLA-A^{∗}02:01 transgenic mouse, and the adjuvant activity of each vaccine was evaluated by the method of testing antigen-specific CTL induction.

Specifically, vaccine a prepared like Test 6 was injected to mice intradermally at the tail base area in an amount for administering 300 µg of Compound of formula 4 per body and 225 µg of Peptide SEQ ID NO. 3 per body. Or, in the step of preparing vaccine a, the compound prepared in Example 2 was added to the diluted peptide solution to prepare a vaccine, and the prepared vaccine was injected to mice intradermally at the tail base area in an amount for administering 300 µg of Compound of formula 4 per body, 225 µg of Peptide SEQ ID No. 3 per body, and 325 ng of the compound prepared in Example 2 per body. Or, in the step of preparing vaccine a, the compound prepared in Reference example 12 was added to the diluted peptide solution to prepare a vaccine, and the prepared vaccine was injected to mice intradermally at the tail base area in an amount for administering 300 µg of Compound of formula 4 per body, 225 µg of Peptide SEQ ID No. 3 per body, and 225 ng of the compound prepared in Reference example 12 per body. The administrations were done twice, which had one week interval. One week after the final administration, the mice were sacrificed with CO₂ gas. Splenocytes were harvested from spleens removed from the mice. In particular, an ELISPOT plate was treated with an anti-mouse-IFNy antibody on the day before preparation of the splenocyte samples. On the next day, the plate was blocked by treatment with an RPMI 1640 medium with 10 % FBS. To the blocked ELISPOT plate, the prepared splenocyte samples were added at 6.25 × 10⁴ cells/well. Peptide (SEQ ID No. 5) was added in the presence of 0.1 % (v/v) DMSO to the splenocyte-containing wells at a final concentration of 10 µg/ml. The peptide-added splenocyte was incubated overnight at 37°C under an atmosphere of 5 % CO₂ to re-stimulate the peptide *in vitro.* Then, after removal of the supernatant from the wells, the ELISPOT plate was subjected to treatment for cell staining in accordance with the manufacturer's protocol. Stained spots were counted on ImmunoSpot Analyzer.

The result is shown in Fig. 6. In Fig. 6, the vertical axis indicates the mean number of cells which produced IFNγ in response to the stimulation with the added cells, from each three mice per group. The vaccine administered to the mice is indicated on the horizontal axis. The black bars and the white bars in Fig. 6 show the results that the splenocyte from the mouse was incubated in the presence or absence of Peptide SEQ ID No. 5, respectively. The results of the present test showed that the CTL counts responsive to Peptide SEQ ID No. 5 were more in the case of adding the compound prepared in Example 2 or the compound prepared in Reference example 12 to vaccine a, compared with the case of adding no compound. In addition, the increase of the CTL counts was more in the case of adding the compound prepared in Example 2 to cocktail vaccine b, compared with the case of the compound prepared in Reference example 12.

The above results show that the induced CTL count increases by adding the compound prepared in Example 2 to the vaccine, and strongly suggest that the compound prepared in Example 2 has *in vivo* adjuvant activity. In addition, the results also show that the effect of increasing CTLs with the compound prepared in Example 2 is higher, compared with the case of the compound prepared in Reference example 12 which has no PEG structure.

### Test 8

### Evaluation of in vivo adjuvant activity in HLA-A^{∗}02:01 transgenic mouse

The *in vivo* adjuvant activity of the compounds prepared in Examples 2 and 3 was evaluated in the following procedure. To a cocktail vaccine comprising Compound of formula 4 prepared in Reference example 9 and Peptide SEQ ID No. 3 prepared in Reference example 3 with Montanide ISA51 VG (hereinafter, referred to as " vaccine b") was added the compound prepared in Example 2 or 3 to prepare each vaccine. The vaccine was administered to a HLA-A^{∗}02:01 transgenic mouse, and the adjuvant activity of each vaccine was evaluated by the method of testing antigen-specific CTL induction.

Specifically, Compound of formula 4 and Peptide SEQ ID No. 3 were dissolved in DMSO, and then diluted with water for injection to concentrations of Compound of formula 4 of 3 mg/mL and Peptide SEQ ID No. 3 of 2.25 mg/mL. The diluted peptide solution was mixed and emulsified with an equal volume of Montanide ISA 51 VG (Seppic) to prepare vaccine b. Cocktail vaccine b was injected to mice intradermally at the tail base area in an amount for administering 300 µg of Compound of formula 4 per body and 225 µg of Peptide SEQ ID No. 3 per body. Or, in the step of preparing vaccine b, the compound prepared in Example 3 was added to the diluted peptide solution to prepare a vaccine, and the prepared vaccine was injected to mice intradermally at the tail base area in an amount for administering 300 µg of Compound of formula 4 per body, 225 µg of Peptide SEQ ID No. 3 per body, and 32.5 ng or 325 ng of the compound prepared in Example 3 per body. Or, in the step of preparing vaccine b, the compound prepared in Example 2 was added to the diluted peptide solution to prepare a vaccine, and the prepared vaccine was injected to mice intradermally at the tail base area in an amount for administering 300 µg of Compound of formula 4 per body, 225 µg of Peptide SEQ ID No. 3 per body, and 32.5 ng or 325 ng of the compound prepared in Example 2 per body. The administrations were done twice, which had one week interval. One week after the final administration, the mice were sacrificed with CO₂ gas. Splenocytes were harvested from spleens removed from the mice. Like Test 6, Peptide (SEQ ID No. 1 or SEQ ID No. 5) was added to the splenocyte-containing ELISPOT plate, and the plate was incubated overnight at 37°C under an atmosphere of 5 % CO₂ to re-stimulate the peptide in *vitro.* Then, after removal of the supernatant from the wells, stained spots on the ELISPOT plate were counted.

The results are shown in Fig. 7 to Fig. 10. In Fig. 7 to Fig. 10, the vertical axis indicates the mean number of cells which produced IFNγ in response to the stimulation with the added cells, from each three mice per group. The vaccine administered to the mice is indicated on the horizontal axis. The black bars and the white bars in Fig. 5 and Fig. 9 show the result that the splenocyte from the mouse was incubated in the presence or absence of Peptide SEQ ID No. 1, respectively. The black bars and the white bars in Fig. 8 and Fig. 10 show the result that the splenocyte from the mouse was incubated in the presence or absence of Peptide SEQ ID No. 5, respectively. The results of the present test showed that the CTL counts responsive to Peptide of SEQ ID No. 1 and the CTL counts responsive to Peptide of SEQ ID No. 5 were more in the case of adding the compounds prepared in Examples 2 and 3 to vaccine b, compared with the case of adding no compound.

The above results show that the induced CTL count increases by adding the compounds prepared in Examples 2 and 3 to the vaccine, and strongly suggest that the compounds prepared in Examples 2 and 3 has *in vivo* adjuvant activity.

### Test 9

### Evaluation of in vivo adjuvant activity in HLA-A^{∗}02:01 transgenic mouse

The *in vivo* adjuvant activity of the compounds prepared in Example 3 was evaluated in the following procedure.
To a vaccine comprising Peptide SEQ ID No. 1 prepared in Reference example 1 with a preliminarily-emulsified composition (hereinafter, referred to as "vaccine c") was added the compound prepared in Example 3 to prepare a vaccine. The vaccine was administered to a HLA-A^{∗}02:01 transgenic mouse, and the adjuvant activity of the vaccine was evaluated by the method of testing antigen-specific CTL induction.

Specifically, Peptide SEQ ID No. 1 was dissolved in DMSO, and then diluted with water for injection to concentrations of Peptide SEQ ID No. 1 of 2 mg/mL. The diluted peptide solution was mixed and emulsified with an equal volume of a preliminarily-emulsified composition to prepare vaccine c. Vaccine c was injected to mice intradermally at the tail base area in an amount for administering 200 µg of Peptide SEQ ID No. 1 per body. Or, in the step of preparing vaccine c, the compound prepared in Example 3 was added to the diluted peptide solution to prepare a vaccine, and the prepared vaccine was injected to mice intradermally at the tail base area in an amount for administering 200 µg of Peptide SEQ ID No. 1 per body, and 32.5 ng of the compound prepared in Example 3 per body. The administrations were done twice, which had one week interval. One week after the final administration, the mice were sacrificed with CO₂ gas. Splenocytes were harvested from spleens removed from the mice. Like Test 6, Peptide (SEQ ID No. 1) was added to the splenocyte-containing ELISPOT plate, and the plate was incubated overnight at 37°C under an atmosphere of 5 % CO₂ to re-stimulate the peptide *in vitro.* Then, after removal of the supernatant from the wells, stained spots on the ELISPOT plate were counted.

The results are shown in Fig. 11. In Fig. 11, the vertical axis indicates the mean number of cells which produced IFNγ in response to the stimulation with the added cells, from each three mice per group. The vaccine administered to the mice is indicated on the horizontal axis. The black bars and the white bars in Fig. 11 show the result that the splenocyte from the HLA-A^{∗}02:01 transgenic mouse was incubated in the presence or absence of Peptide SEQ ID No. 1, respectively. The results of the present test showed that the CTL counts responsive to Peptide of SEQ ID No. 1 were more in the case of adding the compound prepared in Example 3 to vaccine c, compared with the case of adding no compound.

The above results show that the induced CTL count increases by adding the compound prepared in Example 3 to the vaccine, and strongly suggest that the compound prepared in Example 1 has *in vivo* adjuvant activity.

### Test 10

### Evaluation of in vivo adjuvant activity in HLA-A^{∗}02:01/HLA-DRB1^{∗}01:01 transgenic mouse

The *in vivo* adjuvant activity of the compound prepared in Example 3 was evaluated in the following procedure. To a cocktail vaccine comprising Compound SEQ ID No. 1 prepared in Reference example 1 and Peptide SEQ ID No. 4 prepared in Reference example 4 with a preliminarily-emulsified composition (hereinafter, referred to as "vaccine d") was added the compound prepared in Example 3 to prepare a vaccine. The vaccine was administered to a HLA-A^{∗}02:01/HLA-DRB1^{∗}01:01 transgenic mouse (C57BL/6CrHLA-A2.1DR1), and the adjuvant activity of the vaccine was evaluated by the method of testing antigen-specific helper T-cell induction. Peptide SEQ ID No. 4 is a helper peptide derived from HLA-DRB1^{∗}01:01-restricted WT1 protein.

Specifically, Peptide SEQ ID No. 1 and Peptide SEQ ID No. 4 were dissolved in DMSO, and then diluted with water for injection to concentrations of Peptide SEQ ID No. 1 of 0.9 mg/mL and Peptide SEQ ID No. 4 of 1.8 mg/mL. The diluted peptide solution was mixed and emulsified with an equal volume of a preliminarily-emulsified composition to prepare vaccine d. Vaccine d was injected to mice intradermally at the tail base area in an amount for administering 180 µg of Peptide SEQ ID No. 1 per body and 360 µg of Peptide SEQ ID No. 4 per body. Or, in the step of preparing vaccine d, the compound prepared in Example 3 was added to the diluted vaccine to prepare a vaccine, and the prepared vaccine was injected to mice intradermally at the tail base area in an amount for administering 180 µg of Peptide SEQ ID No. 1 per body, 360 µg of Peptide SEQ ID No. 4 per body, and 3.25 ng of the compound prepared in Example 3 per body. The administrations were done twice, which had one week interval. One week after the final administration, the mice were sacrificed with CO₂ gas. Splenocytes were harvested from spleens removed from the mice. Like Test 6, Peptide (SEQ ID No. 4) was added to the splenocyte-containing ELISPOT plate at a final concentration of 10 µg/ml, and the plate was incubated overnight at 37°C under an atmosphere of 5 % CO₂ to re-stimulate the peptide *in vitro.* Then, after removal of the supernatant from the wells, stained spots on the ELISPOT plate were counted.

The result is shown in Fig. 12. In Fig. 12, the vertical axis indicates the mean number of cells which produced IFNγ in response to the stimulation with the added cells, from each three mice per group. The vaccine administered to the mice is indicated on the horizontal axis. The black bars and the white bars in Fig. 12 show the results that the splenocyte from the HLA-A^{∗}02:01/HLA-DRB1^{∗}01:01 transgenic mouse was incubated in the presence or absence of Peptide SEQ ID No. 4, respectively. The results of the present test showed that the T-cell counts responsive to Peptide SEQ ID No. 4 were more in the case of adding the compound prepared in Example 3 to vaccine d, compared with the case of adding no compound.

The above results show that the induced helper T-cell count increases by adding the compound prepared in Example 3 to the vaccine, and strongly suggest that the compound of the present invention has *in vivo* adjuvant activity.

### Test 11

### Action of potentiating immune response of antigen-specific CTL for peptide and tumor cell

The immune response of CTL induced with a vaccine comprising the present compound in the presence of tumor cells was evaluated in the following procedure. The vaccine was administered to a HLA-A^{∗}02:01 transgenic mouse to prepare splenocyte comprising an antigen peptide-specific CTL, and the immune response was evaluated by incubating the spelenocyte in the presence of tumor cells and antigen peptides.

Specifically, vaccine a prepared like Test 6 was injected to a HLA-A^{∗}02:01 transgenic mouse intradermally at the tail base area in an amount for administering 300 µg of Compound of formula 4 per body and 225 µg of Peptide SEQ ID No. 3 per body. Or, in the step of preparing vaccine a, the compound prepared in Example 3 was added to the diluted peptide solution to prepare a vaccine, and the prepared vaccine was injected to a HLA-A^{∗}02:01 transgenic mouse intradermally at the tail base area in an amount for administering 300 µg of Compound of formula 4 per body, 225 µg of Peptide SEQ ID No. 3 per body, and 32.5 ng of the compound prepared in Example 3 per body. The administrations were done twice, which had one week interval. One week after the final administration, the mice were sacrificed with CO₂ gas, and then spleens removed from the mice. A splenocyte suspension was prepared from the spleens with Complete T-cell Medium (hereinafter, CTM). The splenocytes derived from each three mice per group were mixed. The mixed splenocytes were stained with an FITC-labeled anti-CD8 antibody (BD Pharmingen) and a PE-labeled HLA tetramer (MBL) against Peptide SEQ ID NO. 1, and analyzed for peptide-specific CTLs with a flow cytometer.

The peptide (SEQ ID No. 1) solution was added to a part of the splenocyte at a final concentration of 100 µg/mL, and the cells were stood for about one hour at 37°C, 5 % CO₂. After excess peptides were washed out with CTM, the peptide-pulsed splenocyte and unpulsed splenocyte were mixed at a ratio of 1:10, and the mixture was seeded on a U-bottom 96 well plate at 3.85 × 10⁵ cells/well. A cell line (hereinafter, also referred to as LLC-HHD-WT1 tumor cells) was established by stably expressing HHD [a chimeric HLA of a human MHC (HLA-A^{∗}02:01) and a mouse MHC (H-2D^{b}) ] and antigen peptide (SEQ ID No. 1) in a mouse Lewis lung carcinoma cell line LLC as tumor cells. X-ray (50 Gy) was irradiated to the LLC-HHD-WT1 tumor cells, and then the LLC-HHD-WT1 tumor cells were cultured in the presence of a mouse recombinant IFN-γ (100 ng/mL) for about two days and washed with CTM. The LLC-HHD-WT1 tumor cells were seeded on the U-bottom 96 well plate containing the splenocytes at 3.5×10⁴ cells/well and cultured at 37°C under an atmosphere of 5 % CO₂ for about three days. The concentration of mouse IFN-γ in the culture supernatant was measured with an ELISA kit (R&D Systems). After the cultivation, the collected splenocytes were stained with an FITC-labeled anti-CD8 antibody and a PE-labeled HLA tetramer against Peptide SEQ ID NO. 1, and analyzed for peptide-specific CTLs with a flow cytometer.

The flow cytometric analysis showed that the splenocyte of the mice receiving the vaccine comprising the compound prepared in Example 3 included CTLs specific to Peptide (SEQ ID No. 1) 1.7 times as many as that of the mice receiving vaccine a which did not comprise the compound (see, white bar in Fig. 13 A). When these splenocytes were mixed with tumor cells and cultured in the presence of Peptide SEQ ID No. 1, the CTL specific to Peptide SEQ ID No. 1 in the splenocyte from the mice receiving the vaccine comprising the compound prepared in Example 3 increased, while the CTL in the splenocyte from the mice receiving vaccine a which did not comprise the compound decreased. And, the difference between them was 3.6 times (see, black bar in Fig. 13A). In addition, the amount of IFN-γ produced from the CTL in the mixed culture in the splenocyte from the mice receiving the vaccine comprising the compound prepared in Example 3 was 4.9 times as many as that of the splenocyte from the mice receiving vaccine a which did not comprise the compound (see, Fig. 13B, which shows the average of 3 wells).

These results show that the CTL induced with the vaccine comprising the compound prepared in Example 3 is less inhibited by tumor cells, compared with the CTL induced with the vaccine which does not comprise the compound, and thus the present compound can potentiate the responsivity of antigen peptide-specific CTL for peptide and tumor cells.

### Test 12

### Action of potentiating immune response of antigen-specific CTL for peptide and tumor cell

Vaccine b prepared like Test 8 was injected to a HLA-A^{∗}02:01 transgenic mouse intradermally at the tail base area in an amount for administering 300 µg of Compound of formula 4 per body and 225 µg of Peptide SEQ ID No. 3 per body. Or, in the step of preparing vaccine b, the compound prepared in Example 2 was added to the diluted peptide solution to prepare a vaccine, and the prepared vaccine was injected to a HLA-A^{∗}02:01 transgenic mouse intradermally at the tail base area in an amount for administering 300 µg of Compound of formula 4 per body, 225 µg of Peptide SEQ ID No. 3 per body, and 325 ng of the compound prepared in Example 2 per body. One week later, the mice were sacrificed with CO₂ gas, and then spleens removed from the mice. A splenocyte suspension was prepared from the spleens with CTM. Like Test 6, Peptide (SEQ ID No. 1) was added to the splenocyte-containing ELISPOT plate, and the plate was incubated overnight at 37°C under an atmosphere of 5 % CO₂ to re-stimulate the peptide *in vitro.* Then, after removal of the supernatant from the wells, stained spots on the ELISPOT plate were counted. And, like Test 11, Peptide SEQ ID No. 1 was pulsed to a part of the splenocyte, and the cell was mixed with LLC-HHD-WT1 tumor cell and cultured at 37°C under an atmosphere of 5 % CO₂ for about three days. The concentration of mouse IFN-γ in the culture supernatant was measured with an ELISA kit.

The IFN-γ ELISPOT analysis showed that the splenocyte of the mice receiving the vaccine comprising the compound prepared in Example 2 included CTLs specific to Peptide (SEQ ID No. 1) 1.8 times as many as that of the mice receiving vaccine b which did not comprise the compound (see, black bar in Fig. 14 A, which shows the average of 3 wells). These splenocytes were mixed with tumor cells and cultured in the presence of Peptide SEQ ID No. 1, and the amount of IFN-γ produced from the CTL was measured by ELISA. The results showed the amount in the splenocyte of the mice receiving the vaccine comprising the compound prepared in Example 2 was 8.7 times as many as that of the mice receiving vaccine b which did not comprise the compound (see, Fig. 14 B, which shows the average of 3 wells). These results show that the CTL induced with the vaccine comprising the compound prepared in Example 2 is less inhibited by tumor cells, compared with the CTL induced with the vaccine which does not comprise the compound, and thus the present compound can potentiate the responsivity of antigen peptide-specific CTL for peptide and tumor cells.

### Test 13

### Action of anti-PD-1 antibody in immune response of antigen-specific CTL for peptide and tumor cell

Vaccine b prepared like Test 8 was injected to a HLA-A^{∗}02:01 transgenic mouse intradermally at the tail base area in an amount for administering 300 µg of Compound of formula 4 per body and 225 µg of Peptide SEQ ID No. 3 per body. Or, a vaccine prepared by adding the compound prepared in Example 2 to vaccine b was injected to a HLA-A^{∗}02:01 transgenic mouse intradermally at the tail base area in an amount for administering 300 µg of Compound of formula 4 per body, 225 µg of Peptide SEQ ID No. 3 per body, and 325 ng of the compound prepared in Example 2 per body. The administrations were done twice, which had one week interval. One week after the final administration, the mice were sacrificed with CO₂ gas, and then spleens removed from the mice. A splenocyte suspension was prepared from the spleens with CTM. And, like Test 11, Peptide SEQ ID No. 1 was pulsed to a part of the splenocyte, and the cell was mixed with LLC-HHD-WT1 tumor cell. To the mixed cells was added an isotype control antibody (Rat IgG2ak, BD Pharmingen) or anti-PD-1 antibody (clone 29F.1A12, Biolegend) at a final concentration of 37.5 µg/mL, and the mixture was cultured at 37°C under an atmosphere of 5 % CO₂ for about three days. The concentration of mouse IFN-γ in the culture supernatant was measured with an ELISA kit.

The result of the average of 3 wells is shown in Fig. 15. When the splenocyte of the mice receiving the vaccine comprising the compound prepared in Example 2 were mixed with tumor cells and cultured in the presence of Peptide of SEQ ID No. 1 and each antibody, the amount of IFN-γ produced from the CTL in case of the cultivation in the presence of anti-PD-1 antibody was 4 times as many as in case of the cultivation in the presence of an isotype control antibody. These results show that the responsivity of the CTL for peptide and tumor cells which is induced with the vaccine comprising the compound prepared in Example 2 can further increase by the use in combination with anti-PD-1 antibody.

### Test 14

### Evaluation of activity for inducing antigen-specific effector memory CTL with HLA-A^{∗}02:01 transgenic mouse

The activity of a vaccine comprising the compounds prepared in Examples 2 and 3 for inducing effector memory CTL was evaluated through the cell surface antigen expression analysis of CTL by flow cytometry.

Specifically, vaccine a prepared like Test 6 was injected to a HLA-A^{∗}02:01 transgenic mouse intradermally at the tail base area in an amount for administering 300 µg of Compound of formula 4 per body and 225 µg of Peptide SEQ ID No. 3 per body. The administrations were done twice, which had one week interval. Or, a vaccine prepared by adding the compound prepared in Example 3 to vaccine a was injected to the mouse intradermally at the tail base area in an amount for administering 300 µg of Compound of formula 4 per body, 225 µg of Peptide SEQ ID No. 3 per body, and 32.5 ng of the compound prepared in Example 3 per body. The administrations were done twice, which had one week interval. Or, vaccine b prepared like Test 8 was injected to the mouse intradermally at the tail base area in an amount for administering 300 µg of Compound of formula 4 per body and 225 µg of Peptide SEQ ID No. 3 per body. Or a vaccine prepared by adding the compound prepared in Example 3 to vaccine b was injected to the mouse intradermally at the tail base area in an amount for administering 300 µg of Compound of formula 4 per body, 225 µg of Peptide SEQ ID No. 3 per body, and 325 ng of the compound prepared in Example 3 per body. Or a vaccine prepared by adding the compound prepared in Example 2 to vaccine b was injected to the mouse intradermally at the tail base area in an amount for administering 300 µg of Compound of formula 4 per body, 225 µg of Peptide SEQ ID No. 3 per body, and 325 ng of the compound prepared in Example 2 per body. The administrations were done twice, which had one week interval. One week after the final administration, the mice were sacrificed with CO₂ gas, and then splenocytes were harvested from spleens removed from the mice. The splenocytes derived from each three mice per group were mixed. The mixed splenocytes were stained with an FITC-labeled anti-CD8 antibody, a PE-labeled HLA tetramer against Peptide SEQ ID NO. 1, a PE-Cy7-labeled anti-CD127 antibody, and a PerCP-Cy5.5-labeled anti-CD62L antibody, and analyzed for peptide-specific effector memory CTLs with a flow cytometer. The ratio of CD8-positive tetramer-positive CD127-positive CD62L-negative fraction in the lymphocyte fraction was calculated as peptide-specific effector memory CTL.

Vaccine a comprising the compound prepared in Example 3 induced the peptide-specific effector memory CTL with high frequency, compared with vaccine a (Fig. 16A). Similarly, in both cases of adding vaccine b comprising the compound prepared in Example 3 (Fig. 16B) and vaccine b comprising the compound prepared in Example 2 (Fig. 16C), the antigen peptide-specific effector memory CTL was induced with high frequency, compared with the vaccine which did not comprise the compound.

These results show that the number of the induced antigen-specific effector memory CTLs can increase by adding a vaccine comprising the compound prepared in Example 2 or 3.

### Test 15

### Effect for enhancing the in vivo suppressive effect of vaccine for growth of tumor vaccine

The HLA-A^{∗}24:02 transgenic mouse (C57BL/6CrHLA-A24/Kb) used herein is a mouse expressing a chimeric HLA (HLA-A^{∗}2402/Kb) of a human MHC (HLA-A^{∗}24:02) and a mouse MHC (H-2Kb) (Int.J.Cancer 2002; 100: 565-570). The mouse can induce CTLs with a peptide which can induce CTLs with a peptide which can bind to human HLA-A^{∗}24:02.

A suspension of 3-methylcholanthrene in corn oil was administered to a HLA-A^{∗}24:02 transgenic mouse intradermally at the ventral region. From the tumor generated at the administration site, HLA-A^{∗}2402/Kb-expressing tumor cells were obtained. Cell lines established by stably expressing WT1 antigen peptide (SEQ ID No. 2) in the cells (herein, also referred to as MCA-A24/Kb-WT1 tumor cells) were suspended in Hanks' Balanced Salt Solution and intradermally transplanted to the ventral region of HLA-A^{∗}24:02 transgenic mouse (5 × 10⁵ cells per mouse). The mice made to receive a vehicle (Group a), a vaccine (Group b), or a vaccine containing the compound prepared in Example 2 (Group c) were classified. Six mice per group were used. Seven days before the tumor transplantation and seven days after the tumor transplantation, for the mice of Group a, a composition comprising water for injection was emulsified with an equal volume of Montanide ISA 51 VG and the emulsion was administered intradermally at the tail base area of the HLA-A^{∗}24: 02 transgenic mouse (0.1 mL per administration per mouse). For the mice of Group b, a composition comprising Compound of formula 4 and Peptide SEQ ID No. 3 was emulsified with an equal volume of Montanide ISA 51 VG and the emulsion was administered intradermally at the tail base area (per administration, 300 µg of Compound of formula 4 per body and 225 µg of Peptide SEQ ID No. 3 per body). For the mice of Group c, a composition comprising Compound of formula 4, Peptide SEQ ID No. 3, and the compound prepared in Example 2 was emulsified with an equal volume of Montanide ISA 51 VG and the emulsion was administered intradermally at the tail base area (per administration, 300 µg of Compound of formula 4 per body, 225 µg of Peptide SEQ ID No. 3 per body, and 100 ng of the compound prepared in Example 2 per body). The tumor size was measured 27 days after the tumor transplantation and the tumor volume was calculated.

Fig. 17 shows average tumor volumes of six mice in each group on the 27th day after the tumor transplantation. The vaccine (group b) significantly suppressed the growth of tumor cells, compared with the case of the vehicle (group a) (non-parametric Dunnett's multiple test, ∗**:** p < 0.05). Further, the vaccine to which the compound prepared in Example 2 was added more potently suppressed the growth of tumor cells (group c, ∗∗**:** p < 0.01).

The results show that the preventive effect for suppressing the growth of tumor with a vaccine can be enhanced by adding the present compound to the vaccine.

According to the method described in Reference example 1, the peptide shown in Table 10 was prepared as its trifluoroacetate from the corresponding starting material. These compound was dealt as a reference example since it is not within the present compounds.

| Table 10 | | | |
|---|---|---|---|
| Reference example No. | SEQ ID No. | Amino acid sequence and Structure | LCMS Condition A (m/z, Retention time (min)) |
| 18 | 18 | TYAGCLSQIF | 1102.5[M+H]⁺, 1.142 min |

According to the method described in WO 2007/063903, the compound shown in Table 11 (wherein the bond between C-C is disulfide bond) was prepared as its trifluoroacetate. The compound was dealt as a reference example since it is not within the present compounds.

| Table 11 | | | |
|---|---|---|---|
| Reference example No. | Formula No. | Structure | LCMS Condition A (m/z, Retention time (min)) |
| 19 | 5 | 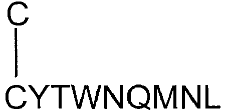 | 1291.4[M+H]⁺, 1.051 min |

### Test 16

### Evaluation of In vivo adjuvant activity in HLA-A^{∗}24:02 transgenic mouse

The *in vivo* adjuvant activity of the compound of Example 2 was evaluated in the following procedure. To a vaccine prepared by mixing Peptide SEQ ID No. 18 prepared in Reference example 18 with Montanide ISA 51 VG (hereinafter, referred to as "vaccine e") was added the compound prepared in Example 2 to prepare a vaccine. The vaccine was administered to a HLA-A^{∗}24:02 transgenic mouse, and the adjuvant activity of the vaccine was evaluated by the method of testing antigen-specific CTL induction. The TYAGCLSQIF in Peptide SEQ ID No. 18 corresponds to an antigen peptide derived from an HLA-A^{∗}24:02-restricted Or7c1 protein.

The character for inducing CTLs specific to the antigen peptide (SEQ ID No. 18) by vaccine e was evaluated by measuring whether IFNγ can be produced upon stimulation of splenocytes from the mouse with the peptide. In addition, the character for exerting *in vivo* adjuvant activity by the compound in Example 2 was evaluated by comparing the number of CTLs induced by vaccine e and the number of CTLs induced by the vaccine prepared by adding the compound prepared in Example 2 to vaccine e, and checking presence or absence of the increase on the number.

Specifically, Peptide of SEQ ID No. 18 was dissolved in DMSO, and then diluted with water for injection to concentrations of 3 mg/mL. The diluted peptide solution was mixed and emulsified with an equal volume of Montanide ISA 51 VG to prepare vaccine e. Vaccine e was injected to mice intradermally at the tail base area in an amount for administering 300 µg of Peptide of SEQ ID No. 18 per body. Or, in the step of preparing vaccine e, the compound prepared in Example 2 was added to the diluted peptide solution to prepare a vaccine, and the prepared vaccine was injected to mice intradermally at the tail base area in an amount for administering 300 µg of Peptide of SEQ ID No. 18 per body and 0.4 nmol of the compound prepared in Example 2 per body. The administrations were done twice, which had one week interval. One week after the final administration, the mice were sacrificed with CO₂ gas. Splenocytes were harvested from spleens removed from the mice. Like Test 6, Peptide (SEQ ID No. 18) was added to the splenocyte-containing ELISPOT plate at a final concentration of 10 µg/ml, and the plate was incubated overnight at 37°C under an atmosphere of 5 % CO₂ to re-stimulate the peptide *in vitro.* Then, after removal of the supernatant from the wells, stained spots on the ELISPOT plate were counted.

The result is shown in Fig. 18. In Fig. 18, the vertical axis indicates the mean number of cells which produced IFNγ in response to the stimulation with the added cells, from each three mice per group. The vaccine administered to the mice is indicated on the horizontal axis. The black bars and the white bars in Fig. 18 show the results that the splenocyte from the HLA-A^{∗}24:02 transgenic mouse was incubated in the presence or absence of Peptide SEQ ID No. 18, respectively. The results of the present test showed that the CTL counts responsive to Peptide SEQ ID No. 18 were more in the case of adding the compound prepared in Example 2 to vaccine e, compared with the case of adding no compound.

The above results show that the induced CTL count increases by adding the compound prepared in Example 2 to the vaccine, and strongly suggest that the compound prepared in Example 2 has *in vivo* adjuvant activity.

### Test 17

### Evaluation of in vivo adjuvant activity in HLA-A^{∗}02:01 transgenic mouse

The *in vivo* adjuvant activity of the compounds prepared in Examples 7, 8, and 9 was evaluated in the following procedure. To vaccine b comprising Compound of formula 4 prepared in Reference example 9 and Peptide SEQ ID No. 3 prepared in Reference example 3 with Montanide ISA51 VG was added the compound prepared in Example 7, 8, or 9 to prepare each vaccine. The vaccine was administered to a HLA-A^{∗}02:01 transgenic mouse, and the adjuvant activity of each vaccine was evaluated by the method of testing antigen-specific CTL induction.

Specifically, Compound of formula 4 and Peptide SEQ ID No. 3 were dissolved in DMSO, and then diluted with water for injection to concentrations of Compound of formula 4 of 3 mg/mL and Peptide SEQ ID No. 3 of 2.25 mg/mL. The diluted peptide solution was mixed and emulsified with an equal volume of Montanide ISA 51 VG to prepare vaccine b. Vaccine b was injected to mice intradermally at the tail base area in an amount for administering 300 µg of Compound of formula 4 per body and 225 µg of Peptide SEQ ID No. 3 per body. Or, in the step of preparing vaccine b, the compound prepared in Example 7, 8, or 9 was added to the diluted peptide solution to prepare a vaccine, and the prepared vaccine was injected to mice intradermally at the tail base area in an amount for administering 300 µg of Compound of formula 4 per body, 225 µg of Peptide SEQ ID No. 3 per body, and 0.04 nmol or 0.4 nmol of the compound prepared in Example 7, 8, or 9 per body. The administrations were done twice, which had one week interval. One week after the final administration, the mice were sacrificed with CO₂ gas. Splenocytes were harvested from spleens removed from the mice. Like Test 6, Peptide (SEQ ID No. 5) was added to the splenocyte-containing ELISPOT plate at a final concentration of 10 µg/ml, and the plate was incubated overnight at 37°C under an atmosphere of 5 % CO₂ to re-stimulate the peptide *in vitro.* Then, after removal of the supernatant from the wells, stained spots on the ELISPOT plate were counted.

The results are shown in Fig. 19. The results of the present test showed that the CTL counts responsive to Peptide SEQ ID No. 5 were more in the case of adding the compound prepared in Example 7, 8, or 9 to vaccine b, compared with the case of adding no compound.

The above results show that the induced CTL count increases by adding the compound prepared in Example 7, 8, or 9 to the vaccine, and strongly suggest that the compound prepared in Example 7, 8, or 9 has in *vivo* adjuvant activity.

### Test 18

### Evaluation of In vivo adjuvant activity in HLA-A^{∗}02:01 transgenic mouse

The *in vivo* adjuvant activity of the compound of Example 6 was evaluated in the following procedure. To vaccine b like Test 17 was added the compound prepared in Example 6 to prepare a vaccine. The vaccine was administered to a HLA-A^{∗}02:01 transgenic mouse, and the adjuvant activity of the vaccine was evaluated by the method of testing antigen-specific CTL induction.

Specifically, Compound of formula 4 and Peptide SEQ ID No. 3 were dissolved in DMSO, and then diluted with water for injection to concentrations of Compound of formula 4 of 3 mg/mL and Peptide SEQ ID No. 3 of 2.25 mg/mL. The diluted peptide solution was mixed and emulsified with an equal volume of Montanide ISA 51 VG to prepare vaccine b. Vaccine b was injected to mice intradermally at the tail base area in an amount for administering 300 µg of Compound of formula 4 per body and 225 µg of Peptide SEQ ID No. 3 per body. Or, in the step of preparing vaccine b, the compound prepared in Example 6 was added to the diluted peptide solution to prepare a vaccine, and the prepared vaccine was injected to mice intradermally at the tail base area in an amount for administering 300 µg of Compound of formula 4 per body, 225 µg of Peptide SEQ ID No. 3 per body, and 0.04 nmol of the compound prepared in Example 6 per body. The administrations were done twice, which had one week interval. One week after the final administration, the mice were sacrificed with CO₂ gas. Splenocytes were harvested from spleens removed from the mice. Like Test 6, Peptide (SEQ ID No. 5) was added to the splenocyte-containing ELISPOT plate at a final concentration of 10 µg/ml, and the plate was incubated overnight at 37°C under an atmosphere of 5 % CO₂ to re-stimulate the peptide *in vitro.* Then, after removal of the supernatant from the wells, stained spots on the ELISPOT plate were counted.

The result is shown in Fig. 20. The results of the present test showed that the CTL counts responsive to Peptide SEQ ID No. 5 were more in the case of adding the compound prepared in Example 6 to vaccine b, compared with the case of adding no compound.

The above results show that the induced CTL count increases by adding the compound prepared in Example 6 to the vaccine, and strongly suggest that the compound prepared in Example 6 has *in vivo* adjuvant activity.

### Test 19

### Evaluation of in vivo adjuvant activity in HLA-A^{∗}24:02 transgenic mouse

The *in vivo* adjuvant activity of the compound of Example 2 was evaluated in the following procedure. To a vaccine prepared by mixing Peptide SEQ ID No. 5 prepared in Reference example 19 with a preliminarily-emulsified composition (hereinafter, referred to as "vaccine f") was added the compound prepared in Example 2 to prepare a vaccine. The vaccine was administered to a HLA-A^{∗}24:02 transgenic mouse, and the adjuvant activity of the vaccine was evaluated by the method of testing antigen-specific CTL induction. The CYTWNQMNL (SEQ ID No. 2) in Peptide SEQ ID No. 5 corresponds to an antigen peptide derived from an HLA-A^{∗}24:02-restricted WT1 protein.

The character for inducing CTLs specific to the antigen peptide (SEQ ID No. 2) by vaccine f was evaluated by measuring whether IFNγ can be produced upon stimulation of splenocytes from the mouse with the peptide. In addition, the character for exerting *in vivo* adjuvant activity by the compound in Example 2 was evaluated by comparing the number of CTLs induced by vaccine f and the number of CTLs induced by the vaccine prepared by adding the compound prepared in Example 2 to vaccine f, and checking presence or absence of the increase on the number.

Specifically, Peptide of SEQ ID No. 5 was dissolved in DMSO, and then diluted with water for injection to concentrations of 3 mg/mL. The diluted peptide solution was mixed and emulsified with an equal volume of the preliminarily-emulsified composition as prepared in Test 6 to vaccine f. Vaccine f was injected to mice intradermally at the tail base area in an amount for administering 300 µg of Compound of formula 5 per body. Or, in the step of preparing vaccine f, the compound prepared in Example 2 was added to the diluted peptide solution to prepare a vaccine, and the prepared vaccine was injected to mice intradermally at the tail base area in an amount for administering 300 µg of Compound of formula 5 per body and 0.4 nmol of the compound prepared in Example 2 per body. The administrations were done twice, which had one week interval. One week later, the mice were sacrificed with CO₂ gas. Splenocytes were harvested from spleens removed from the mice. Like Test 6, Peptide (SEQ ID No. 2) was added to the splenocyte-containing ELISPOT plate at a final concentration of 10 µg/ml, and the plate was incubated overnight at 37°C under an atmosphere of 5 % CO₂ to re-stimulate the peptide *in vitro.* Then, after removal of the supernatant from the wells, stained spots on the ELISPOT plate were counted.

The result is shown in Fig. 21. In Fig. 21, the vertical axis indicates the mean number of cells which produced IFNγ in response to the stimulation with the added cells, from each three mice per group. The vaccine administered to the mice is indicated on the horizontal axis. The black bars and the white bars in Fig. 21 show the result that the splenocyte from the HLA-A^{∗}24:02 transgenic mouse was incubated in the presence or absence of Peptide SEQ ID No. 2, respectively. The results of the present test showed that the CTL counts responsive to Peptide of SEQ ID No. 2 were more in the case of adding the compound prepared in Example 2 to vaccine f, compared with the case of adding no compound.

The above results show that the induced CTL count increases by adding the compound prepared in Example 2 to the vaccine, and strongly suggest that the compound prepared in Example 2 has *in vivo* adjuvant activity.

## Claims

1. A compound of formula (1): 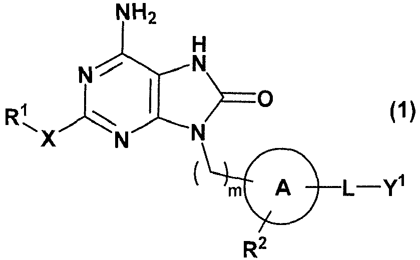 or a pharmaceutically acceptable salt thereof, wherein
X is single bond, oxygen atom, sulfur atom, SO, SO₂, or NR⁶, wherein R⁶ is hydrogen atom or C₁₋₆ alkyl,
R¹ is C₁₋₆ alkyl which may be substituted with 1 - 5 substituents selected independently from the group consisting of halogen, hydroxy, and C₁₋₆ alkoxy,
R² is hydrogen atom, halogen, hydroxy, C₁₋₆ alkyl, C₁₋₆ alkoxy or cyano,
m is an integer of 1 - 5,
A is 5- to 8-membered monocyclic aromatic carbon ring or 4- to 7-membered monocyclic aromatic hetero ring,
L is a linker, and
Y¹ is - (CH₂CH₂O)ₙ-R³, wherein R³ is hydrogen atom or C₁₋₆ alkyl, and n is an integer of 3 - 100,
provided that the compound having the following structure is excluded.

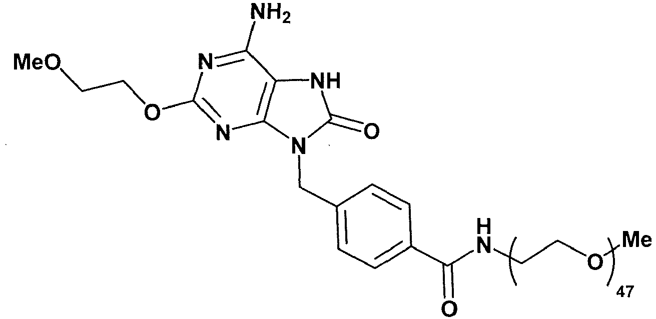

2. The compound of claim 1 or a pharmaceutically acceptable salt thereof, wherein X is single bond, oxygen atom, or NR⁶ (wherein R⁶ is hydrogen atom or C₁₋₆ alkyl) .

3. The compound of claim 2 or a pharmaceutically acceptable salt thereof, wherein X is single bond or oxygen atom.

4. The compound of any one of claims 1 to 3 or a pharmaceutically acceptable salt thereof, wherein R¹ is C₁₋₆ alkyl which may be substituted with 1 - 3 the same or different C₁₋₆ alkoxy.

5. The compound of claim 4 or a pharmaceutically acceptable salt thereof, wherein R¹ is C₁₋₆ alkyl which may be substituted with one C₁₋₆ alkoxy.

6. The compound of any one of claims 1 to 5 or a pharmaceutically acceptable salt thereof, wherein R² is hydrogen atom, halogen, C₁₋₆ alkyl, or C₁₋₆ alkoxy.

7. The compound of claim 6 or a pharmaceutically acceptable salt thereof, wherein R² is hydrogen atom or halogen.

8. The compound of any one of claims 1 to 7 or a pharmaceutically acceptable salt thereof, wherein m is 1.

9. The compound of any one of claims 1 to 8 or a pharmaceutically acceptable salt thereof, wherein A is benzene ring or pyridine ring.

10. The compound of claim 9 or a pharmaceutically acceptable salt thereof, wherein A is pyridine ring.

11. The compound of any one of claims 1 to 10 or a pharmaceutically acceptable salt thereof, wherein
L is -O-, -NR^{Y}-, -C(O)-, -C(O)O-, -OC(O)-, -C(O)NR^{Y}-, - NR^{Y}C(O)-, -CH₂NR^{Y}-, -CH₂O-, -OC(O)O-, -NR⁴C(O)O-, -OC(O)NR^{Y}-, -NR⁴C(O)NR^{Y}-, -OC(S)NR^{Y}-, or -NR⁴C(S)NR^{Y}-, wherein R⁴ is hydrogen atom or C₁₋₆ alkyl, and R^{Y} is hydrogen atom, C₁₋₆ alkyl or Y² wherein Y² is -(CH₂CH₂O)ₚ-R⁵ (wherein R⁵ is hydrogen atom or C₁₋₆ alkyl, and p is an integer of 3 - 100).

12. The compound of Item 11 or a pharmaceutically acceptable salt thereof, wherein L is -CH₂NR^{Y}-.

13. The compound of claim 12 or a pharmaceutically acceptable salt thereof, wherein L is -CH₂NR^{Y}- wherein R^{Y} is hydrogen atom, C₁₋₆ alkyl or Y² wherein Y² is -(CH₂CH₂O)ₚ-R⁵ (wherein R⁵ is hydrogen atom or C₁₋₆ alkyl, and p is an integer of 3 - 100).

14. The compound of any one of claims 1 to 13 or a pharmaceutically acceptable salt thereof, wherein Y¹ is - (CH₂CH₂O)ₙ-R³ wherein n is an integer of 3 - 40, and R³ is hydrogen atom or C₁₋₆ alkyl.

15. The compound of claim 1 or a pharmaceutically acceptable salt thereof, wherein the compound is a compound of formula (2) : 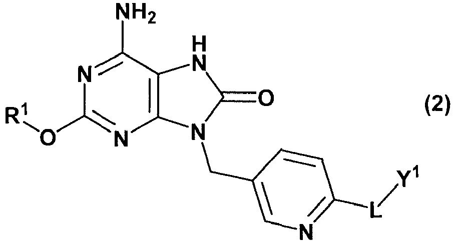 or formula (3): 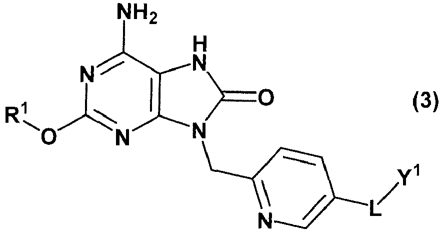 wherein
R¹ is C₁₋₆ alkyl which may be substituted with one C₁₋₆ alkoxy,
L is -CH₂NR^{Y}- wherein R^{Y} is hydrogen atom or C₁₋₆ alkyl,
Y¹ is -(CH₂CH₂O)ₙ-R³ (wherein R³ is hydrogen atom or C₁₋₆ alkyl, and n is an integer of 3 - 40).

16. The compound of claim 1 or a pharmaceutically acceptable salt thereof, which is selected from:
6-amino-2-butoxy-9-{ [5-(16-hydroxy-2-methyl-5,8,11,14-tetraoxa-2-azahexadecan-1-yl)pyridin-2-yl]methyl}-7,9-dihydro-8H-purin-8-one,
6-amino-2-butoxy-9-{ [6-(16-hydroxy-2-methyl-5,8,11,14-tetraoxa-2-azahexadecan-1-yl)pyridin-3-yl]methyl}-7,9-dihydro-8H-purin-8-one,
6-amino-2-butoxy-9-{[4-(16-hydroxy-2-methyl-5,8,11,14-tetraoxa-2-azahexadecan-1-yl)phenyl]methyl}-7,9-dihydro-8H-purin-8-one,
6-amino-9-{[4-(16-hydroxy-2-methyl-5,8,11,14-tetraoxa-2-azahexadecan-1-yl)phenyl]methyl}-2-(2-methoxyethoxy)-7,9-dihydro-8H-purin-8-one,
6-amino-2-butoxy-9-({6-[13-hydroxy-2-(2-{2-[2-(2-hydroxyethoxy)ethoxy]ethoxy}ethyl)-5,8,11-trioxa-2-azatridecan-1-yl]pyridin-3-yl}methyl)-7,9-dihydro-8H-purin-8-one,
6-amino-2-butoxy-9-{[6-(31-hydroxy-2-methyl-5,8,11,14,17,20,23,26,29-nonaoxa-2-azahentriacontan-1-yl)pyridin-3-yl]methyl}-7,9-dihydro-8H-purin-8-one,
6-amino-2-butoxy-9-{[6-(73-hydroxy-2-methyl-5, 8, 11, 14, 17, 20, 23, 26, 29, 32, 35, 38, 41, 44, 47, 50, 53, 56, 59, 62, 6 5, 68, 71-tricosaoxa-2-azatriheptacontan-1-yl)pyridin-3-yl]methyl}-7,9-dihydro-8H-purin-8-one, and
6-amino-2-butoxy-9-{[6-(109-hydroxy-2-methyl-5, 8, 11, 14, 17, 20, 23, 26, 29, 32, 35, 38, 41, 44, 47, 50, 53, 56, 59, 62, 6 5, 68, 71, 74, 77, 80, 83, 86, 89, 92, 95, 98, 101, 104, 107-pentatriacontaoxa-2-azanonahectan-1-yl)pyridin-3-yl]methyl}-7,9-dihydro-8H-purin-8-one.

17. A pharmaceutical composition comprising the compound of any one of claims 1 to 16 or a pharmaceutically acceptable salt thereof.

18. The pharmaceutical composition of claim 17, which is an emulsion formulation.

19. The pharmaceutical composition of claim 18, wherein the emulsion formulation is a water-in-oil emulsion.

20. The pharmaceutical composition of claim 19, wherein the emulsion formulation comprises (1) ethyl oleate, octyldodecyl myristate, sorbitan monooleate, glyceryl monooleate, polyoxyethylene hydrogenated castor oil 20, glycerin, and sodium dihydrogen phosphate, or (2) Montanide ISA 51VG.

21. The pharmaceutical composition of any one of claims 18 to 20, which further comprises a tumor antigen.

22. The pharmaceutical composition of claim 21, wherein the tumor antigen is a tumor antigen peptide.

23. The pharmaceutical composition of claim 22, wherein the tumor antigen peptide is a combination of a peptide represented by the amino acid sequence of formula (4): 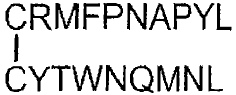 wherein the bond between C-C is disulfide bond, or a pharmaceutically acceptable salt thereof, and
a peptide represented by the amino acid sequence of SEQ ID NO 3: WAPVLDFAPPGASAYGSL, or a pharmaceutically acceptable salt thereof.

24. A vaccine adjuvant for cancer vaccine comprising a compound of formula (1): 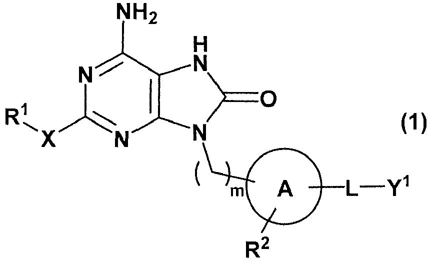 or a pharmaceutically acceptable salt thereof, wherein
X is single bond, oxygen atom, sulfur atom, SO, SO₂, or NR⁶, wherein R⁶ is hydrogen atom or C₁₋₆ alkyl,
R¹ is C₁₋₆ alkyl which may be substituted with 1 - 5 substituents selected independently from the group consisting of halogen, hydroxy, and C₁₋₆ alkoxy,
R² is hydrogen atom, halogen, hydroxy, C₁₋₆ alkyl, C₁₋₆ alkoxy or cyano,
m is an integer of 1 - 5,
A is 5- to 8-membered monocyclic aromatic carbon ring or 4- to 7-membered monocyclic aromatic hetero ring,
L is a linker, and
Y¹ is - (CH₂CH₂O)ₙ-R³, wherein R³ is hydrogen atom or C₁₋₆ alkyl, and n is an integer of 3 - 100.

25. A kit comprising
a) the compound of claim 1 or a pharmaceutically acceptable salt thereof, or a pharmaceutical composition comprising the compound of claim 1 or a pharmaceutically acceptable salt thereof; and
b) a tumor antigen or a pharmaceutical composition comprising a tumor antigen.
